# EUROPÄISCHE PATENTSCHRIFT

(11) **EP 2 488 030 B1**
(45) Veröffentlichungstag und Bekanntmachung des Hinweises auf die Patenterteilung: **07.01.2015**
(21) Anmeldenummer: 10766020.1
(22) Anmeldetag: 07.10.2010
(51) Int. Cl.: A01N 43/78, A01N 43/82, A01N 43/88, A01N 55/08, C07D 213/84, C07D 401/04, C07D 403/04, C07D 403/14, A01P 7/00, A01P 5/00, A01N 43/56, A01N 43/647, A01N 43/653, A01N 43/707, A01N 43/76, C07D 405/14, C07D 413/14, C07D 417/14

(54) **1-(PYRID-3-YL)-PYRAZOLE UND 1-(PYRIMID-5-YL)-PYRAZOLE ALS SCHÄDLINGSBEKÄMPFUNGSMITTEL**
1-(PYRID-3-YL)-PYRAZOLES AND 1-(PYRIMID-5-YL)-PYRAZOLES AS PEST CONTROLLERS
1-(PYRID-3-YL)-PYRAZOLES ET 1-(PYRIMID-5-YL)-PYRAZOLES COMME MOYEN DE LUTTE CONTRE LES PARASITES

(30) Priorität: 12.10.2009 EP 09172737; 13.10.2009 US 251058 P
(43) Veröffentlichungstag der Anmeldung: 22.08.2012
(73) Patentinhaber: Bayer CropScience AG, 40789 Monheim (DE)
(72) Erfinder: BRETSCHNEIDER, Thomas, 53797 Lohmar (DE); FÜSSLEIN, Martin, 40225 Düsseldorf (DE); KÖHLER, Adeline, 42105 Wuppertal (DE); MÜHLTHAU, Friedrich, August, 65812 Bad Soden am Taunus (DE); FRANKEN, Eva-Maria, 51381 Leverkusen (DE); VOERST, Arnd, 50674 Köln (DE)
(74) Vertreter: BIP Patents
(86) Internationale Anmeldenummer: PCT/EP2010/064973
(87) Internationale Veröffentlichungsnummer: WO 2011/045224

(56) Entgegenhaltungen:
- EP-A1- 1 668 984
- EP-A1- 1 938 686
- EP-A2- 0 412 849
- WO-A1-2005/041950
- WO-A2-03/051833
- WO-A2-2008/073942
- WO-A2-2009/018185

## Beschreibung

Die vorliegende Anmeldung betrifft die Verwendung zum Teil bekannter heterocyclischer Verbindungen zur Bekämpfung von tierischen Schädlingen, zu denen Arthropoden und insbesondere Insekten zählen, ferner neue heterocyclische Verbindungen und Verfahren zu deren Herstellung.

Bestimmte Pyrazolylverbindungen sind bereits bekannt geworden, eine Verwendung zur Bekämpfung tierischer Schädlinge wurde jedoch nicht beschrieben (vgl. WO 2003/051833 A2, WO 2006/044502 A2).

Auch in WO 1998/056785 A1 und WO 1996/032938 A1 werden Pyrazolderivate offenbart, für die pharmazeutische Verwendungen angegeben werden.

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch zum Teil neue Verbindungen der Formel (I), 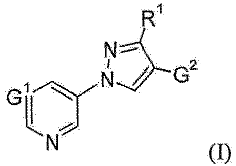 worin
- G¹: für N, CH oder C-Halogensteht,
- R¹: für Wasserstoff oder Alkyl steht und
- G²: für gegebenenfalls substituiertes Heterocyclyl, für gegebenenfalls substituiertes Heteroaryl oder für gegebenenfalls substituiertes Aryl steht,
sowie Salze, Metallkomplexe und N-Oxide der Verbindungen der Formel (I), die zur Bekämpfung von tierischen Schädlingen verwendet werden können.

Es wurde gefunden, das die Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die bekannten Verbindungen der Formel (I) sind nach den Herstellverfahren erhältlich, die in den oben genannten Publikationen beschrieben sind.

Die erfindungsgemäß zu verwendenden Verbindungen sind durch die Formel (I) allgemein definiert.

Bevorzugte Substituenten bzw. Bereiche der in den oben erwähnten Verbindungen der Formel (I) aufgeführten Reste werden im Folgenden erläutert.

Bevorzugt verwendet werden Verbindungen der Formel (I), worin
- G¹: für N, CH oder C-Halogen steht,
- R¹: für Wasserstoff oder Alkyl steht,
- G²: für A- R²ₐ, D- R³_{b} oder E- R⁴_{c} steht, worin
- A: für Heterocyclyl aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, Oxazolin-2-yl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 5,6-Dihydro-[1,3,4]-oxadiazin-2-yl, 5,6-Dihydro-[1,3,4]-thiadiazin-2-yl, 5,6-Dihydro-[1,4,2]-dioxazin-3-yl, Hydroxypyridyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-l-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl steht,
- R²: für einen Rest aus der Reihe Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl, Aminocarbonyl, Pyridyl und Pyrimidyl (wobei Pyridyl und Pyrimidyl selbst wiederum substituiert sein können durch Halogen, Alkyl, Halogenalkyl, Alkoxy und Halogenalkoxy) steht,
- a: für eine Zahl aus der Reihe 0, 1, 2 und 3 steht,
- D: für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl) steht,
- R³: für einen Rest aus der Reihe Halogen, Nitro, Amino, Cyano, Alkylamino, Halogenalkylamino, Dialkylamino, Alkyl, Halogenalkyl, Cycloalkylalkyl, Alkoxy, Halogenalkoxy, Alkoxyalkyl, halogeniertes Alkoxyalkyl, Bis(alkoxy)alkyl, Alkoxycarbonyl, Alkoxycarbonylalkyl, alpha-Hydroxyimino-alkoxycarbonylalkyl, alpha-Alkoxyimino-alkokycarbonylalkyl, C(X)NR⁵R⁶ (worin X für Sauerstoff, Schwefel oder NOH steht, R⁵ für Wasserstoff oder Alkyl steht und R⁶ für einen Rest aus der Reihe Wasserstoff, Alkyl, Halogenalkyl, Cyanoalkyl, Alkinyl, Cycloalkyl, Cycloalkylalkyl, Alkoxyalkyl, Alkoxycarbonyl, Alkoxycarbonylalkyl, Alkylthioalkyl, Aryl, Arylalkyl und Hetarylalkyl steht oder R⁵ und R⁶ gemeinsam mit dem Stickstoffatom, an das sie gebunden sind, einen Ring bilden, der ein oder mehrere weitere Heteroatome aus der Reihe Stickstoff, Sauerstoff und Schwefel enthalten kann), NR⁷R⁸ (worin R⁷ für Wasserstoff oder Alkyl steht und R⁸ für einen Rest aus der ReiheWasserstoff, Alkyl, Halogenalkyl, Cyanoalkyl, Alkinyl, Cycloalkyl, Cycloalkylalkyl, Alkoxy, Halogenalkoxy, Alkoxyalkyl, Alkoxycarbonyl, Alkoxycarbonylalkyl, Alkylthioalkyl, Aryl, Arylalkyl oder Hetarylalkyl steht oder R⁷ und R⁸ gemeinsam mit dem Stickstoffatom, an das sie gebunden sind, einen Ring bilden, der ein oder mehrere weitere Heteroatome aus der Reihe Stickstoff, Sauerstoff und Schwefel enthalten kann), Alkylthio, Alkylsulfinyl, Alkylsulfonyl, die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (welche selbst wiederum substituiert sein können durch Alkyl, Halogenalkyl, Alkoxy und Alkoxyalkyl), Phenyl (welches selbst wiederum substituiert sein kann durch Halogen, Cyano, Nitro, Alkyl und Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl) und die Heteroarylalkylreste Triazolylalkyl, Pyridylalkyl, Pyrimidylalkyl und Oxadiazolylalkyl (welche selbst wiederum substituiert sein können durch Halogen und Alkyl),
- b: für eine Zahl aus der Reihe 0, 1, 2 und 3 steht,
- E: für Aryl, insbesondere Phenyl, steht,
- R⁴: für einen Rest aus der Reihe Halogen, Cyano, Alkyl, Halogenalkyl, Alkoxy, Halogenalkoxy, Dioxolanyl oder Dihydrodioxazinyl steht und
- c: für eine Zahl aus der Reihe 0, 1, 2 und 3 steht.

Besonders bevorzugt verwendet werden Verbindungen der Formel (I), worin
- G¹: für N, CH oder C-Halogen steht,
- R¹: für Wasserstoff oder C₁-C₆-Alkyl steht und
- G²: für A- R²ₐ, D- R³_{b} oder E- R⁴_{c} steht, worin
- A: für Heterocyclyl aus der Reihe Oxazolin-2-yl, 5,6-Dihydro-[1,3,4]-oxadiazin-2-yl, 5,6-Dihydro-[1,3,4]-thiadiazin-2-yl, 5,6-Dihydro-[1,4,2]-dioxazin-3-yl und Hydroxypyridyl steht,
- R²: für einen Rest aus der Reihe Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-lkyl, C₁-C₆-Alkylcarbonyl, C₁-C₆-Alkoxycarbonyl, Aminocarbonyl, Pyridyl und Pyrimidyl (wobei Pyridyl und Pyrimidyl selbst wiederum substituiert sein können durch Halogen, C₁-C₆-Alkyl, C₁-C₆-Halogenalkyl, C₁-C₆-Alkoxy und C₁-C₆-Halogenalkoxy) steht,
- a: für eine Zahl aus der Reihe 0, 1, 2 und 3 steht,
- D: für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl) steht,
- R³: für einen Rest aus der Reihe Halogen, Nitro, Amino, Cyano, C₁-C₆-Alkylamino, C₁-C₆-Halogenalkylamino Di-(C₁-C₆-alkyl)amino, C₁-C₆-Alkyl, C₁-C₆-Halogenalkyl, C₃-C₆-Cycloalkyl-C₁-C₆-alkyl, C₁-C₆-Alkoxy, C₁-C₆-Halogenalkoxy, C₁-C₆-Alkoxy-C₁-C₆-alkyl, halogeniertes C₁-C₆-Alkoxy-C₁-C₆-alkyl, Bis(C₁-C₆-alkoxy)-C₁-C₆-alkyl, C₁-C₆-Alkoxycarbonyl, alpha-Hydroxyimino-C₁-C₆-alkoxycarbonylmethyl, alpha- C₁-C₆-Alkoxyimino-C₁-C₆-alkoxycarbonylmethyl, C(X)NR⁵R⁶, (worin X für Sauerstoff, Schwefel oder NOH steht, R⁵ für Wasserstoff oder C₁-C₆-Alkyl steht und R⁶ für einen Rest aus der Reihe Wasserstoff, C₁-C₆-Alkyl, C₁-C₆-Halogenalkyl, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy-C₁-C₆-alkyl, C₁-C₆-Alkoxycarbonyl, C₁-C₆-Alkoxycarbonyl-C₁-C₆-alkyl, C₁-C₆-Alkylthio-C₁-C₆-alkyl, Phenyl, Phenyl-C₁-C₆-alkyl oder Hetatyl-C₁-C₆-alkyl steht, worin Hetaryl für Pyrimidyl steht oder R⁵ und R⁶ gemeinsam mit dem Stickstoffatom, an das sie gebunden sind, einen 5- bis 7-gliedrigen Ring bilden, der ein weiteres Heteroatom aus der Reihe Stickstoff, Sauerstoff und Schwefel enthalten kann), NR⁷R⁸ (worin R⁷ für Wasserstoff oder C₁-C₆-Alkyl steht und R⁸ für einen Rest aus der Reihe Wasserstoff, C₁-C₆-Alkyl, C₁-C₆-Halogenalkyl, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₁-C₆-Alkoxy-C₁-C₆-alkyl, C₁-C₆-Alkoxycarbonyl, C₁-C₆-Alkoxycarbonyl-C₁-C₆-alkyl, C₁-C₆-Alkylthio-C₁-C₆-alkyl, Phenyl, Phenyl-C₁-C₆-alkyl und Hetaryl-C₁-C₆-alkyl steht, worin Hetaryl für Pyrimidyl steht oder R⁷ und R⁸ gemeinsam mit dem Stickstoffatom, an das sie gebunden sind, einen 5- bis 7-gliedrigen Ring bilden, der ein weiteres Heteroatom aus der Reihe Stickstoff, Sauerstoff und Schwefel enthalten kann), C₁-C₆-Alkylthio, C₁-C₆-Alkylsulfinyl, C₁-C₆-Alkylsulfonyl, die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (insbesonderel,3-Dioxolan-2-yl, 1,3-Dioxan-2-yl und 1,2,4-Triazolin-3-on-2-yl) (welche selbst wiederum substituiert sein können durch C₁-C₆-Alkyl, C₁-C₆-Halogenalkyl, C₁-C₆-Alkoxy und C₁-C₆-Alkoxy-C₁-C₆-alkyl), Phenyl (welches selbst wiederum substituiert sein kann durch Halogen, Cyano, Nitro, C₁-C₆-Alkyl und C₁-C₆-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (insbesondere Pyrid-2-yl, Pyrimid-2-yl, Pyrimid-4-yl, Pyrazol-1-yl, Pyrazol-2-yl, 1,2,4-Oxadiazol-3-yl, 1,2,4-Triazol-3-yl und 1,2,4-Triazin-3-yl) (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl) und die Heteroarylalkylreste Triazolyl-C₁-C₆-alkyl, Pyridyl-C₁-C₆-alkyl, Pyrimidyl-C₁-C₆-alkyl und Oxadiazolyl-C₁-C₆-alkyl (welche selbst wiederum substituiert sein können durch Halogen und C₁-C₆-Alkyl) steht,
- b: für eine Zahl aus der Reihe 1, 2 und 3 steht,
- E: für Phenyl steht,
- R⁴: für einen Rest aus der Reihe Halogen, Cyano, C₁-C₆-Alkyl, C₁-C₆-Halogenalkyl, C₁-C₆-Alkoxy, C₁-C₆-Halogenalkoxy, Dioxolanyl und Dihydrodioxazinyl steht und
- c: für eine Zahl aus der Reihe 0, 1, 2 und 3 steht.

Ganz besonders bevorzugt verwendet werden Verbindungen der Formel (I), worin
- G¹: für N, CH oder C-Halogen steht,
- R¹: für Wasserstoff oder Methyl (insbesondere Wasserstoff) steht und
- G²: für A- R²ₐ, D- R³_{b} oder E- R⁴_{c} steht, worin
- A: für Heterocyclyl aus der Reihe Oxazolin-2-yl, 5,6-Dihydro-[1,3,4]-oxadiazin-2-yl, 5,6-Dihydro-[1,3,4]-thiadiazin-2-yl und 5,6-Dihydro-[1,4,2]-dioxazin-3-yl steht,
- R²: für einen Rest aus der Reihe Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl C₁-C₄-alkyl, C₁-C₄-Alkylcarbonyl, C₁-C₄-Alkoxycarbonyl, Aminocarbonyl, Pyridyl und Pyrimidyl (wobei Pyridyl und Pyrimidyl selbst wiederum substituiert sein können durch Halogen, C₁-C₄-Alkyl, C₁-C₄-Halogenalkyl, C₁-C₄-Alkoxy und C₁-C₄-Halogenalkoxy) steht,
- a: für eine Zahl aus der Reihe 0, 1, 2 und 3 steht,
- D: für einen Heteroarylrest aus der Reihe Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl, 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl (insbesondere Pyrazol-1-yl, Pyrazol-3-yl, Pyrid-2-yl, Pyrid-3-yl, Pyrimid-2-yl, Pyrimid-4-yl, Thiazol-2-yl und Thiazol-4-yl) steht,
- R³: für einen Rest aus der Reihe Halogen, Nitro, Amino, Cyano, C₁-C₄-Alkylamino, C₁-C₄-Halogenalkylamino, Di-(C₁-C₄-alkyl)amino, C₁-C₄-Alkyl, C₁-C₄-Halogenalkyl, C₃-C₆-Cycloalkyl-C₁-C₄-alkyl, C₁-C₄-Alkoxy, C₁-C₄-Halogenalkoxy, C₁-C₄-Alkoxy-C₁-C₄-alkyl, halogeniertes C₁-C₄-Alkoxy-C₁-C₄-alkyl, Bis(C₁-C₄-alkoxy)-C₁-C₄-alkyl, C₁-C₄-Alkoxycarbonyl, alpha-Hydroxyimino-C₁-C₄-alkoxycarbonylmethyl, alpha- C₁-C₄-Alkoxyimino-C₁-C₄-alkoxycarbonylmethyl, C(X)NR⁵R⁶, (worin X für Sauerstoff oder Schwefel steht, R⁵ für Wasserstoff oder C₁-C₄-Alkyl steht und R⁶ für einen Rest aus der-Reihe Wasserstoff, C₁-C₅-Alkyl, C₁-C₄-Halogenalkyl, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl und Phenyl-C₁-C₄-alkyl steht oder R⁵ und R⁶ gemeinsam mit dem Stickstoffatom, an das sie gebunden sind, einen 5- oder 6-gliedrigen Ring bilden, der ein weiteres Heteroatom aus der Reihe Sauerstoff, Schwefel und Stickstoff enthalten kann), NR⁷R⁸ (worin R⁷ für Wasserstoff oder C₁-C₄-Alkyl steht und R⁸ für einen Rest aus der Reihe Wasserstoff, C₁-C₅-Alkyl, C₁-C₄-Halogenalkyl, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy, C₁-C₄-Halogenalkoxy, C₁-C₄-Alkoxycarbonyl, C₁-C₄-Alkoxycarbonyl-C₁-C₄-alkyl, C₁-C₄-Alkoxy-C₁-C₄-alkyl, C₁-C₄-Alkylthio-C₁-C₄-alkyl oder Phenyl-C₁-C₄-alkyl steht oder R⁷ und R⁸ gemeinsam mit dem Stickstoffatom, an das sie gebunden sind, einen 5- oder 6-gliedrigen Ring bilden, der ein weiteres Heteroatom aus der Reihe Sauerstoff, Schwefel und Stickstoff enthalten kann), C₁-C₄-Alkylthio, C₁-C₄-Alkylsulfinyl, C₁-C₄-Alkylsulfoliyl, die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (insbesonderel,3-Dioxolan-2-yl, 1,3-Dioxan-2-yl und 1,2,4-Triazolin-3-on-2-yl) (welche selbst wiederum substituiert sein können durch C₁-C₄-Alkyl, C₁-C₄-Halogenalkyl und C₁-C₄-Alkoxy-C₁-C₄-alkyl), Phenyl (welches selbst wiederum substituiert sein kann durch Halogen, Cyano, Nitro, C₁-C₄-Alkyl und C₁-C₄-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (insbesondere Pyrid-2-yl, Pyrimid-2-yl, Pyrimid-4-yl, Pyrazol-1-yl, Pyrazol-2-yl, 1,2,4-Oxadiazol-3-yl, 1,2,4-Triazol-3-yl und 1,2,4-Triazin-3-yl) (welche selbst wiederum substituiert sein können durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl) und die Heteroarylalkylreste Triazolyl-C₁-C₄-alkyl, Pyridyl-C₁-C₄-alkyl, Pyrimidyl-C₁-C₄-alkyl oder Oxadiazolyl-C₁-C₄-alkyl (welche selbst wiederum substituiert sein können durch Halogen und C₁-C₄-Alkyl),
- b: für eine Zahl aus der Reihe 1, und 2 steht,
- E: für Phenyl steht und
- R⁴: für einen Rest aus der Reihe Halogen, Cyano, C₁-C₄-Alkyl, C₁-C₄-Halogenalkyl, C₁-C₄-Alkoxy und C₁-C₄-Halogenalkoxy steht und
- c: für eine Zahl aus der Reihe 0, 1, und 2 steht.

Ganz besonders hervorgehoben verwendet werden Verbindungen der Formel (I), worin
- G¹: für N, CH oder C-Halogen (im Fall von C-Halogen insbesondere für C-F) steht,
- R¹: für Wasserstoff oder Methyl (insbesondere für Wasserstoff) steht,
- G²: für A- R²ₐ oder D- R³_{b} steht, worin
- A: für Heterocyclyl aus der Reihe Oxazolin-2-yl, 5,6-Dihydro-[1,3,4]-oxadiazin-2-yl und 5,6-Dihydro-[1,3,4]-thiadiazin-2-yl steht,
- R²: für einen Rest aus der Reihe Pyridyl und Pyrimidin-2-yl steht, welche durch Halogen, C₁-C₄-Alkyl, C₁-C₄-Halogenalkyl, C₁-C₄-Alkoxy und C₁-C₄-Halogenalkoxy substituiert sein können,
- a: für 0, 1 oder 2 und insbesondere für 1 steht,
- D: für einen Heteroarylrest aus der Reihe Pyrazol-1-yl, Pyrazol-3-yl, Pyrid-2-yl, Pyrid-3-yl, Pyrimid-2-yl, Pyrimid-4-yl, Thiazol-2-yl und Thiazol-4-yl steht,
- R³: für einen Rest aus der Reihe Halogen (insbesondere Chlor, Brom), Nitro, Amino, Cyano, C₁-C₄-Halogenalkyl (insbesondere CF₃, CF₃CH₂, CH₃CF₂, CF₃CF₂, CF₂Cl, CF₃CF₂CF₂, CH₃CHF), C₃-C₆-Cycloalkyl-C₁-C₄-alkyl (insbesondere Cyclopropylmethyl), C₁-C₄-Alkoxy (insbesondere Methoxy), C₁-C₄-Halogenalkoxy (insbesondere CF₃CH₂O), C₁-C₄-Alkoxy-C₁-C₄-alkyl (insbesondere Methoxymethyl), halogeniertes C₁-C₄-Alkoxy-C₁-C₄-alkyl (insbesondere CF₂CH₂OCH₃), Bis(C₁-C₄-alkoxy)-C₁-C₄-alkyl (insbesondere (CH₃O)₂CH), C₁-C₄-Alkoxycarbonyl (insbesondere Methoxycarbonyl), alpha-Hydroxyimino-C₁-C₄-alkoxycarbonylmethyl, C(X)NR⁵R⁶, (worin X für Sauerstoff oder Schwefel steht, R⁵ für Wasserstoff oder C₁-C₄-Alkyl (insbesondere Methyl) steht und R⁶ für einen Rest aus der Reihe C₁-C₅₋Alkyl (insbesondere Methyl, Ethyl, n-Propyl, i-Propyl, iso-Butyl, tert.-Butyl), C₁-C₄-Halogenalkyl (insbesondere CF₃CH₂), Cyano-C₁-C₄-alkyl (insbesondere NCCH₂CH(C₂H₅)), C₃-C₆-Cycloalkyl (insbesondere Cyclopropyl, Cyclopentyl, Cyclohexyl), C₃-C₆-Cycloalkyl-C₁-C₄-alkyl (insbesondere Cyclopropylmethyl), C₁-C₄-Alkoxy-C₁-C₄-alkyl (insbesondere CH₃OCH₂CH(CH₃), CH₃CH₂CH₂OCH₂CH(CH₃), CH₃CH₂OCH₂CH₂, CH₃0CH₂CH₂CH₂, CH₃OCH₂CHC₂H₅, CH₃CH₂OCH₂CH(CH₃), CH₃CH₂OCH₂CH₂CH₂, CH₃OC(CH₃)₂), C₁-C₄-Alkylthio-C₁-C₄-alkyl (insbesondere CH₃SCH₂CH₂) und Phenyl-C₁-C₄-alkyl (insbesondere C₆H₅CH(CH₃) steht oder R⁵ und R⁶ gemeinsam mit dem Stickstoffatom, an das sie gebunden sind, einen 5- oder 6-gliedrigen Ring bilden, der ein weiteres Heteroatom aus der Reihe Sauerstoff, Schwefel und Stickstoff enthalten kann (insbesondere stehen R⁵ und R⁶ zusammen für (CH₂)₄, CH₂)₅ oder (CH₂)₂O(CH₂)₂), NR⁷R⁸ (worin R⁷ für Wasserstoff oder Methyl steht und R⁸ für einen Rest aus der Reihe Wasserstoff, C₁-C₄-Alkyl (insbesondere Methyl und Ethyl), C₁-C₄-Halogenalkyl (insbesondere CH₂CF₃ und CH₂CF₂H), C₁-C₄-Alkoxy (insbesondere OCH₃), C₁-C₄-Alkylcarbonyl (insbesondere COCH₃), C₁-C₄-Alkoxycarbonyl (insbesondere CO₂CH₃, CO₂CH₂CH₃ und CO₂C(CH₃)₃,) und C₁-C₄-Alkoxycarbonyl-C₁-C₄-alkyl (insbesondere CH₂CO₂CH₃) steht oder R⁷ und R⁸ bilden gemeinsam mit dem Stickstoffatom, an das sie gebunden sind, einen 5- oder 6-gliedrigen Ring, der ein Sauerstoffatom enthalten kann, insbesondere stehen R⁷ und R⁸ zusammen für (CH₂)₄, (CH₂)₅ oder (CH₂)₂O(CH₂)₂), C₁-C₄-Alkylthio (insbesondere Methylthio), C₁-C₄-Alkylsulfonyl (insbesondere CH₃SO₂), die Heterocyclylreste Dioxanyl, Dioxolanyl, Morpholinyl, Triazolinonyl, Oxazolinyl, 5,6-Dihydro-[1,3,4]-oxadiazin-2-yl, 5,6-Dihydro-[1,4,2]-dioxazin-3-yl und Pyrazolinonyl (insbesondere1,3-Dioxolan-2-yl, 1,3-Dioxan-2-yl und 1,2,4-Triazolin-3-on-2-yl) (welche selbst wiederum substituiert sein können durch C₁-C4-Alkyl (insbesondere Methyl), C₁-C₄-Halogenalkyl (insbesondere CF₃) und C₁-C₄-Alkoxy-C₁-C₄-alkyl (insbesondere Methoxymethyl)), Phenyl (welches selbst wiederum substituiert sein kann durch Halogen (insbesondere Fluor, Chlor), die Heteroarylreste Pyridyl, Pyridyl-N-oxid, Pyrimidinyl, Pyrazolyl, Thiazolyl, Furanyl, Thienyl, Triazolyl, Oxadiazolyl, Thiadiazolyl, Pyrazinyl, Triazinyl, und Isochinolinyl (insbesondere Pyrid-2-yl, Pyrimid-2-yl, Pyrimid-4-yl, Pyrazol-1-yl, Pyrazol-2-yl, 1,2,4-Oxadiazol-3-yl, 1,2,4-Triazol-3-yl und 1,2,4-Triazin-3-yl) (welche selbst wiederum substituiert sein können durch Halogen (insbesondere Fluor und Chlor), Nitro, C₁-C₄-Alkyl (insbesondere Methyl, Ethyl, n-Propyl, i-Propyl und tert.-Butyl), C₁-C₄-Halogenalkyl (insbesondere CF₃, CHF₂ und CFClH), C₁-C₄-Alkoxy (insbesondere Methoxy, Ethoxy) und C₃-C₆-Cycloalkyl (insbesondere Cyclopropyl, Cyclobutyl, Cyclopentyl)), die Heteroarylalkylreste Triazolyl-C₁-C₄-alkyl (insbesondere Triazolylmethyl), Pyridyl-C₁-C₄-alkyl (insbesondere Pyridylmethyl), Pyrimidinyl-C₁-C₄-alkyl (insbesondere Pyrimidinylmethyl) oder Oxadiazolyl-C₁-C₄-alkyl (insbesondere Oxadiazolylmethyl (welche selbst wiederum substituiert sein können durch C₁-C₄-Alkyl (insbesondere Methyl)) und
- b: für 1 oder 2 und insbesondere für 1 steht.

Durch Halogen (auch "Halo" abgekürzt) substituierte Reste, z.B. Halogenalkyl (Haloalkyl), sind einfach oder mehrfach bis zur maximal möglichen Substituentenzahl halogeniert. In Begriffen wie "Halogenalkoxyalkyl" können sich die Halogenatome sowohl im Alkoxy- als auch im Alkyl-Teil des Restes befinden, wenn nicht ausdrücklich etwas anderes vermerkt ist. Bei mehrfacher Halogenierung können die Halogenatome gleich oder verschieden sein. Halogen steht dabei für Fluor, Chlor, Brom und Iod, insbesondere für Fluor, Chlor und Brom, hervorgehoben für Fluor und Chlor.

Der Rest "Pyrimidyl" wird auch als "Pyrimidinyl" bezeichnet.

Bevorzugt, besonders bevorzugt, ganz besonders bevorzugt und ganz besonders hervorgehoben verwendet werden Verbindungen, welche jeweils die unter bevorzugt, besonders bevorzugt, ganz besonders bevorzugt und ganz besonders hervorgehoben genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt.

Erfindungsgemäß besonders bevorzugt verwendet werden Verbindungen der Formel (I), in welchen eine Kombination der vorstehend als besonders bevorzugt aufgeführten Bedeutungen vorliegt.

Erfindungsgemäß ganz besonders bevorzugt verwendet werden Verbindungen der Formel (I), in welchen eine Kombination der vorstehend als ganz besonders bevorzugt aufgeführten Bedeutungen vorliegt.

Erfindungsgemäß ganz besonders hervorgehoben verwendet werden Verbindungen der Formel (I), in welchen eine Kombination der vorstehend als ganz besonders hervorgehoben aufgeführten Bedeutungen vorliegt.

In einer hervorgehobenen Gruppe von erfindungsgemäß zu verwendenden Verbindungen der Formel (I) steht G¹ für CH.

In einer weiteren hervorgehobenen Gruppe von erfindungsgemäß zu verwendenden Verbindungen der Formel (I) steht G¹ für N.

In einer weiteren hervorgehobenen Gruppe von erfindungsgemäß zu verwendenden Verbindungen der Formel (I) steht G¹ für C-Halogen.

In einer weiteren hervorgehobenen Gruppe von erfindungsgemäß zu verwendenden Verbindungen der Formel (I) steht G¹ für CH und R¹ für Wasserstoff.

In einer weiteren hervorgehobenen Gruppe von erfindungsgemäß zu verwendenden Verbindungen der Formel (I) steht G¹ für N und R¹ für Wasserstoff.

In einer weiteren hervorgehobenen Gruppe von erfindungsgemäß zu verwendenden Verbindungen der Formel (I) steht G¹ für C-Halogen und R¹ für Wasserstoff.

In einer weiteren hervorgehobenen Gruppe von erfindungsgemäß zu verwendenden Verbindungen der Formel (I) steht G¹ für CH und R¹ für Methyl.

In einer weiteren hervorgehobenen Gruppe von erfindungsgemäß zu verwendenden Verbindungen der Formel (I) steht G¹ für N und R¹ für Methyl.

In einer weiteren hervorgehobenen Gruppe von erfindungsgemäß zu verwendenden Verbindungen der Formel (I) steht G¹ für C-Halogen und R¹ für Methyl.

In einer weiteren hervorgehobenen Gruppe von erfindungsgemäß zu verwendenden Verbindungen der Formel (I) steht G² für D- R³_{b}.

In einer weiteren hervorgehobenen Gruppe von erfindungsgemäß zu verwendenden Verbindungen der Formel (I) steht G² für D- R³_{b} und b für 1.

Die vorliegende Erfindung betrifft ferner neue Verbindungen der Formel (I) 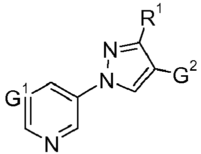 worin
- G¹: für N, CH, C-Halogen C-Nitro, C-Cyano, C-Alkyl, C-Haloalkyl, C-Cycloalkyl, C-Alkoxy, C-Haloalkoxy steht,
- R¹: für Wasserstoff, Alkyl Haloalkyl, Cycloalkyl, Halogen, Cyano, Alkoxy, Haloalkoxy, Amino, Alkylamino, Dialkylamino oder Alkylthio steht,
- G²: für A- R²ₐ, D- R³_{b} oder E- R⁴_{c} steht, worin
- A: für Heterocyclyl aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, Oxazolin-2-yl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 5,6-Dihydro-[1,3,4]-oxadiazin-2-yl, 5,6-Dihydro-[1,3,4]-thiadiazin-2-yl, 5,6-Dihydro-[1,4,2]-dioxazin-3-yl, Hydroxypyridyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2 , 3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-l-yl, 3,4-Dihydropyrazol-3-yl, 3 , 4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl steht,
- R²: für einen Rest aus der Reihe Halogen, Cyano, Nitro, Alkyl, Halogenalkyl, gegebenenfalls substituiertes Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, gegebenenfalls substituiertes Cycloalkylalkyl, Alkylcarbonyl, Alkoxycarbonyl, Aminocarbonyl, Pyridyl und Pyrimidyl (wobei Pyridyl und Pyrimidyl selbst wiederum substituiert sein können durch Halogen, Alkyl, Halogenalkyl, Alkoxy und Halogenalkoxy) steht,
- a: für eine Zahl aus der Reihe 0, 1, 2 und 3 steht,
- D: für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl) steht,
- R³: für einen Rest aus der Reihe gegebenenfalls substituiertes Cycloalkyl, gegebenenfalls substituiertes Cycloalkenyl, gegebenenfalls substituiertes Cycloalkylalkyl, wobei im Cycloalkylteil des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, Bis(alkoxy)alkyl, Bis(halogenalkoxy)alkyl, Alkoxy(alkylsulfanyl)alkyl, Alkoxy(alkylsulfinyl)alkyl, Alkoxy(alkylsulfonyl)alkyl, Bis(alkylsulfanyl)alkyl, Bis(halogenalkylsulfanyl)alkyl, Bis(hydroxyalkylsulfanyl)alkyl, Alkoxycarbonylalkyl, alpha-Hydroxyimino-alkoxycarbonylalkyl, alpha-Alkoxyimino-alkoxycarbonylalkyl, C(X)NR⁵R⁶, (worin X für Schwefel, oder NOH steht, R⁵ für Wasserstoff oder Alkyl steht und R⁶ für einen Rest aus der Reihe Wasserstoff, Alkyl, Halogenalkyl, Cyanoalkyl, Alkinyl, Cycloalkyl, Cycloalkylalkyl, Alkylcarbonyl, Alkoxyalkyl, Alkoxycarbonyl, Alkoxycarbonylalkyl, Alkylthioalkyl, Aryl (insbesondere Phenyl), Arylalkyl (insbesondere Benzyl) und Hetarylalkyl (insbesondere 2-Pyrimidylmethyl) stehen oder X für Schwefel, NOH, NR¹⁵ oder Sauerstoff steht und R⁵ und R⁶ gemeinsam mit dem Stickstoffatom, an das sie gebunden sind, einen Ring bilden, der gegebenenfalls ein oder mehrere weitere Heteroatome aus der Reihe NH, NCH₃, NC₂H₅, Sauerstoff und Schwefel enthält, wobei zwei Sauerstoffatome nicht unmittelbar benachbart sein dürfen oder R⁵ und R¹⁵ gemeinsam mit den Stickstoffatomen, an die sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen), NR⁷R⁸ (worin R⁷ für Wasserstoff oder Alkyl steht und R⁸ für Alkinyl, Cycloalkylalkyl, Alkoxy, Halogenalkoxy, Alkoxycarbonylalkyl, Alkylthioalkyl, Arylalkyl oder Hetarylalkyl steht oder R⁷ und R⁸ gemeinsam mit dem Stickstoffatom, an das sie gebunden sind, einen Ring bilden, der gegebenenfalls ein oder mehrere weitere Heteroatome aus der Reihe NH, NCH₃, NC₂H₅, Sauerstoff und Schwefel enthält, wobei zwei Sauerstoffatome nicht unmittelbar benachbart sein dürfen), die Heterocyclylreste Cycloalkyl und Cycloalkenyl, in welchen eine oder zwei CH₂-Gruppen durch Sauerstoff oder Schwefel ersetzt sind, wobei zwei Sauerstoffatome nicht unmittelbar benachbart sein dürfen, insbesondere Dioxanyl, Dioxolanyl, Dioxepanyl, Dioxocanyl, Oxathianyl, Oxathiolanyl, Oxathiepanyl, Oxathiocanyl, Dithianyl, Dithiolanyl, Dithiepanyl, Dithiocanyl, Oxathianyloxid, Oxathiolanyloxid, Oxathiepanyloxid, Oxathiocanyloxid, Oxathianyldioxid, Oxathiolanyldioxid, Oxathiepanyldioxid, Oxathiocanyldioxid (wobei in diesen Heterocyclylresten zwei am selben C-Atom gebundene H-Atome durch =CH₂, -CH₂-CH₂-, -CH₂-CH₂-CH₂- oder - CH₂-CH₂-CH₂-CH₂- ersetzt sein können), Morpholinyl, Triazolinonyl, Oxazolinyl, Dihydrooxadiazinyl, Dihydrodioxazinyl, Dihydrooxazolyl, Dihydrooxazinyl und Pyrazolinonyl (wobei alle Heterocyclylreste selbst wiederum substituiert sein können durch Alkyl, Halogenalkyl, Alkoxy und Alkoxyalkyl), Phenyl (welches selbst wiederum substituiert sein kann durch Halogen, Cyano, Nitro, Alkyl und Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl) und die Heteroarylalkylreste Triazolylalkyl, Pyridylalkyl, Pyrimidylalkyl und Oxadiazolylalkyl (welche selbst wiederum substituiert sein können durch Alkyl),
- b: für eine Zahl aus der Reihe 1, 2 und 3 steht,
- E: für Aryl, insbesondere Phenyl, steht,
- R⁴: für einen Rest aus der Reihe Halogen, Nitro, Amino, Formyl, Cyano, Alkylamino, Halogenalkylamino, Dialkylamino, Alkyl, Halogenalkyl, gegebenenfalls substituiertes Cycloalkyl, gegebenenfalls substituiertes Cycloalkenyl, gegebenenfalls substituiertes Cycloalkylalkyl, wobei im Cycloalkylteil des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, Alkoxy, Halogenalkoxy, Alkoxyalkyl, halogeniertes Alkoxyalkyl, Bis(alkoxy)alkyl, Bis(halogenalkoxy)alkyl, Alkoxy(alkylsulfanyl)alkyl, Alkoxy(alkylsulfinyl)alkyl, Alkoxy(alkylsulfonyl)alkyl, Bis(alkylsulfanyl)alkyl, Bis(halogenalkylsulfanyl)alkyl, Bis(hydroxyalkylsulfanyl)alkyl, Alkoxycarbonyl, Alkoxycarbonylalkyl, alpha-Hydroxyimino-alkoxycarbonylalkyl, alpha-Alkoxyiminoalkokycarbonylalkyl, C(X)NR⁵R⁶ (worin X für Sauerstoff, Schwefel, NR¹⁵ oder NOH steht, R⁵ für Wasserstoff oder Alkyl steht und R⁶ und R¹⁵ unabhängig voneinander für einen Rest aus der Reihe Wasserstoff, Alkyl, Halogenalkyl, Cyanoalkyl, Alkinyl, Cycloalkyl, Cycloalkylalkyl, Alkylcarbonyl, Alkoxyalkyl, Alkoxycarbonyl, Alkoxycarbonylalkyl, Alkylthioalkyl, Aryl, Arylalkyl und Hetarylalkyl stehen und R6 auch für OH stehen kann oder R⁵ und R⁶ gemeinsam mit dem Stickstoffatom, an das sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen oder R⁵ und R¹⁵ gemeinsam mit den Stickstoffatomen, an das sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen), NR⁷R⁸ (worin R⁷ für Wasserstoff oder Alkyl steht und R⁸ für einen Rest aus der Reihe Wasserstoff, Alkyl, Halogenalkyl, Cyanoalkyl, Alkinyl, Cycloalkyl, Cycloalkylalkyl, Alkoxy, Halogenalkoxy, Alkoxyalkyl, Alkoxycarbonyl, Alkoxycarbonylalkyl, Alkylthioalkyl, Aryl, Arylalkyl oder Hetarylalkyl steht oder R⁷ und R⁸ gemeinsam mit dem Stickstoffatom, an das sie gebunden sind, einen Ring bilden, der ein oder mehrere weitere Heteroatome aus der Reihe NH, NCH₃, NC₂H₅, Sauerstoff und Schwefel enthalten kann), Alkylthio, Alkylsulfinyl, Alkylsulfonyl, die Heterocyclylreste Cycloalkyl und Cycloalkenyl, in welchen eine oder zwei CH₂-Gruppen durch Sauerstoff oder Schwefel ersetzt sind, wobei zwei Sauerstoffatome nicht unmittelbar benachbart sein dürfen, insbesondere Dioxanyl, Dioxolanyl, Dioxepanyl, Dioxocanyl, Oxathianyl, Oxathiolanyl, Oxathiepanyl, Oxathiocanyl, Dithianyl, Dithiolanyl, Dithiepanyl, Dithiocanyl, Oxathianyloxid, Oxathiolanyloxid, Oxathiepanyloxid, Oxathiocanyloxid, Oxathianyldioxid, Oxathiolanyldioxid, Oxathiepanyldioxid, Oxathiocanyldioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können), Morpholinyl, Triazolinonyl, Oxazolinyl, Dihydrooxadiazinyl, Dihydrodioxazinyl, Dihydrooxazolyl, Dihydrooxazinyl und Pyrazolinonyl (wobei alle Heterocyclylreste selbst wiederum substituiert sein können durch Alkyl, Halogenalkyl, Alkoxy und Alkoxyalkyl), Phenyl (welches selbst wiederum substituiert sein kann durch Halogen, Cyano, Nitro, Alkyl und Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl) und die Heteroarylalkylreste Triazolylalkyl, Pyridylalkyl, Pyrimidylalkyl und Oxadiazolylalkyl (welche selbst wiederum substituiert sein können durch Halogen und Alkyl),
und
- c: für eine Zahl aus der Reihe 0, 1, 2 und 3 steht,
sowie deren Salze, Metallkomplexe und N-Oxide.

Bevorzugt sind neue Verbindungen der Formel (I), worin
- G¹: für N, CH, C-Halogen, C-Nitro, C-Cyano, C-(C₁-C₆)-Alkyl, C-(C₁-C₆)-Haloalkyl, C-(C₃-C₆)-Cycloalkyl, C-(C₁-C₆)-Alkoxy, C-(C₁-C₆)-Haloalkoxy, insbesondere für N, CH, C-Halogen, C-Cyano oder C-Trifluoromethyl steht,
- R¹: für Wasserstoff, C₁-C₆-Alkyl, C₁-C₆-Haloalkyl, C₃-C₆-Cycloalkyl, Halogen, Cyano, C₁-C₆-Alkoxy, C₁-C₆-Haloalkoxy, Amino, C₁-C₆-Alkylamino, Di-(C₁-C₆)-alkylamino oder C₁-C₆-Alkylthio, insbesondere für Wasserstoff und Methyl steht und
- G²: für A- R²ₐ, D- R³_{b} oder E- R⁴, steht, worin
- A: für Heterocyclyl aus der Reihe Oxazolin-2-yl, 5,6-Dihydro-[1,3,4]-oxadiazin-2-yl, 5,6-Dihydro-[1,3,4]-thiadiazin-2-yl, 5,6-Dihydro-[1,4,2]-dioxazin-3-yl und Hydroxypyridyl steht,
- R²: für einen Rest aus der Reihe Halogen, Cyano, Nitro, C₁-C₆-Alkyl, C₁-C₆-Halogenalkyl, gegebenenfalls durch Halogen, Cyano, C₁-C₆-Alkyl, C₁-C₆-Halogenalkyl und C₁-C₆-Alkoxy substituiertes C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, gegebenenfalls durch Halogen, Cyano, C₁-C₆-Alkyl, C₁-C₆-Halogenalkyl und C₁-C₆-Alkoxy substituiertes C₃-C₆-Cycloalkyl-C₁-C₆-alkyl, C₁-C₆-Alkylcarbonyl, C₁-C₆-Alkoxycarbonyl, Aminocarbonyl, Pyridyl und Pyrimidyl (wobei Pyridyl und Pyrimidyl selbst wiederum substituiert sein können durch Halogen, C₁-C₆-Alkyl, C₁-C₆-Halogenalkyl, C₁-C₆-Alkoxy und C₁-C₆-Halogenalkoxy) steht,
- a: für eine Zahl aus der Reihe 0, 1, 2 und 3 steht,
- D: für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl) steht,
- R³: für einen Rest aus der Reihe gegebenenfalls durch Halogen, Cyano, C₁-C₆-Alkyl, C₁-C₆-Halogenalkyl und C₁-C₆-Alkoxy substituiertes C₃-C₆-Cycloalkyl, gegebenenfalls durch Halogen, Cyano, C₁-C₆-Alkyl, C₁-C₆-Halogenalkyl und C₁-C₆-Alkoxy substituiertes C₃-C₆-Cycloalkenyl, gegebenenfalls durch Halogen, Cyano, C₁-C₆-Alkyl, C₁-C₆-Halogenalkyl und C₁-C₆-Alkoxy substituiertes C₃-C₆-Cycloalkyl-C₁-C₆-alkyl, wobei im Cycloalkylteil, sofern 5- oder 6-gliedrig, des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, Bis(C₁-C₆-halogenalkoxy)-C₁-C₆-alkyl, C₁-C₆-Alkoxy(C₁-C₆-alkylsulfanyl)-C₁-C₆-alkyl, C₁-C₆-Alkoxy(C₁-C₆-alkylsulfinyl)-C₁-C₆-alkyl, C₁-C₆-Alkoxy(C₁-C₆-alkylsulfonyl)-C₁-C₆-alkyl, Bis(C₁-C₆-alkylsulfanyl)-C₁-C₆-alkyl, Bis(C₁-C₆-halogenalkylsulfanyl)-C₁-C6-alkyl, Bis(hydroxyalkylsulfanyl)-C₁-C₆-alkyl, C₁-C₆-Alkoxycarbonyl-C₁-C₆-alkyl, alpha-Hydroxyimino-C₁-C₆-alkoxycarbonylmethyl, alpha-C₁-C₆-Alkoxyimino-C₁-C₆-alkoxycarbonylmethyl, C(X)NR⁵R⁶, (worin X für Schwefel, oder NOH steht, R⁵ für Wasserstoff oder C₁-C₆-Alkyl steht und R⁶ für einen Rest aus der Reihe Wasserstoff, C₁-C₆-Akl, C₁-C₆-Halogenakl, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxycarbonyl, C₁-C₆-Alkoxy-C₁-C₆-alkyl, C₁-C₆-Alkoxycarbonyl-C₁-C₆-alkyl, C₁-C₆-Alkylthio-C₁-C₆-alkyl, Phenyl-C₁-C₆-alkyl und Hetaryl-C₁-C₆-alkyl steht, worin Hetaryl für Pyrimidyl steht oder X für Schwefel, NR¹⁵, NOH oder Sauerstoff steht und R⁵ und R⁶ gemeinsam mit dem Stickstoffatom, an das sie gebunden sind, einen 5- bis 7-gliedrigen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen oder R⁵ und R¹⁵ gemeinsam mit den Stickstoffatomen, an das sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen), NR⁷R⁸ (worin R⁷ für Wasserstoff oder C₁-C₆-Alkyl steht und R⁸ für einen Rest aus der Reihe C₂-C₆-Alkinyl, C₃-C₆-Cycloalkyl-Cᵢ-C₆-alkyl, C₁-C₆-Alkoxy, C₁-C₆-Halogenalkoxy, C₁-C₆-Alkoxycarbonyl-C₁-C₆-alkyl, C₁-C₆-Alkylthio-C₁-C₆-alkyl, Phenyl-C₁-C₆-alkyl und Hetaryl-C₁-C₆-alkyl steht, worin Hetaryl für Pyrimidyl steht oder R⁷ und R⁸ gemeinsam mit dem Stickstoffatom, an das sie gebunden sind, einen 5- bis 7-gliedrigen Ring bilden, der ein weiteres Heteroatom aus der Reihe NH, NCH₃, NC₂H₅, Sauerstoff und Schwefel enthalten kann), die Heterocyclylreste C₃-C₈-Cycloalkyl und C₄-C₈-Cycloalkenyl, in welchen eine oder zwei CH₂-Gruppen durch Sauerstoff oder Schwefel ersetzt sind, wobei zwei Sauerstoffatome nicht unmittelbar benachbart sein dürfen, insbesondere Dioxanyl, Dioxolanyl, Dioxepanyl, Dioxocanyl, Oxathianyl, Oxathiolanyl, Oxathiepanyl, Oxathiocanyl, Dithianyl, Dithiolanyl, Dithiepanyl, Dithiocanyl, Oxathianyloxid, Oxathiolanyloxid, Oxathiepanyloxid, Oxathiocanyloxid, Oxathianyldioxid, Oxathiolanyldioxid, Oxathiepanyldioxid, Oxathiocanyldioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können), Morpholinyl, Triazolinonyl, Oxazolinyl, Dihydrooxadiazinyl, Dihydrodioxazinyl, Dihydrooxazolyl, Dihydrooxazinyl und Pyrazolinonyl (insbesondere 1,3-Dioxolan-2-yl, 1,3-Dioxan-2-yl, 1,3-Dioxepan-2-yl, 1,3-Dioxocan-2-yl, 1,3-Oxathian-2-yl, 1,3-Oxathiolan-2-yl, 1,3-Oxathiepan-2-yl, 1,3-Oxathiocan-2-yl, 1,3-Dithian-2-yl, 1,3-Dithiolan-2-yl, 1,3-Dithiepan-2-yl, 1,3-Dithiocan-2-yl, 4,5-Dihydro-1,3-oxazol-2-yl, 5,6-Dihydro-4H-1,3-oxazin-2-yl, 1,3-Oxathianyl-3-oxid, 1,3-Oxathiolanyl-3-oxid, 1,3-Oxathiepanyl-3-oxid, 1,3-Oxathiocanyl-3-oxid, 1,3-Oxathianyl-3,3-dioxid, 1,3-Oxathiolanyl-3,3-dioxid, 1,3-Oxathiepanyl-3,3-dioxid, 1,3-Oxathiocanyl-3,3-dioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂-ersetzt sein können) und 1,2,4-Triazolin-3-on-2-yl) (wobei alle Heterocyclylreste selbst wiederum substituiert sein können durch C₁-C₆-Alkyl, C₁-C₆-Halogenalkyl, C₁-C₆-Alkoxy und C₁-C₆-Alkoxy-C₁-C₆-alkyl), Phenyl (welches selbst wiederum substituiert sein kann durch Halogen, Cyano, Nitro, C₁-C₆-Alkyl und C₁-C₆-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl (insbesondere Pyrid-2-yl, Pyrimid-2-yl, Pyrimid-4-yl, Pyrazol-1-yl, Pyrazol-3-yl, Pyrazol-5-yl, 1,2,4-Oxadiazol-3-yl, 1,2,4-Triazol-3-yl, 1,2,3-Triazol-1-yl, 1,2,3-Triazol-2-yl, 1,2,4-Thiadiazol-3-yl und 1,2,4-Triazin-3-yl) (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl) und die Heteroarylalkylreste Triazolyl-C₁-C₆-alkyl, Pyridyl-C₁-C₆-alkyl, Pyrimidyl-C₁-C₆-alkyl und Oxadiazolyl-C₁-C₆-alkyl (welche selbst wiederum substituiert sein können durch C₁-C₆-Alkyl) steht,
- b: für eine Zahl aus der Reihe 1, 2 und 3 steht,
- E: für Phenyl steht,
- R⁴: für einen Rest aus der Reihe Halogen, Nitro, Amino, Formyl, Cyano, C₁-C₆-Alkylamino, C₁-C₆-Halogenalkylamino Di-(C₁-C₆-alkyl)amino, C₁-C₆-Alkyl, C₁-C₆-Halogenalkyl, gegebenenfalls durch Halogen, Cyano, C₁-C₆-Alkyl, C₁-C₆-Halogenalkyl und C₁-C₆-Alkoxy substituiertes C₃-C₆-Cycloalkyl, gegebenenfalls durch Halogen, Cyano, C₁-C₆-Alkyl, C₁-C₆-Halogenalkyl und C₁-C₆-Alkoxy substituiertes C₃-C₆-Cycloalkenyl, gegebenenfalls durch Halogen, Cyano, C₁-C₆-Alkyl, C₁-C₆-Halogenalkyl und C₁-C₆-Alkoxy substituiertes C₃-C₆-Cycloalkyl-C₁-C₆-alkyl, wobei im Cycloalkylteil, sofern 5- oder 6-gliedrig, des Cycloalkylalkylrestes eine oder zwei CH₂-Gruppen durch Sauerstoff oder Schwefel ersetzt sein können, aber zwei Sauerstoffatome nicht unmittelbar benachbart sein dürfen" C₁-C₆-Alkoxy, C₁-C₆-Halogenalkoxy, C₁-C₆-Alkoxy-C₁-C₆-alkyl, halogeniertes C₁-C₆-Alkoxy-C₁-C₆-alkyl, Bis(C₁-C₆-alkoxy)-C₁-C₆-alkyl, Bis(C₁-C₆-halogenalkoxy)-C₁-C₆-alkyl, C₁-C₆-Alkoxy(C₁-C₆-alkylsulfanyl)-C₁-C₆-alkyl, C₁-C₆-Alkoxy(C₁-C₆-alkylsulfinyl)-C₁-C₆-alkyl, C₁-C₆-Alkoxy(C₁-C₆-alkylsulfonyl)-C₁-C₆-alkyl, Bis(C₁-C₆-alkylsulfanyl)-C₁-C₆-alkyl, Bis(C₁-C₆-halogenalkylsulfanyl)-C₁-C₆-alkyl, Bis(C₁-C₆-hydroxyalkylsulfanyl)-C₁-C₆-alkyl, C₁-C₆-Alkoxycarbonyl, C₁-C₆-Alkoxycarbonyl-C₁-C₆-alkyl, alpha-Hydroxyimino-C₁-C₆-alkoxycarbonylmethyl, alpha- C₁-C₆-Alkoxyimino-C₁-C₆-alkoxycarbonylmethyl, C(X)NR⁵R⁶, (worin X für Sauerstoff, Schwefel, NR¹⁵ oder NOH steht, R⁵ für Wasserstoff oder C₁-C₆-Alkyl steht und R⁶ und R¹⁵ unabhängig voneinander für einen Rest aus der Reihe Wasserstoff, C₁-C₆-Alkyl, C₁-C₆-Halogenalkyl, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkylcarbonyl, C₁-C₆-Alkoxy-C₁-C₆-alkyl, C₁-C₆-Alkoxycarbonyl, C₁-C₆-Alkoxycarbonyl-C₁-C₆-alkyl, C₁-C₆-Alkylthio-C₁-C₆-alkyl, Phenyl, Phenyl-C₁-C₆-alkyl oder Hetaryl-C₁-C₆-alkyl stehen, worin Hetaryl für Pyrimidyl steht und R6 auch für OH stehen kann oder R⁵ und R⁶ gemeinsam mit dem Stickstoffatom, an das sie gebunden sind, einen 5- bis 7-gliedrigen Ring bilden, der ein weiteres Heteroatom aus der Reihe NH, NCH₃, NC₂H₅, Sauerstoff und Schwefel enthalten kann wobei zwei Sauerstoffatome nicht unmittelbar benachbart sein dürfen oder R⁵ und R¹⁵ gemeinsam mit den Stickstoffatomen, an die sie gebunden sind, einen 5- bis 7-gliedrigen Ring bilden, der ein oder mehrere, insbesondere ein oder zwei, weitere Heteroatome aus der Reihe NH, NCH₃, NC₂H₅, Sauerstoff und Schwefel enthalten kann, wobei zwei Sauerstoffatome nicht unmittelbar benachbart sein dürfen), NR⁷R⁸ (worin R⁷ für Wasserstoff oder C₁-C₆-Alkyl steht und R⁸ für einen Rest aus der Reihe Wasserstoff, C₁-C₆-Alkyl, C₁-C₆-Halogenalkyl, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy, C₁-C₆-Halogenalkoxy, C₁-C₆-Alkoxy-C₁-C₆-alkyl, C₁-C₆-Alkoxycarbonyl, C₁-C₆-Alkoxycarbonyl-C₁-C₆-alkyl, C₁-C₆-Alkylthio-C₁-C₆-alkyl, Phenyl, Phenyl-C₁-C₆-alkyl und Hetaryl-C₁-C₆-alkyl steht, worin Hetaryl für Pyrimidyl steht oder R⁷ und R⁸ gemeinsam mit dem Stickstoffatom, an das sie gebunden sind, einen 5- bis 7-gliedrigen Ring bilden, der ein weiteres Heteroatom aus der Reihe NH, NCH₃, NC₂H₅, Sauerstoff und Schwefel enthalten kann), C₁-C₆-Alkylthio, C₁-C₆-Alkylsulfinyl, C₁-C₆-Alkylsulfonyl, die Heterocyclylreste C₃-C₈-Cycloalkyl und C₄-C₈-Cycloalkenyl, in welchen eine oder zwei CH₂-Gruppen durch Sauerstoff oder Schwefel ersetzt sind, insbesondere Dioxanyl, Dioxolanyl, Dioxepanyl, Dioxocanyl, Oxathianyl, Oxathiolanyl, Oxathiepanyl, Oxathiocanyl, Dithianyl, Dithiolanyl, Dithiepanyl, Dithiocanyl, Oxathianyloxid, Oxathiolanyloxid, Oxathiepanyloxid, Oxathiocanyloxid, Oxathianyldioxid, Oxathiolanyldioxid, Oxathiepanyldioxid, Oxathiocanyldioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können), Morpholinyl, Triazolinonyl, Oxazolinyl, Dihydrooxadiazinyl, Dihydrodioxazinyl, Dihydrooxazolyl, Dihydrooxazinyl und Pyrazolinonyl (insbesondere 1,3-Dioxolan-2-yl, 1,3-Dioxan-2-yl, 1,3-Dioxepan-2-yl, 1,3-Dioxocan-2-yl, 1,3-Oxathian-2-yl, 1,3-Oxathiolan-2-yl, 1,3-Oxathiepan-2-yl, 1,3-Oxathiocan-2-yl, 1,3-Dithian-2-yl, 1,3-Dithiolan-2-yl, 1,3-Dithiepan-2-yl, 1,3-Dithiocan-2-yl, 4,5-Dihydro-1,3-oxazol-2-yl, 5,6-Dihydro-4H-1,3-oxazin-2-yl, 1,3-Oxathianyl-3-oxid, 1,3-Oxathiolanyl-3-oxid, 1,3-Oxathiepanyl-3-oxid, 1,3-Oxathiocanyl-3-oxid, 1,3-Oxathianyl-3,3-dioxid, 1,3-Oxathiolanyl-3,3-dioxid, 1,3-Oxathiepanyl-3,3-dioxid, 1,3-Oxathiocanyl-3,3-dioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂-ersetzt sein können) und 1,2,4-Triazolin-3-on-2-yl) (wobei alle Heterocyclylreste selbst wiederum substituiert sein können durch C₁-C₆-Alkyl, C₁-C₆-Halogenalkyl, C₁-C₆-Alkoxy und C₁-C₆-Alkoxy-C₁-C₆-alkyl), Phenyl (welches selbst wiederum substituiert sein kann durch Halogen, Cyano, Nitro, C₁-C₆-Alkyl und C₁-C₆-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl (insbesondere Pyrid-2-yl, Pyrimid-2-yl, Pyrimid-4-yl, Pyrazol-1-yl, Pyrazol-3-yl, Pyrazol-5-yl, 1,2,4-Oxadiazol-3-yl, 1,2,4-Triazol-3-yl, 1,2,3-Triazol-1-yl, 1,2,3-Triazol-2-yl, 1,2,4-Thiadiazol-3-yl und 1,2,4-Triazin-3-yl) (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl) und die Heteroarylalkylreste Triazolyl-C₁-C₆-alkyl, Pyridyl-C₁-C₆-alkyl, Pyrimidyl-C₁-C₆-alkyl und Oxadiazolyl-C₁-C₆-alkyl (welche selbst wiederum substituiert sein können durch Halogen und C₁-C₆-Alkyl) steht und
- c: für eine Zahl aus der Reihe 0, 1, 2 und 3 steht,
sowie Salze, Metallkomplexe und N-Oxide der Verbindungen der Formel (I).

Besonders bevorzugt sind neue Verbindungen der Formel (I), worin
- G¹: für N, CH, C-Halogen, C-Nitro, C-Cyano, C-(C₁-C₄)-Alkyl, C-(C₁-C₄)-Haloalkyl, C-(C₃-C₆)-Cycloalkyl, C-(C₁-C₄)-Alkoxy, C-(C₁-C₄)-Haloalkoxy, insbesondere für N, CH, C-Halogen, C-Cyano oder C-Trifluoromethyl steht,
- R¹: für Wasserstoff, C₁-C₄-Alkyl, C₁-C₄-Haloalkyl, C₃-C₆-Cycloalkyl, Halogen, Cyano, C₁-C₄-Alkoxy, C₁-C₄-Haloalkoxy, Amino, C₁-C₄-Alkylamino, Di-(C₁-C₄)-alkylamino oder C₁-C₄-Alkylthio, insbesondere für Wasserstoff und Methyl steht,
- G²: für A- R²ₐ, D- R³_{b} oder E- R⁴_{c}, steht, worin
- A: für Heterocyclyl aus der Reihe Oxazolin-2-yl, 5,6-Dihydro-[1,3,4]-oxadiazin-2-yl 5,6-Dihydro-[1,3,4]-thiadiazin-2-yl und 5,6-Dihydro-[1,4,2]-dioxazin-3-yl steht,
- R²: für einen Rest aus der Reihe Halogen, Cyano, Nitro, C₁-C₄-Alkyl, C₁-C₄-Halogenalkyl, gegebenenfalls durch Fluor, Chlor, Cyano, Methyl, Methoxy und Trifluormethyl substituiertes C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, gegebenenfalls durch Fluor, Chlor, Cyano, Methyl, Methoxy und Trifluormethyl substituiertes C₃-C₆-Cycloalkyl-C₁-C₄-alkyl, C₁-C₄-Alkylcarbonyl, C₁-C₄-Alkoxycarbonyl, Aminocarbonyl, Pyridyl und Pyrimidyl (wobei Pyridyl und Pyrimidyl selbst wiederum substituiert sein können durch Halogen, C₁-C₄-Alkyl, C₁-C₄-Halogenalkyl, C₁-C₄-Alkoxy und C₁-C₄-Halogenalkoxy) steht,
- a: für eine Zahl aus der Reihe 0, 1, 2 und 3 steht,
- D: für einen Heteroarylrest aus der Reihe Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl, 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl (insbesondere Pyrazol-1-yl, Pyrazol-3-yl, Pyrid-2-yl, Pyrid-3-yl, Pyrimid-2-yl, Pyrimid-4-yl, Thiazol-2-yl und Thiazol-4-yl) steht,
- R³: für einen Rest aus der Reihe gegebenenfalls durch Fluor, Chlor, Cyano, Methyl, Methoxy und Trifluormethyl substituiertes C₃-C₆-Cycloalkyl, gegebenenfalls durch Fluor, Chlor, Cyano, Methyl, Methoxy und Trifluormethyl substituiertes C₃-C₆-Cycloalkenyl, gegebenenfalls durch Fluor, Chlor, Cyano, Methyl, Methoxy und Trifluormethyl substituiertes C₃-C₆-Cycloalkyl-C₁-C₄-alkyl, wobei im Cycloalkylteil, sofern 5- oder 6-gliedrig, des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, Bis(C₁-C₄-halogenalkoxy)-C₁-C₄-alkyl, C₁-C₄-Alkoxy(C₁-C₄-aklsulfanyl)-C₁-C₄-alkyl, C₁-C₄-Alkoxy(C₁-C₄-alkylsulfinyl)-C₁-C₄-alkyl, C₁-C₄-Alkoxy(C₁-C₄-alkylsulfonyl)-C₁-C₄-alkyl, Bis(C₁-C₄-alkylsulfanyl)-C₁-C₄-alkyl, Bis(C₁-C₄-halogenalkylsulfanyl)-C₁-C₄-alkyl, Bis(hydroxyalkylsulfanyl)-C₁-C₄-alkyl, C₁-C₄-Alkoxycarbonyl-C₁-C₄-alkyl, alpha-Hydroxyimino-C₁-C₄-alkoxycarbonylmethyl, alpha- C₁-C₄-Alkoxyimino-C₁-C₄-alkoxycarbonylmethyl, C(X)NR⁵R⁶, (worin X für Schwefel steht, R⁵ für Wasserstoff oder C₁-C₄-Alkyl steht und R⁶ für einen Rest aus der Reihe Wasserstoff, C₁-C₅-Alkyl, C₁-C₄-Halogenalkyl, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl oder Phenyl-C₁-C₄-alkyl steht oder X für Schwefel oder Sauerstoff steht und R⁵ und R⁶ gemeinsam mit dem Stickstoffatom, an das sie gebunden sind, einen 5- bis 7-gliedrigen Ring bilden, der ein weiteres Heteroatom aus der Reihe NH, NCH₃, NC₂H₅, Sauerstoff und Schwefel enthalten kann), NR⁷R⁸ (worin R⁷ für Wasserstoff oder C₁-C₄-Alkyl steht und R⁸ für einen Rest aus der Reihe C₂-C₄-Alkinyl, C₃-C₆-Cycloalkyl-C₁-C₄-alkyl, C₁-C₄-Alkoxy, C₁-C₄-Halogenalkoxy, C₁-C₄-Alkoxycarbonyl-C₁-C₄-alkyl, C₁-C₄-Aklthio-C₁-C₄-alkyl oder Phenyl-C₁-C₄-alkyl steht oder R⁷ und R⁸ gemeinsam mit dem Stickstoffatom, an das sie gebunden sind, einen 5- oder 6-gliedrigen Ring bilden, der ein weiteres Heteroatom aus der Reihe Sauerstoff, Schwefel, NH, NCH₃ und NC₂H₅ enthalten kann), die Heterocyclylreste Dioxanyl, Dioxolanyl, Dioxepanyl, Oxathianyl, Oxathiolanyl, Oxathiepanyl, Dithianyl, Dithiolanyl, Dithiepanyl, Oxathianyloxid, Oxathiolanyloxid, Oxathiepanyloxid, Oxathianyldioxid, Oxathiolanyldioxid, Oxathiepanyldioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können), Morpholinyl, Triazolinonyl, Oxazolinyl, Dihydrooxadiazinyl, Dihydrodioxazinyl, Dihydrooxazolyl, Dihydrooxazinyl und Pyrazolinonyl (insbesondere 1,3-Dioxolan-2-yl, 1,3-Dioxan-2-yl, 1,3-Dioxepan-2-yl, 1,3-Oxathian-2-yl, 1,3-Oxathiolan-2-yl, 1,3-Oxathiepan-2-yl, 1,3-Dithian-2-yl, 1,3-Dithiolan-2-yl, 1,3-Dithiepan-2-yl, 4,5-Dihydro-1,3-oxazol-2-yl, 5,6-Dihydro-4H-1,3-oxazin-2-yl, 1,3-Oxathianyl-3-oxid, 1,3-Oxathiolanyl-3-oxid, 1,3-Oxathiepanyl-3-oxid, 1,3-Oxathianyl-3,3-dioxid, 1,3-Oxathiolanyl-3,3-dioxid, 1,3-Oxathiepanyl-3,3-dioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können) und 1,2,4-Triazolin-3-on-2-yl) (wobei alle Heterocyclylreste selbst wiederum substituiert sein können durch C₁-C₄-Alkyl, C₁-C₄-Halogenalkyl und C₁-C₄-Alkoxy-C₁-C₄-alkyl), Phenyl (welches selbst wiederum substituiert sein kann durch Halogen, Cyano, Nitro, C₁-C₄-Alkyl und C₁-C₄-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl (insbesondere Pyrid-2-yl, Pyrimid-2-yl, Pyrimid-4-yl, Pyrazol-1-yl, Pyrazol-3-yl, Pyrazol-5-yl, 1,2,4-Oxadiazol-3-yl, 1,2,4-Triazol-3-yl, 1,2,3-Triazol-1-yl, 1,2,3-Triazol-2-yl, 1,2,4-Thiadiazol-3-yl, Tetrazolyl und 1,2,4-Triazin-3-yl) (welche selbst wiederum substituiert sein können durch Halogen, Nitro, C₁-C₄-Alkyl, C₁-C₄-Halogenalkyl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl) und die Heteroarylalkylreste Triazolyl-C₁-C₄-alkyl, Pyridyl-C₁-C₄-alkyl, Pyrimidyl-C₁-C₄-alkyl oder Oxadiazolyl-C₁-C₄-alkyl (welche selbst wiederum substituiert sein können durch Halogen und C₁-C₄-Alkyl),
- b: für eine Zahl aus der Reihe 1 und 2 steht,
- E: für Phenyl steht,
- R⁴: für einen Rest aus der Reihe Halogen, Cyano, C₁-C₄-Alkyl, C₁-C₄-Halogenalkyl, C₁-C₄-Alkoxy und C₁-C₄-Halogenalkoxy steht und
- c: für eine Zahl aus der Reihe 0, 1 und 2 steht.

Ganz besonders bevorzugt sind neue Verbindungen der Formel (I), worin
- G¹: für N, CH, C-Halogen, C-Cyano, C-CH₃, C-CF₃, C-Cyclopropyl, C-OCH₃, C-OCF₃, insbesondere für N, CH, C-Halogen steht,
- R¹: für Wasserstoff, Methyl, Trifluoromethyl, Cyclopropyl, Halogen, Cyano, Methoxy, Trifluormethoxy, Amino, Methylamino, Dimethylamino, insbesondere für Wasserstoff steht,
- G²: für A- R²ₐ oder D- R³_{b} steht, worin
- A: für Heterocyclyl aus der Reihe Oxazolin-2-yl, 5,6-Dihydro-[1,3,4]-thiadiazin-2-yl und 5,6-Dihydro-[1,3,4]-oxadiazin-2-yl steht,
- R²: für einen Rest aus der Reihe Pyridyl und Pyrimidin-2-yl steht, welche durch Halogen, C₁-C₄-Alkyl, C₁-C₄-Halogenalkyl, C₁-C₄-Alkoxy und C₁-C₄-Halogenalkoxy substituiert sein können,
- a: für 0, 1 oder 2 und insbesondere für 1 steht,
- D: für einen Heteroarylrest aus der Reihe Pyrazol-1-yl, Pyrazol-3-yl, Pyrid-2-yl, Pyrid-3-yl, Pyrimid-2-yl, Pyrimid-4-yl, Thiazol-2-yl und Thiazol-4-yl steht,
- R³: für einen Rest aus der Reihe gegebenenfalls durch Fluor, Chlor, Cyano, Methyl, Methoxy und Trifluormethyl substituiertes C₃-C₆-Cycloalkyl (insbesondere Cyclopropylmethyl), gegebenenfalls durch Fluor, Chlor, Cyano, Methyl, Methoxy und Trifluormethyl substituiertes C₃-C₆-Cycloalkenyl, gegebenenfalls durch Fluor, Chlor, Cyano, Methyl, Methoxy und Trifluormethyl substituiertes C₃-C₆-Cycloalkyl-C₁-C₄-alkyl, wobei im Cycloalkylteil, sofern 5- oder 6-gliedrig, des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, alpha-Hydroxyimino-C₁-C₄-alkoxycarbonylmethyl, C(X)NR⁵R⁶, (worin X für Schwefel steht, R⁵ für Wasserstoff steht und R⁶ für einen Rest aus der Reihe Wasserstoff, C₁-C₅-Alkyl (insbesondere Methyl, Ethyl, n-Propyl, i-Propyl, iso-Butyl, tert.-Butyl, C₁-C₄-Halogenalkyl (insbesondere CF₃CH₂), Cyano-C₁-C₄-alkyl (insbesondere NCCH₂CH(C₂H₅), C₃-C₆-Cycloalkyl (insbesondere Cyclopropyl, Cyclopentyl, Cyclohexyl), C₃-C₆-Cycloalkyl-C₁-C₄-alkyl (insbesondere Cyclopropylmethyl), C₁-C₄-Alkoxy-C₁-C₄-alkyl (insbesondere CH₃OCH₂CH(CH₃), CH₃CH₂CH₂OCH₂CH(CH₃), CH₃CH₂OCH₂CH₂, CH₃OCH₂CH₂CH₂, CH₃OCH₂CHC₂H₅, CH₃CH₂OCH₂CH(CH₃), CH₃CH₂OCH₂CH₂CH₂, CH₃OC(CH₃)₂, C₁-C₄-Alkylthio-C₁-C₄-alkyl (insbesondere CH₃SCH₂CH₂) und Phenyl-C₁-C₄-alkyl (insbesondere C₆H₅CH(CH₃)) steht oder X für Schwefel oder Sauerstoff steht und R⁵ und R⁶ gemeinsam mit dem Stickstoffatom, an das sie gebunden sind, einen 5- bis 7-gliedrigen Ring bilden, der gegebenenfalls ein weiteres Heteroatom aus der Reihe NH, NCH₃, NC₂H₅, Sauerstoff und Schwefel enthält (insbesondere stehen R⁵ und R⁶ zusammen für (CH₂)₄, (CH₂)₅ oder (CH₂)₂O(CH₂)₂)), NR⁷R⁸ (worin R⁷ für Wasserstoff oder Methyl steht und R⁸ für C₁-C₄-Alkoxycarbonyl-C₁-C₄-alkyl (insbesondere CH₂CO₂CH₃) steht oder R⁷ und R⁸ bilden gemeinsam mit dem Stickstoffatom, an das sie gebunden sind, einen 5- oder 6-gliedrigen Ring, der ein Sauerstoffatom enthalten kann, beispielsweise stehen R⁷ und R⁸ zusammen für (CH₂)₄, (CH₂)₅ oder (CH₂)₂O(CH₂)₂), die Heterocyclylreste Dioxanyl, Dioxolanyl, Oxathianyl, Oxathiolanyl, Dithianyl, Dithiolanyl, Oxathianyloxid, Oxathiolanyloxid, Oxathianyldioxid, Oxathiolanyldioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können), Morpholinyl, Triazolinonyl, Oxazolinyl, Dihydrooxadiazinyl, Dihydrodioxazinyl, Dihydrooxazolyl, Dihydrooxazinyl und Pyrazolinonyl (insbesondere 1,3-Dioxolan-2-yl, 1,3-Dioxan-2-yl, 1,3-Oxathian-2-yl, 1,3-Oxathiolan-2-yl, 1,3-Dithian-2-yl, 1,3-Dithiolan-2-yl, 4,5-Dihydro-1,3-oxazol-2-yl, 5,6-Dihydro-4H-1,3-oxazin-2-yl, 1,3-Oxathianyl-3-oxid, 1,3-Oxathiolanyl-3-oxid, 1,3-Oxathianyl-3,3-dioxid, 1,3-Oxathiolanyl-3,3-dioxid (wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können) und 1,2,4-Triazolin-3-on-2-yl) (wobei alle Heterocyclylreste selbst wiederum substituiert sein können durch C₁-C₄-Alkyl (insbesondere Methyl), C₁-C₄-Halogenalkyl (insbesondere CF₃) und C₁-C₄-Alkoxy-C₁-C₄-alkyl (insbesondere Methoxymethyl)), Phenyl (welches selbst wiederum substituiert sein kann durch Halogen (insbesondere Fluor, Chlor), die Heteroarylreste Pyridyl, Pyridyl-N-oxid, Pyrimidinyl, Pyrazolyl, Thiazolyl, Furanyl, Thienyl, Triazolyl, Tetrazolyl, Oxadiazolyl, Thiadiazolyl, Pyrazinyl, Triazinyl, und Isochinolinyl (insbesondere Pyrid-2-yl, Pyrimid-2-yl, Pyrimid-4-yl, Pyrazol-1-yl, Pyrazol-3-yl, Pyrazol-5-yl, 1,2,4-Oxadiazol-3-yl, 1,2,4-Triazol-3-yl, 1,2,3-Triazol-1-yl, 1,2,3-Triazol-2-yl, 1,2,4-Thiadiazol-3-yl, Tetrazolyl und 1,2,4-Triazin-3-yl) (welche selbst wiederum substituiert sein können durch Halogen (insbesondere Fluor und Chlor), Nitro, C₁-C₄-Alkyl (insbesondere Methyl, Ethyl, n-Propyl, i-Propyl und tert.-Butyl), C₁-C₄-Halogenalkyl (insbesondere CF₃, CHF₂ und CF₂Cl), C₁-C₄-Alkoxy (insbesondere Methoxy, Ethoxy) und C₃-C₆-Cycloalkyl (insbesondere Cyclopropyl, Cyclobutyl, Cyclopentyl)), die Heteroarylalkylreste Triazolyl-C₁-C₄-alkyl (insbesondere Triazolylmethyl), Pyridyl-C₁-C₄-alkyl (insbesondere Pyridylmethyl), Pyrimidinyl-C₁-C₄-alkyl (insbesondere Primidinylmethyl) oder Oxadiazolyl-C₁-C₄-alkyl (insbesondere Oxadiazolylmethyl (welche selbst wiederum substituiert sein können durch C₁-C₄-Alkyl (insbesondere Methyl)) und
- b: für 1 oder 2 und insbesondere für 1 steht.

Die Erfindung betrifft auch neue Verbindungen der Formel (I), worin
- G¹: für N, CH oder C-Halogen steht,
- R¹: für Wasserstoff oder Alkyl steht,
- G²: für A- R²ₐ, D- R³_{b} oder E- R⁴, steht, worin
- A: für Heterocyclyl aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, Oxazolin-2-yl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 5,6-Dihydro-[1,3,4]-oxadiazin-2-yl, 5,6-Dihydro-[1,3,4]-thiadiazin-2-yl, 5,6-Dihydro-[1,4,2]-dioxazin-3-yl, Hydroxypyridyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3 , 4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4 , 5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl steht,
- R²: für einen Rest aus der Reihe Halogen, Cyano, Nitro, Alkyl, Halogenalkyl, Cycloalkyl, Alkoxy, Halogenalkoxy, Alkylthio, Haloalkylthio, Alkylsulfinyl, Alkylsulfonyl, Haloalkylsulfi-nyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl, Aminocarbonyl, Pyridyl und Pyrimidyl (wobei Pyridyl und Pyrimidyl selbst wiederum substituiert sein können durch Halogen, Alkyl, Halogenalkyl, Alkoxy und Halogenalkoxy) steht,
- a: für eine Zahl aus der Reihe 0, 1, 2 und 3 steht,
- D: für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl) steht,
- R³: für einen Rest aus der Reihe Cycloalkylalkyl, Alkoxycarbonylalkyl, alpha-Hydroxyiminoalkoxycarbonylalkyl, alpha-Alkoxyimino-alkoxycarbonylalkyl, C(X)NR⁵R⁶, (worin X für Schwefel oder NOH steht, R⁵ für Wasserstoff oder Alkyl steht und R⁶ für einen Rest aus der Reihe Wasserstoff, Alkyl, Halogenalkyl, Cyanoalkyl, Alkinyl, Cycloalkyl, Cycloalkylalkyl, Alkoxyalkyl, Alkoxycarbonyl, Alkoxycarbonylalkyl, Alkylthioalkyl, Aryl (insbesondere Phenyl), Arylalkyl (insbesondere Benzyl) und Hetarylalkyl (insbesondere 2-Pyrimidylmethyl) steht oder X für Schwefel, NOH oder Sauerstoff steht und R⁵ und R⁶ gemeinsam mit dem Stickstoffatom, an das sie gebunden sind, einen Ring bilden, der gegebenenfalls ein oder mehrere weitere Heteroatome aus der Reihe Stickstoff, Sauerstoff und Schwefel enthält), NR⁷R⁸ (worin R⁷ für Wasserstoff oder Alkyl steht und R⁸ für Alkinyl, Cycloalkylalkyl, Alkoxy, Halogenalkoxy, Alkoxycarbonylalkyl, Alkylthioalkyl, Arylalkyl oder Hetarylalkyl steht oder R⁷ und R⁸ gemeinsam mit dem Stickstoffatom, an das sie gebunden sind, einen Ring bilden, der gegebenenfalls ein oder mehrere weitere Heteroatome aus der Reihe Stickstoff, Sauerstoff und Schwefel enthält), die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (welche selbst wiederum substituiert sein können durch Alkyl, Halogenalkyl, Alkoxy und Alkoxyalkyl), Phenyl (welches selbst wiederum substituiert sein kann durch Halogen, Cyano, Nitro, Alkyl und Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl) und die Heteroarylalkylreste Triazolylalkyl, Pyridylalkyl, Pyrimidylalkyl und Oxadiazolylalkyl (welche selbst wiederum substituiert sein können durch Alkyl),
- b: für eine Zahl aus der Reihe 1, 2 und 3 steht,
- E: für Aryl, insbesondere Phenyl, steht,
- R⁴: für einen Rest aus der Reihe Halogen, Cyano, Alkyl, Halogenalkyl, Alkoxy, Halogenalkoxy, Dioxolanyl oder Dihydrodioxazinyl steht und
- c: für eine Zahl aus der Reihe 0, 1, 2 und 3 steht,
sowie deren Salze, Metallkomplexe und N-Oxide.

Bevorzugt sind auch neue Verbindungen der Formel (I), worin
- G¹: für N, CH oder C-Halogen steht,
- R¹: für Wasserstoff oder C₁-C₆-Alkyl steht und
- G²: für A- R²ₐ, D- R³_{b} oder E- R⁴, steht, worin
- A: für Heterocyclyl aus der Reihe Oxazolin-2-yl, 5,6-Dihydro-[1,3,4]-oxadiazin-2-yl, 5,6-Dihydro-[1,3,4]-thiadiazin-2-yl, 5,6-Dihydro-[l,4,2]-dioxazin-3-yl und Hydroxypyridyl steht,
- R²: für einen Rest aus der Reihe Halogen, Cyano, Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl, Aminocarbonyl, Pyridyl und Pyrimidyl (wobei Pyridyl und Pyrimidyl selbst wiederum substituiert sein können durch Halogen, C₁-C₆-Alkyl, C₁-C₆-Halogenalkyl, C₁-C₆-Alkoxy und C₁-C₆-Halogenalkoxy) steht,
- a: für eine Zahl aus der Reihe 0, 1, 2 und 3 steht,
- D: für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl (insbesondere Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl) steht,
- R³: für einen Rest aus der Reihe C₃-C₆-Cycloalkyl-C₁-C₆-alkyl, C₁-C₆-Alkoxycarbonyl-C₁-C₆-alkyl, alpha-Hydroxyimino-C₁-C₆-alkoxycarbonylmethyl, alpha-C₁-C₆-Alkoxyimino-C₁-C₆-alkoxycarbonylmethyl, C(X)NR⁵R⁶, (worin X für Schwefel oder NOH steht, R⁵ für Wasserstoff oder C₁-C₆-Alkyl steht und R⁶ für einen Rest aus der Reihe Wasserstoff, C₁-C₆-Alkyl, C₁-C₆-Halogenalkyl, Cyano-C₁-C₆-alkyl, C₂-C₆-Alkinyl, C₃-C₆-Cycloalkyl, C₃-C₆-Cycloalkyl-C₁-C₆-alkyl, C₁-C₆-Alkoxy-C₁-C₆-alkyl, C₁-C₆-Alkoxycarbonyl-C₁-C₆-alkyl, C₁-C₆-Alkylthio-C₁-C₆-alkyl, Phenyl-C₁-C₆-alkyl und Hetaryl-C₁-C₆-alkyl steht, worin Hetaryl für Pyrimidyl steht oder X für Schwefel, NOH oder Sauerstoff steht und R⁵ und R⁶ gemeinsam mit dem Stickstoffatom, an das sie gebunden sind, einen 5- bis 7-gliedrigen Ring bilden, der ein oder mehrere weitere Heteroatome aus der Reihe Stickstoff, Sauerstoff und Schwefel enthalten kann), NR⁷R⁸ (worin R⁷ für Wasserstoff oder C₁-C₆-Alkyl steht und R⁸ für einen Rest aus der Reihe C₂-C₆-Alkinyl, C₃-C₆-Cycloalkyl-C₁-C₆-alkyl, C₁-C₆-Alkoxy, C₁-C₆-Halogenalkoxy, C₁-C₆-Alkoxycarbonyl-C₁-C₆-alkyl, C₁-C₆-Alkylthio-C₁-C₆-alkyl, Phenyl-C₁-C₆-alkyl und Hetaryl-C₁-C₆-alkyl steht, worin Hetaryl für Pyrimidyl steht oder R⁷ und R⁸ gemeinsam mit dem Stickstoffatom, an das sie gebunden sind, einen 5- bis 7-gliedrigen Ring bilden, der ein weiteres Heteroatom aus der Reihe Stickstoff, Sauerstoff und Schwefel enthalten kann), die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (welche selbst wiederum substituiert sein können durch C₁-C₆-Alkyl, C₁-C₆-Halogenalkyl, C₁-C₆-Alkoxy und C₁-C₆-Alkoxy-C₁-C₆-alkyl), Phenyl (welches selbst wiederum substituiert sein kann durch Halogen, Cyano, Nitro, C₁-C₆-Alkyl und C₁-C₆-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₆-Alkyl, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio, C₁-C₆-Alkylthio-C₁-C₆-alkyl und C₃-C₆-Cycloalkyl) und die Heteroarylalkylreste Triazolyl-C₁-C₆-alkyl, Pyridyl-C₁-C₆-alkyl, Pyrimidyl-C₁-C₆-alkyl und Oxadiazolyl-C₁-C₆-alkyl (welche selbst wiederum substituiert sein können durch C₁-C₆-Alkyl) steht,
- b: für eine Zahl aus der Reihe 1, 2 und 3 steht,
- E: für Phenyl steht,
- R⁴: für einen Rest aus der Reihe Halogen, Cyano, C₁-C₆-Alkyl, C₁-C₆-Halogenalkyl, C₁-C₆-Alkoxy, C₁-C₆-Halogenalkoxy, Dioxolanyl und Dihydrodioxazinyl steht und
- c: für eine Zahl aus der Reihe 0, 1, 2 und 3 steht.

Besonders bevorzugt sind auch neue Verbindungen der Formel (I), worin
- G¹: für N, CH oder C-Halogen steht,
- R¹: für Wasserstoff steht und
- G²: für A- R²ₐ, D- R³_{b} oder E- R⁴_{c} steht, worin
- A: für Heterocyclyl aus der Reihe Oxazolin-2-yl, 5,6-Dihydro-[1,3,4]-oxadiazin-2-yl 5,6-Dihydro-[1,3,4]-thiadiazin-2-yl und 5,6-Dihydro-[1,4,2]-dioxazin-3-yl steht,
- R²: für einen Rest aus der Reihe Halogen, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Haloalkylthio, C₁-C₄-Alkylsulfinyl, C₁-C₄-Alkylsulfonyl, C₁-C₄-Haloalkylsulfinyl, C₁-C₄-Haloalkylsulfonyl, Amino, C₁-C₄-Alkylamino, Di-(C₁-C₄-akyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl, Aminocarbonyl, Pyridyl und Pyrimidyl (wobei Pyridyl und Pyrimidyl selbst wiederum substituiert sein können durch Halogen, C₁-C₄-Alkyl, C₁-C₄-Halogenalkyl, C₁-C₄-Alkoxy und C₁-C₄-Halogenalkoxy) steht,
- a: für eine Zahl aus der Reihe 0, 1, 2 und 3 steht,
- D: für einen Heteroarylrest aus der Reihe Pyrid-2-yl, Pyrid-3-yl, Pyrimid-2-yl, Pyrimid-4-yl, Imidazolyl, Pyrazol-1-yl, Pyrazol-3-yl Pyrazinyl, 1,2,3-Triazinyl, 1,2,4-Triazinyl, 1,3,5-Triazinyl, Thiazol-2-yl, Thiazol-4-yl, 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl und Oxazolyl (insbesondere Pyrazol-1-yl, Pyrazol-3-yl, Pyrid-2-yl, Pyrid-3-yl, Pyrimid-2-yl, Pyrimid-4-yl, Thiazol-2-yl und Thiazol-4-yl) steht,
- R³: für einen Rest aus der Reihe C₃-C₆-Cycloalkyl-C₁-C₄-alkyl, C₁-C₄-Alkoxycarbonyl-C₁-C₄-alkyl, alpha-Hydroxyimino-C₁-C₄-alkoxycarbonylmethyl, alpha- C₁-C₄-Alkoxyimino-C₁-C₄-alkoxycarbonylmethyl, C(X)NR⁵R⁶, (worin X für Schwefel steht, R⁵ für Wasserstoff oder C₁-C₄-Alkyl steht und R⁶ für einen Rest aus der Reihe Wasserstoff, C₁-C₅-Akl, C₁-C₄-Halogenalkyl, Cyano-C₁-C₄-alkyl, C₂-C₄-Alkinyl, C₃-C₆-Cycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl oder Phenyl-C₁-C₄-alkyl steht oder X für Schwefel oder Sauerstoff steht und R⁵ und R⁶ gemeinsam mit dem Stickstoffatom, an das sie gebunden sind, einen 5- bis 7-gliedrigen Ring bilden, der ein weiteres Heteroatom aus der Reihe Stickstoff, Sauerstoff und Schwefel enthalten kann), NR⁷R⁸ (worin R⁷ für Wasserstoff oder C₁-C₄-Alkyl steht und R⁸ für einen Rest aus der Reihe C₂-C₄-Alkinyl, C₃-C₆-Cycloalkyl-C₁-C₄-alkyl, C₁-C₄-Alkoxy, C₁-C₄-Halogenalkoxy, C₁-C₄-Alkoxycarbonyl-C₁-C₄-alkyl, C₁-C₄-Alkylthio-C₁-C₄-alkyl oder Phenyl-C₁-C₄-alkyl steht oder R⁷ und R⁸ gemeinsam mit dem Stickstoffatom, an das sie gebunden sind, einen 5- oder 6-gliedrigen Ring bilden, der ein weiteres Heteroatom aus der Reihe Sauerstoff, Schwefel und Stickstoff enthalten kann), die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (insbesondere1,3-Dioxolan-2-yl, 1,3-Dioxan-2-yl und 1,2,4-Triazolin-3-on-2-yl) (welche selbst wiederum substituiert sein können durch C₁-C₄-Alkyl und C₁-C₄-Halogenalkyl), Phenyl (welches selbst wiederum substituiert sein kann durch Halogen, Cyano, Nitro, C₁-C₄-Alkyl oder C₁-C₄-Halogenalkyl), die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Oxadiazolyl, Thiadiazolyl, Pyrazinyl, Triazinyl, Tetrazinyl und Isochinolinyl (welche selbst wiederum substituiert sein können durch Halogen, Nitro, C₁-C₄-Alkyl, C₁-C₄-Halogenakl, C₁-C₄-Alkoxy, C₁-C₄-Halogenalkoxy, C₁-C₄-Alkoxy-C₁-C₄-alkyl, C₁-C₄-Alkylthio, C₁-C₄-Alkylthio-C₁-C₄-alkyl und C₃-C₆-Cycloalkyl) und die Heteroarylalkylreste Triazolyl-C₁-C₄-alkyl, Pyridyl-C₁-C₄-alkyl, Pyrimidyl-C₁-C₄-alkyl und Oxadiazolyl-C₁-C₄-alkyl (welche selbst wiederum substituiert sein können durch C₁-C₄-Alkyl),
- b: für eine Zahl aus der Reihe 1 und 2 steht,
- E: für Phenyl steht und
- R⁴: für einen Rest aus der Reihe Halogen, Cyano, C₁-C₄-Alkyl, C₁-C₄-Halogenalkyl, C₁-C₄-Alkoxy und C₁-C₄-Halogenalkoxy steht und
- c: für eine Zahl aus der Reihe 0, 1 und 2 steht.

Ganz besonders bevorzugt sind auch neue Verbindungen der Formel (I), worin
- G¹: für N, CH oder C-Halogen (im Fall von C-Halogen insbesondere CF und CI) steht,
- R¹: für Wasserstoff steht,
- G²: für A- R²ₐ oder D- R³_{b} steht, worin
- A: für Heterocyclyl aus der Reihe Oxazolin-2-yl, 5,6-Dihydro-[1,3,4]-thiadiazin-2-yl und 5,6-Dihydro-[1,3,4]-oxadiazin-2-yl steht,
- R²: für einen Rest aus der Reihe Pyridyl und Pyrimidin-2-yl steht, welche durch Halogen, C₁-C₄-Alkyl, C₁-C₄-Halogenalkyl, C₁-C₄-Alkoxy und C₁-C₄-Halogenalkoxy substituiert sein können,
- a: für 0, 1 oder 2 und insbesondere für 1 steht,
- D: für einen Heteroarylrest aus der Reihe Pyrazol-1-yl, Pyrazol-3-yl, Pyrid-2-yl, Pyrid-3-yl, Pyrimid-2-yl, Pyrimid-4-yl, Thiazol-2-yl und Thiazol-4-yl steht,
- R³: für einen Rest aus der Reihe C₃-C₆-Cycloalkyl-C₁-C₄-alkyl (insbesondere Cyclopropylmethyl), alpha-Hydroxyimino-C₁-C₄-alkoxycarbonylmethyl, C(X)NR⁵R⁶, (worin X für Schwefel steht, R⁵ für Wasserstoff steht und R⁶ für einen Rest aus der Reihe Wasserstoff, C₁-C₅-Alkyl (insbesondere Methyl, Ethyl, n-Propyl, i-Propyl, iso-Butyl, tert.-Butyl, C₁-C₄-Halogenalkyl (insbesondere CF₃CH₂), Cyano-C₁-C₄-alkyl (insbesondere NCCH₂CH(C₂H₅), C₃-C₆-Cycloalkyl (insbesondere Cyclopropyl, Cyclopentyl, Cyclohexyl), C₃-C₆-Cycloalkyl-C₁-C₄-alkyl (insbesondere Cyclopropylmethyl), C₁-C₄-Alkoxy-C₁-C₄-alkyl (insbesondere CH₃OCH₂CH(CH₃), CH₃CH₂CH₂OCH₂CH(CH₃), CH₃CH₂OCH₂CH₂, CH₃OCH₂CH₂CH₂, CH₃OCH₂CHC₂H₅, CH₃CH₂OCH₂CH(CH₃), CH₃CH₂OCH₂CH₂CH₂, CH₃OC(CH₃)₂, C₁-C₄-Alkylthio-C₁-C₄-alkyl (insbesondere CH₃SCH₂CH₂) und Phenyl-C₁-C₄-alkyl (insbesondere C₆H₅CH(CH₃) steht oder X für Schwefel oder Sauerstoff steht und R⁵ und R⁶ gemeinsam mit dem Stickstoffatom, an das sie gebunden sind, einen 5- bis 7-gliedrigen Ring bilden, der gegebenenfalls ein weiteres Heteroatom aus der Reihe Stickstoff, Sauerstoff und Schwefel enthält (insbesondere stehen R⁵ und R⁶ zusammen für (CH₂)₄, (CH₂)₅ oder (CH₂)₂O(CH₂)₂)), NR⁷R⁸ (worin R⁷ für Wasserstoff oder Methyl steht und R⁸ für C₁-C₄-Alkoxycarbonyl-C₁-C₄-alkyl (insbesondere CH₂CO₂CH₃) steht oder R⁷ und R⁸ bilden gemeinsam mit dem Stickstoffatom, an das sie gebunden sind, einen 5- oder 6-gliedrigen Ring, der ein Sauerstoffatom enthalten kann, beispielsweise stehen R⁷ und R⁸ zusammen für (CH₂)₄, (CH₂)₅ oder (CH₂)₂O(CH₂)₂), die Heterocyclylreste Dioxanyl, Dioxolanyl, Morpholinyl, Triazolinonyl, Oxazolinyl, Dihydrooxadiazinyl, Dihydrodioxazinyl und Pyrazolinonyl (insbesondere1,3-Dioxolan-2-yl, 1,3-Dioxan-2-yl und 1,2,4-Triazolin-3-on-2-yl) (welche selbst wiederum substituiert sein können durch C₁-C₄-Alkyl (insbesondere Methyl) und C₁-C4-Halogenalkyl (insbesondere CF₃)), Phenyl (welches selbst wiederum substituiert sein kann durch Halogen (insbesondere Fluor und Chlor), die Heteroarylreste Pyridyl, Pyridyl-N-oxid, Pyrimidinyl, Pyrazolyl, Thiazolyl, Furanyl, Thienyl, Triazolyl, Oxadiazolyl, Thiadiazolyl, Pyrazinyl, Triazinyl, und Isochinolinyl (welche selbst wiederum substituiert sein können durch Halogen (insbesondere Fluor und Chlor), Nitro, C₁-C₄-Alkyl (insbesondere Methyl, Ethyl, n-Propyl, i-Propyl und tert.-Butyl), C₁-C₄-Halogenalkyl (insbesonere CF₃, CHF₂ und CFClH), C₁-C₄-Alkoxy (insbesondere Methoxy und Ethoxy), C₃-C₆-Cycloalkyl (insbesondere Cyclopropyl, Cyclobutyl und Cyclopentyl)), die Heteroarylalkylreste Triazolyl-C₁-C₄-alkyl (insbesondere Triazolylmethyl), Pyridyl-C₁-C₄-alkyl (insbesondere Pyridylmethyl), Pyrimidinyl-C₁-C₄-alkyl (insbesondere Primidinylmethyl) oder Oxadiazolyl-C₁-C₄-alkyl (insbesondere Oxadiazolylmethyl (welche selbst wiederum substituiert sein können durch C₁-C₄-Alkyl (insbesondere Methyl)) und
- b: für 1 oder 2 und insbesondere für 1 steht.

Die durch a und b bestimmte Zahl von Substituenten kann durch die Zahl der substituierbaren Wasserstoffatome an den Resten A, D und E begrenzt sein.

Erfindungsgemäß bevorzugt sind Verbindungen der Formel (I), in welchen eine Kombination der vorstehend als bevorzugt aufgeführten Bedeutungen vorliegt.

Erfindungsgemäß besonders bevorzugt sind Verbindungen der Formel (I), in welchen eine Kombination der vorstehend als besonders bevorzugt aufgeführten Bedeutungen vorliegt.

Erfindungsgemäß ganz besonders bevorzugt sind Verbindungen der Formel (I), in welchen eine Kombination der vorstehend als ganz besonders bevorzugt aufgeführten Bedeutungen vorliegt.

In einer hervorgehobenen Gruppe von neuen Verbindungen der Formel (I) steht G¹ für CH.

In einer weiteren hervorgehobenen Gruppe von neuen Verbindungen der Formel (I) steht G¹ für N.

In einer weiteren hervorgehobenen Gruppe von neuen Verbindungen der Formel (I) steht G¹ für C-Halogen.

In einer weiteren hervorgehobenen Gruppe von neuen Verbindungen der Formel (I) steht G¹ für CH und R¹ für Wasserstoff.

In einer weiteren hervorgehobenen Gruppe von neuen Verbindungen der Formel (I) steht G¹ für N und R¹ für Wasserstoff.

In einer weiteren hervorgehobenen Gruppe von neuen Verbindungen der Formel (I) steht G¹ für C-Halogen und R¹ für Wasserstoff.

In einer weiteren hervorgehobenen Gruppe von neuen Verbindungen der Formel (I) steht G¹ für CH und R¹ für Methyl.

In einer weiteren hervorgehobenen Gruppe von neuen Verbindungen der Formel (I) steht G¹ für N und R¹ für Methyl.

In einer weiteren hervorgehobenen Gruppe von neuen Verbindungen der Formel (I) steht G¹ für C-Halogen und R¹ für Methyl.

In einer weiteren hervorgehobenen Gruppe von neuen Verbindungen der Formel (I) steht G² für D-R³_{b}.

In einer weiteren hervorgehobenen Gruppe von neuen Verbindungen der Formel (I) steht G² für D-R³_{b} und b für 1.

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die Erfindung betrifft auch Salze, N-Oxide und Metallkomplexe der Verbindungen der Formel (I).

Beispielhaft und ergänzend wird die Herstellung von Verbindungen der Formel (I) in den folgenden Formelschemata erläutert. An dieser Stelle sei auch auf die Herstellungsbeispiele verwiesen. Die Reste G¹ und G² können in den Schemata auch als G₁ und G₂ geschrieben sein.

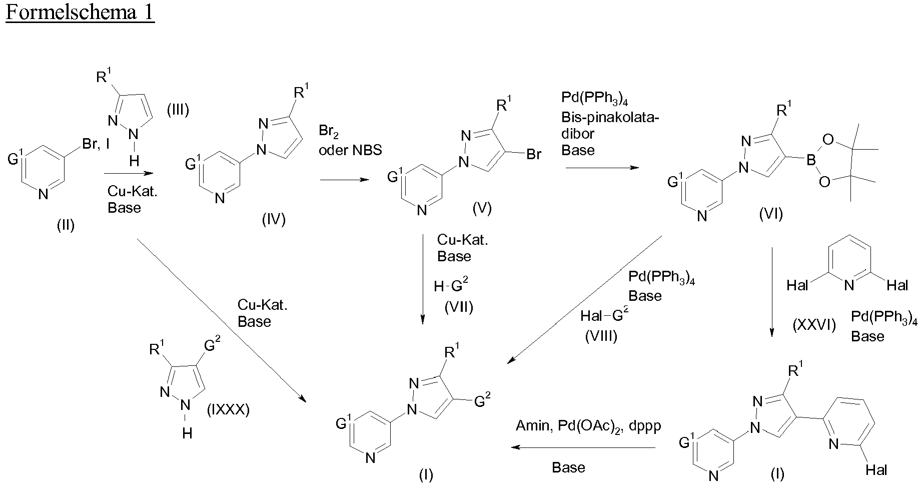

Die Herstellung von erfindungsgemäßen Verbindungen der Formel (I) erfolgt ausgehend von 3-Halogenpyridinen der Formel (II), beispielsweise erhält man durch Umsetzung eines Bromides der Formel (II) mit einem Pyrazol der Formel (III) in Gegenwart eines Kupfer-Katalysators und einer Hilfsbase wie Kaliumcarbonat die Verbindungen der Formel (IV). Siehe beispielsweise für 3-(4-Bromo-pyrazol-1-yl)-pyridin: Journal of Heterocyclic Chemistry 1981, 18, 9-14; European Journal of Organic Chemistry, 2004, 695. Aus diesen Pyrazolen der Formel (IV) werden durch Umsetzung mit Brom oder N-Bromsuccinimid die Bromide der Formel (V) erhalten. Siehe beispielsweise für 3-(4-Bromo-pyrazol-1-yl)-pyridin: Journal of Heterocyclic Chemistry 18, 1981, 9-14. Aus den Bromiden der Formel (V) werden durch Umsetzung mit Bis-Pinakolata-Dibor in Gegenwart eines Palladium-Katalysators und einer Hilfsbase die Boronester der Formel (VI) erhalten. Die erfindungsgemä-ßen Verbindungen der Formel (I) können aus den Bromiden der Formel (V) oder aus den Halogeniden der Formel (II) durch Umsetzung mit einer Verbindung der Formel (VII), die einen Baustein H-G² darstellt, welcher ein N-H enthält, wie beispielsweise einem 3-Hetaryl-pyrazol, in Gegenwart eines Kupferkatalysators und einer Hilfsbase oder nach dem gleichen Verfahren durch Umsetzung der Bromide der Formel (II) mit einem geeigneten Pyrazol der Formel (IXXX) erhalten werden. Weiterhin lassen sich die Halogenpyridine der Formel (I) durch Umsetzung der Boronester der Formel (VI) mit einem Dihalogenpyridin der Formel (XXVI) in Gegenwart eines Palladium-Katalysators und einer Hilfsbase (Suzuki-Reaktion) erhalten. Die Umsetzung der Halogenpyridine der Formel (I) mit Aminen in Gegenwart eines Palladium-Katalysators und einer Hilfsbase liefert analog zu der in Journal of Organic Chemistry 72, 2007, 3606-3607 beschriebenen Methode anschließend diejenigen erfindungsgemäßen Verbindungen der Formel (I), welche in G² das entsprechende Amin tragen (dppp = 1,3-Bis(diphenylphosphino)propan).

Zusätzlich lassen sich die erfindungsgemäßen Verbindungen der Formel (I) durch Umsetzung der Boronester der Formel (VI) mit einem Halogenid der Formel (VIII) in Gegenwart eines Palladium-Katalysators und einer Hilfsbase (Suzuki-Reaktion) erhalten.

Die benötigten Verbindungen der Formel (VII) und (IIX) sind bekannt oder lassen sich nach im Prinzip bekannten Methoden herstellen, siehe dazu die folgenden beispielhaft angeführten Referenzen.

2-(6-Bromo-pyridin-2-yl)-pyrimidin ist in Tetrahedron Letters, 2000, 1653 beschrieben, ein verbessertes Herstellungsverfahren ergibt sich durch die Berücksichtigung der in Tetrahedron Letters 1996 , 2537 beschriebenen Beobachtungen.

Für 6-Brom-pyridin-2-carbonsäuredimethylamid siehe Journal of the Chemical Society, Perkin1, 16, 1996, 1927-1934.

Für (6-Brom-pyridin-2-yl)-morpholin-4-yl-methanon siehe WO 2006/65209.

Für 2-Bromo-6-pyrazol-1-yl-pyridin siehe Journal of Organic Chemistry, 1990, 4992-4994.

Für (6-Bromo-pyridin-2-yl)-carbaminsäure- *tert*-butylester siehe Journal of Medicinal Chemistry, 2005, 1886-1900.

Für [(6-Brom-pyridin-2-yl)-methyl-amino]-essigsäuremethylester siehe US 5,008,275.

Für 2-Brom-6-methoxy-pyridin siehe Journal of the American Chemical Society 1994, 3657-3658.

Für 5-(6-Brom-pyridin-2-yl)-1*H*-pyrimidin-2,4-dion siehe Journal of Heterocyclic Chemistry 32, 4, 1995, 1159-1164.

Für 2-(1*H*-Pyrazol-4-yl)-pyridin siehe Journal of Medicinal Chemistry, 2004, 4645-4648.

Für 3-(1*H*-Pyrazol-4-yl)-pyridin siehe Angewandte Chemie Int. Ed. 2006, 1282-1284.

Für [(Z)-3-Dimethylamino-2-(4-trifluoromethyl-phenyl)-allyliden]-dimethyl-ammoniumperchlorat siehe Bioorganic and Medicinal Chemistry 2008, 2463-2472.

Für [(Z)-3-Dimethylamino-2-(3-trifluoromethyl-phenyl)-allyliden]-dimethyl-ammoniumperchlorat siehe Journal of Organic Chemistry 1995, 3750.

Für (Z)-3-Hydroxy-2-(4-methoxy-phenyl)-propenal siehe Journal of Heterocyclic Chemistry 11, 1974,51,52.

Für (Z)-3-Hydroxy-2-pyrazin-2-yl-propenal siehe Tetrahedron Letters 49, 2, 2008, 305-310.

Für (Z)-3-Hydroxy-2-pyridin-4-yl-propenal siehe Tetrahedron Letters 49, 2, 2008, 305-310.

Für 4-(4-Chloro-phenyl)-1*H*-pyrazol siehe Synthetic Communications 17, 2, 1987, 165-172 oder Journal of Organic Chemistry, 1991, 976; Journal of Heterocyclic Chemistry 28, 1991, 1281-1285.

Die Synthese von 2-Brom-6-(dimethoxymethyl)pyridin ausgehend von 6-Bromopyridin-2-carbaldehyd ergibt sich durch die Berücksichtigung der in WO 2004/37808 beschriebenen Beobachtungen.

Die Synthese von 2-Brom-6-(3,5-dimethyl-1H-pyrazol-1-yl)pyridin ergibt sich ausgehend von 6-Chlor-2-pyridyl)hydrazin und 2,4-Pentadion durch die Berücksichtigung der in WO 2008/104077 beschriebenen Methoden.

Die Synthese von 2-Chlor-6-(2,2,2-trifluorethoxy)pyridin ergibt sich ausgehend von 2,6-Dichlorpyridin durch die Berücksichtigung der in Journal of Fluorine Chemistry 53, 1991 143-153 beschriebenen Methoden.

Die Synthese von 2-Brom-5-(1,1-difluorethyl)pyridin ausgehend von 1-(6-Brompyridin-3-yl)ethanon mit Diethylaminoschwefeltrifluorid (DAST) ergibt sich durch die Berücksichtigung der in WO 2003/93231 beschriebenen Beobachtungen.

2-(6-Brompyridin-2-yl)-4-methyl-5-(trifluormethyl)-2,4-dihydro-3H-1,2,4-triazol-3-on lässt sich ausgehend von 4-Methyl-5-(trifluormethyl)-2,4-dihydro-3H-1,2,4-triazol-3-on durch Kupfer-Diamin-katalysierte N-Arylierung herstellen. Die Synthese wurde analog zu der in Journal of Organie Chemistry 69, 2004, 5578-5587 beschriebenen Methode durchgeführt.

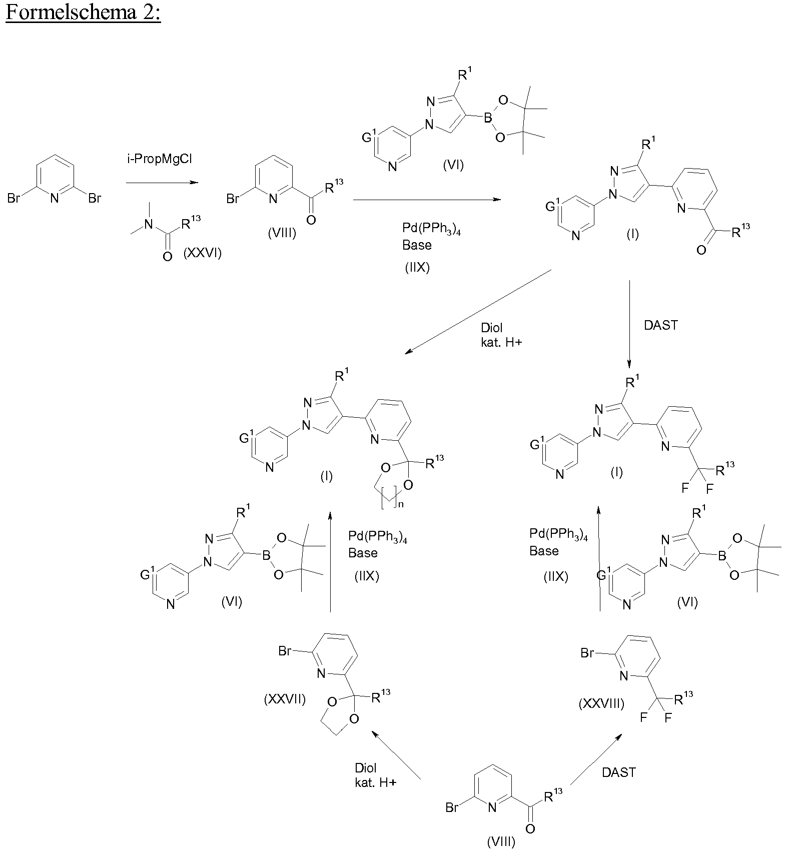

Bestimmte für den in Formelschema 1 beschriebenen Prozeß benötigte Verbindungen der Formel (VIII) des Typs Hal-G² lassen sich gemäß Formelschema 2 erhalten und zu den erfindungsgemäßen Verbindungen der Formel (I) mit einer Gruppe R¹³, die einen Teil der Gruppe R³ bildet, umsetzen. 2,6-Dibrompyridin wird beispielsweise durch Umsetzung mit Isopropylmagnesiumchlorid metalliert, siehe dazu Tetrahedron 2000, 1349; Tetrahedron Letters 1999, 4339; und mit einem Acylierungsmittel wie den Dimethylamiden der Formel (XXVI) zu den Ketonen der Formel (VIII) umgesetzt. Die Dimethylamide der Formel (XXVI) sind bekannt oder lassen sich nach im Prinzip bekannten Methoden herstellen, siehe beispielsweise für 2-Methoxy-*N,N*-dimethyl-acetamid DE 875807; Journal of Organic Chemistry 1974, 1233.

Enstprechend dem in Formelschema 1 beschriebene Prozeß werden aus den Ketonen der Formel (VIII) durch Umsetzung mit den Boronestern der Formel (VI) in einer übergangangsmetallvermittelten Reaktion (beispielsweise durch Suzuki-Reaktion) die Ketone der Formel (I) erhalten, aus denen sich durch Umsetzung mit einem Diol bei Säurekatalyse und Wasserentzug die Ketale der Formel (I) oder durch Umsetzung mit einem Fluorierungsmittel wie DAST die fluorierten Verbindungen der Formel (I) erhalten lassen, siehe dazu WO 2003/93231.

Die Umsetzung von Ketonen der Formel (VIII) mit einem Diol unter Säurekatalyse und Wasserentzug liefert die Ketale der Formel (XXVII), die durch Umsetzung mit den Boronestern der Formel (VI) in einer übergangangsmetallvermittelten Reaktion (beispielsweise durch Suzuki-Reaktion) die Ketale der Formel (I) liefern. Die Umsetzung von Ketonen der Formel (VIII) mit einem Fluorierungsmittel wie DAST liefert die fluorierten Verbindungen der Formel (XXVIII), aus denen sich durch Umsetzung mit den Boronestern der Formel (VI) in einer übergangangsmetallvermittelten Reaktion (beispielsweise durch Suzuki-Reaktion) die fluorierten Verbindungen der Formel (I) erhalten lassen, siehe dazu WO 2003/93231.

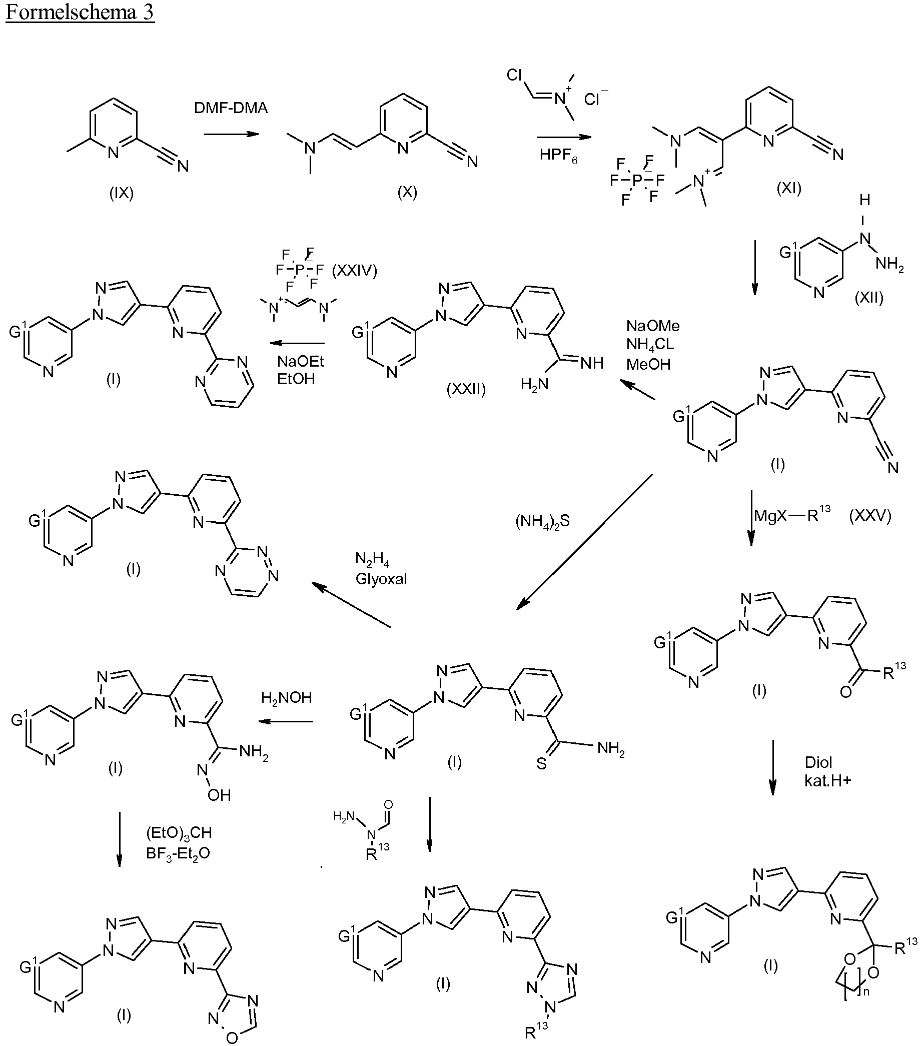

Alternativ zu dem in Formelschema 1 beschriebenen Verfahren können die erfindungsgemäßen Verbindungen der Formel (I) auch gemäß Formelschema 3 erhalten werden. 6-Methyl-pyridin-2-carbonitril (IX) ist bekannt, siehe dazu beispielsweise EP 1 424 336 oder Chem. Pharm Bull. 7, 1959, 925. Durch Umsetzung mit Dimethylformamid-dimethylacetal wird das Enamin (X) erhalten, siehe dazu US 4,176,183 und Synthesis 1997, 696. Durch Umsetzung mit Chloromethylen-dimethylammoniumchlorid, dem Vilsmeier-Reagenz, siehe dazu Organic Synthesis, 1987, 121, wird das Vinamidinium-Salz (XI) erhalten, siehe dazu wiederum Synthesis 1997, 696. Durch Umsetzung mit Pyridylhydrazinen der Formel (XII) werden daraus die Nitrile der Formel (I) erhalten, eine Umsetzung dieser Art ist beschrieben in Tetrahedron Letters 1996, 1829. Pyridylhydrazine der Formel (XII) sind bekannt oder lassen sich nach im Prinzip bekannten Methoden herstellen, siehe beispielsweise EP 1 426 366 oder Liebigs Annalen 486, 1931, 95.

Die Nitrile der Formel (I) werden mit Ammoniumsulfid zu den Thioamiden der Formel (I) umgesetzt. Aus den Thioamiden werden durch Umsetzung mit Hydrazin und Glyoxal die Triazine der Formel (I) erhalten, siehe dazu Chemische Berichte 101, 1968, 3952-3956; European Journal of Organic Chemistry 1999, 313-321; intermediär entsteht das Amidrazon, siehe dazu Chemical Reviews 1970, 70(1), 151-170. Aus den Thioamiden der Formel (I) werden durch Umsetzung mit Formhydraziden, siehe dazu Chemische Berichte 1965, 3377, die Triazole der Formel (I) erhalten.

Aus den Thioamiden der Formel (I) können durch Umsetzung mit Hydroxylamin die Hydroxamsäuren der Formel (I) erhalten werden, siehe dazu Journal of Heterocyclic Chemistry 1980, 819-821; diese werden mit Orthoester zu den Oxdiazolen der Formel (I) umgesetzt, siehe dazu Journal of Medicinal Chemistry 1994, 2421-2436.

Aus den Nitrilen der Formel (I) können durch Umsetzung mit Natriumalkoholat und Ammoniumchlorid die Amidine der Formel (XXII) erhalten werden, aus denen, alternativ zu dem in Formelschema 1 beschriebenen Prozeß, durch Umsetzung mit einem Vinamidiniumsalz der Formel (XXIV) die Pyrimidine der Formel (I) erhalten werden können. Die benötigten Vinamidiniumsalze der Formel (XXIV) sind bekannt oder lassen sich nach im Prinzip bekannten Methoden erhalten, siehe dazu Journal of Organic Chemistry, 1960, 3812; Collect. Czech. Chem. Comm. Vol 61, 1996, 1637.

Aus den Nitrilen der Formel (I) können durch Umsetzung mit einem metallorganischen Reagenz der Formel (XXV) wie beispielsweise einer Gringnardverbindung alternativ zu dem in Formelschema 2 beschriebenen Verfahren die Ketone der Formel (I) erhalten werden, siehe dazu Journal of Heterocyclic Chemistry 1999, 81 und Journal of Organic Chemistry 1987, 3901. Aus diesen Ketonen lassen sich durch Umsetzung mit Diolen bei Säurekatalyse und Wasserentzug die Ketale der Formel (I) erhalten.

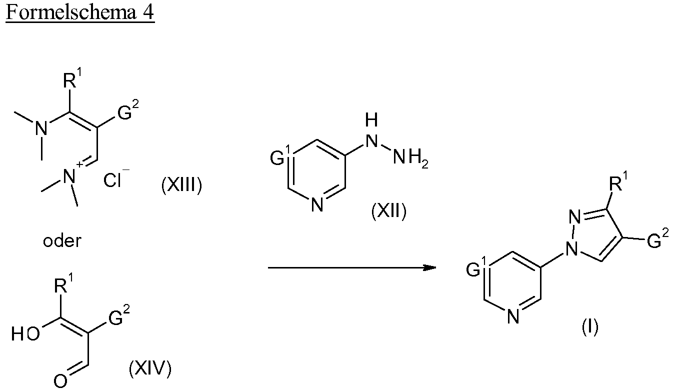

Alternativ zu den in den Formelschemata 1 und 2 beschriebenen Verfahren können die erfindungsgemäßen Verbindungen der Formel (I) auch allgemeiner gemäß Formelschema 4 erhalten werden. Dazu werden die Vinamidiniumsalze der Formel (XIII) oder die Dialdehyde bzw. Enoladehyde der Formel (XIV) mit den Pyridylhydrazinen der Formel (XII) zu den erfindungsgemäßen Verbindungen der Formel (I) umgesetzt. Die Verbindungen der Formel (XIII) und (XIV) sind bekannt oder lassen sich nach im Prinzip bekannten Methoden erhalten, siehe dazu beispielsweise Journal of Heterocyclic Chemistry 28, 1991, 1281; Angewandte Chemie 1976, 496.

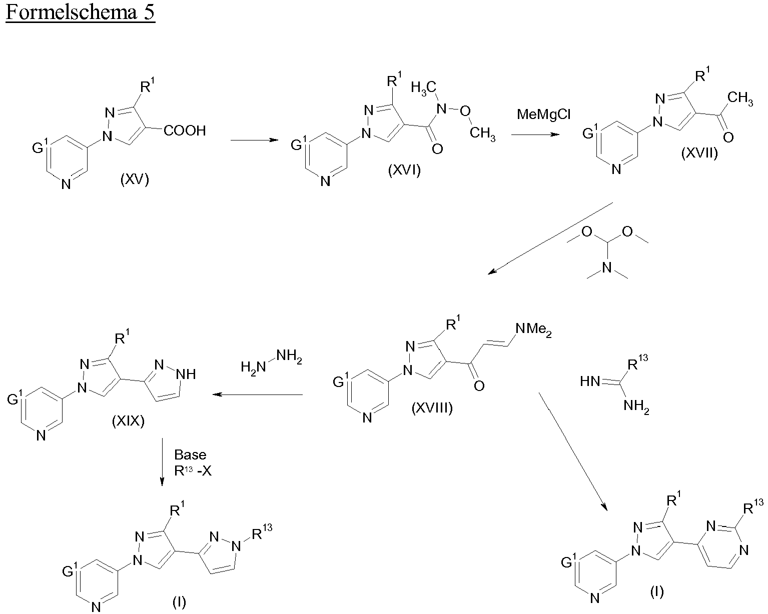

Gemäß Formelschema 5 können diejenigen erfindungsgemäßen Verbindungen der Formel (I) erhalten werden, welche im Rest G² ein Pyrazol oder Pyrimidin tragen. Die Pyrazolcarbonsäuren der Formel (XV) sind bekannt oder lassen sich nach im Prinzip bekannten Methoden herstellen. Beispielsweise wird 1-(Pyridin-3-yl)-1H-pyrazol-4-carbonsäure duch alkalische Esterspaltung aus dem entsprechenden Ethylester erhalten, dessen Herstellung in Journal of Organic Chemistry 2004, 5578 beschrieben ist. Danach werden die Säuren der Formel (XV) mit einem Chlorierungsmittel wie Thionylchlorid in die entsprechenden Säurechloride überführt. Weitere Umsetzung mit *O,N*-Dimethylhydroxylamin in einem Verdünnungsmittel wie beispielsweise Dichlormethan oder Tetrahydrofuran und in Gegenwart einer Base wie beispielsweise Triethylamin oder Diisopropylethylamin, führt zu den Amiden der Formel (XVI), welche durch Umsetzung mit einer Methylmetallverbindung wie Methylmagnesiumchlorid in die Ketone der Formel (XVII) überführt werden können. Durch Umsetzung der Verbindungen der Formel (XVII) mit Dimethylformamid-Dimethylacetal werden die Enaminone der Formel (XVIII) erhalten, siehe dazu Heterocycles, 43,1, 1996, 221 und Journal of Heterocyclic Chemistry 24, 1987, 837.

Aus den Enaminonen der Formel (XVIII) werden durch Umsetzung mit Hydrazinhydrat in einem Verdünnungsmittel wie Ethanol die NH-Pyrazole der Formel (XIX) erhalten. Diese Pyrazole können durch Reaktion mit einem Alkylierungs- oder (Het-)-Arylierungsmittel und einer Hilfsbase wie Natriumhydrid in einem Verdünnungsmittel wie DMF in die erfindungsgemäßen Pyrazole der Formel (I) überführt werden.

Durch Umsetzung der Enaminone der Formel (XVIII) mit Amidinen in Gegenwart einer Hilfsbase wie Natriumethanolat in einem Verdünnungsmittel wie Ethanol sind die erfindungsgemäßen Pyrimidine der Formal (I) erhältlich. Die benötigten Amidine sind bekannt oder nach im Prinzip bekannten Methoden herstellbar.

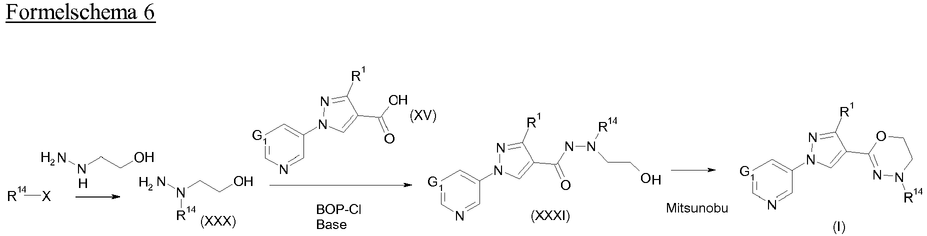

Die erfindungsgemäßen Dihydrooxdiazine der Formel (I) mit einer Gruppe R¹⁴, die einen Teil der Gruppe R² bildet, werden gemäß Formelschema 6 erhalten, indem zunächst 2-Hydrazinoethanol mit einem Alkylierungs-oder (Het-)Arylierungsmittel R¹⁴-X zu Verbindungen der Formel (XXX) umgesetzt wird, eine Reaktion dieser Art ist beschrieben in Khim. Geterosikl. Soedin 1990, 8, 1065. Daraus können mit Säuren der Formel (XV) mit Hilfe eines Aktivierungsreagenzes wie Bis(2-oxo-3-oxazolidinyl)-phosphinsäurechlorid (BOP-C1) oder den enstprechenden Säurechloriden in Gegenwart einer Hilfsbase wie Triethylamin in einem Verdünnungsmittel wie DMF die Hydrazide der Formel (XXXI) erhalten werden, die sich beispielsweise durch Mitsunobu-Reaktion, wie in Heterocycles 37, 3, 1994, 1645 beschrieben, in die erfindungsgemäßen Dihydrooxdiazine der Formel (I) überführen lassen.

Folgende Zwischenprodukte sind neu und auch Gegenstand der Erfindung.

### Verbindungen der Formel (VI)

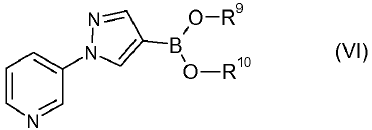

worin
R⁹ und R¹⁰ unabhängig voneinander für H, C₁-C₆-Alkyl (insbesondere Methyl, Ethyl, Propyl und Isopropyl) stehen oder gegebenenfalls zusammen mit den Atomen, an die sie gebunden sind, einen Ring bilden, der bevorzugt keine weiteren Heteroatome enthält und der wiederum einfach oder mehrfach mit C₁-C₆-Alkyl (insbesondere Methyl, Ethyl, Propyl, Isopropyl) substituiert sein kann.

### Verbindungen der Formel (X)

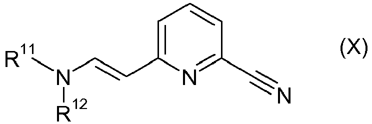

worin
R¹¹ und R¹² für C₁-C₆-Alkyl (insbesondere Methyl, Ethyl, Propyl und Isopropyl) stehen oder zusammen mit dem Stickstoffatom, an das sie gebunden sind, einen Ring bilden (insbesondere Pyrrolidinyl, Piperidinyl und Morpholinyl).

### Verbindungen der Formel (XI)

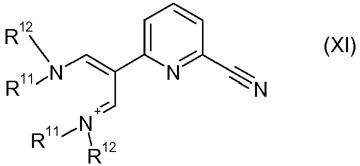

worin
R¹¹ und R¹² für C₁-C₆-Alkyl (insbesondere Methyl, Ethyl, Propyl und Isopropyl) stehen oder zusammen zusammen mit dem Stickstoffatom, an das sie gebunden sind, einen Ring bilden (insbesondere Pyrrolidinyl, Piperidinyl, Morpholinyl)
in Form von Salzen, insbesondere aus der Reihe
- hexafluorophosphat, - perchlorat, - hydrochlorid, -oxalat und - tetrafluoroborat.

### Verbindung der Formel

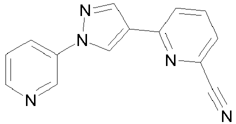

### Verbindung der Formel

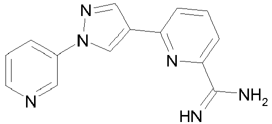

auch in Form ihrer Salze, insbesondere als Amidinium-hydrochlorid und -sulfat.

Die erfindungsgemäßen bekannten und neuen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Omithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden. Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemittel, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Kombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemä-ßen Wirkstoffe mit Mischpartnern synergistische Effekte, d.h. die Wirksamkeit der jeweiligen Mischung ist größer als aufgrund der Wirksamkeit der Einzelkomponenten zu erwarten war. Generell können die Kombinationen sowohl als Saatgutanwendungen als auch in Vor-, Tank- oder Fertigmischungen verwendet werden.

Besonders günstige Mischungspartner sind z.B. die folgenden:

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl *O*-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Organochlorine, z.B. Chlordane und Endosulfan (alpha-); oder
   Fiprole (Phenylpyrazole), z.B. Ethiprole, Fipronil, Pyrafluprole und Pyriprole.
(3) Natrium-Kanal-Modulatoren / Spannungsabhängige Natrium-Kanal-Blocker, wie beispielsweise
   Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; DDT; oder Methoxychlor.
(4) Nikotinerge Acetylcholin-Rezeptor-Agonisten, wie beispielsweise
   Neonikotinoide, z.B. Acetamiprid, Clothianidin, Dinotefuran, Imidacloprid, Nitenpyram, Thiacloprid, Thiamethoxam; oder Nikotin.
(5) Allosterische Acetylcholin-Rezeptor-Modulatoren (Agonisten), wie beispielsweise
   Spinosyne, z.B. Spinetoram und Spinosad.
(6) Chlorid-Kanal-Aktivatoren, wie beispielsweise
   Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise Begasungsmittel, z.B. Methylbromid und andere Alkylhalogenide; oder
   Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis,* und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin, Fenbutatin oxide; oder
   Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
   Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-1-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Chlorantraniliprole (Rynaxypyr), Cyantraniliprole (Cyazypyr) und Flubendiamide.

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen

4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlorpyridin-3-yl)ethyl](methyl)oxido-λ⁴-sulfanylidencyanamid (bekannt aus WO 2007/149134) und seine Diastereomere (A) und (B) 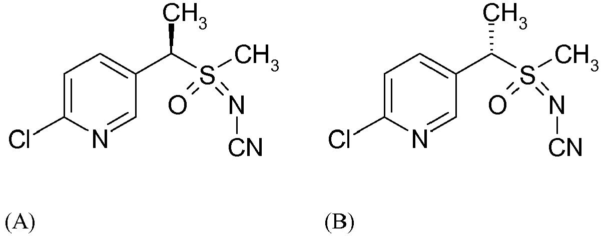 (ebenfalls bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor (ebenfalls bekannt aus WO 2007/149134), 1 1-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911) und 1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/043635).

### Fungizide

(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1 H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-ethyl-2-{2-[(E)-({1-[3-(trifluormethyl)phenyl]ethoxy}imino)methyl]phenyl} ethanamid, (2E)-2- {2-[({[(1E)-1-(3-{[(E)-1-Fluor-2-phenylethenyl]oxy}phenyl)ethyliden]amino}oxy)methyl]phenyl} -2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazo1-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid , N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Penty1-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dimethyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon und N-[2-(4-{[3-(4-Chlorphenyl)prop-2-in-1-yl]oxy}-3-methoxyphenyl)ethyl]-N2-(methylsulfonyl)valinamid.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Jeder zusätzliche Wirkstoff kann in einem weiten Bereich, vorzugsweise in einem Gewichtsverhältnis von 100:1 bis 1:100, besonders bevorzugt von 5 : 1 bis 1 : 5, mit den erfindungsgemäßen Wirkstoffen gemischt werden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen (genannt seien Turf, Reben, Getreide, beispielsweise Weizen, Gerste, Roggen, Hafer, Reis, Mais und Hirse; Rüben, beispielsweise Zuckerrüben und Futterrüben; Früchte, beispielsweise Kernobst, Steinobst und Beerenobst, beispielsweise Äpfel, Birnen, Pflaumen, Pfirsiche, Mandeln, Kirschen und Beeren, z. B. Erdbeeren, Himbeeren, Brombeeren; Hülsenfrüchte, beispielsweise Bohnen, Linsen, Erbsen und Sojabohnen; Ölkulturen, beispielsweise Raps, Senf, Mohn, Oliven, Sonnenblumen, Kokos, Castorölpflanzen, Kakaobohnen und Erdnüsse; Gurkengewächse, beispielsweise Kürbis, Gurken und Melonen; Fasergewächse, beispielsweise Baumwolle, Flachs, Hanf und Jute; Citrusfrüchte, beispielsweise Orangen, Zitronen, Pampelmusen und Mandarinen; Gemüsesorten, beispielsweise Spinat, (Kopf)-Salat, Spargel, Kohlarten, Möhren, Zwiebeln, Tomaten, Kartoffeln und Paprika; Lorbeergewächse, beispielsweise Avocado, Cinnamomum, Kampfer, oder ebenso Pflanzen wie Tabak, Nüsse, Kaffee, Aubergine, Zuckerrohr, Tee, Pfeffer, Weinreben, Hopfen, Bananen, Naturkautschukgewächse sowie Zierpflanzen, beispielsweise Blumen, Sträucher, Laubbäume und Nadelbäume wie Koniferen, wobei diese Aufzählung keine Limitierung darstellt) und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und Cry-IF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizidtolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufinilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp. (Ctenocephalides canis, Ctenocephalides felis), Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

### Beispiel 1:

### Stufe 1: 3-(4-Bromo-pyrazol-1-yl)-pyridin

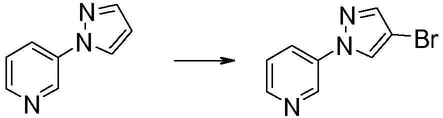

3-Pyrazol-1-yl-pyridin (500 mg, 3,44 mmol) wurde in Acetonitril (15 ml) gelöst und mit Ammoniumcer(IV)nitrat (944 mg, 1,72 mmol) versetzt (leicht exotherm). N-Bromosuccinimid (736 mg, 4,13 mmol) wurde portionsweise zugegeben (leicht exotherm) und die Mischung wurde 30 min bei Raumtemperatur nachgerührt und anschließend 3 Stunden (h) unter Rückfluß erhitzt. Nachdem die Mischung abgekühlt war, wurde Essigester zugegeben. Die organische Phase wurde mit Wasser gewaschen, mit einer Natriumsulfat-Lösung gewaschen und anschließend mit Magnesiumsulfat getrocknet. Das Lösungsmittel wurde im Vakuum am Rotationsverdampfer entfernt.
Ausbeute: 750 mg (93% d.Th), logP (HCOOH) 1,56, [M⁺+1] 224,0
¹H-NMR (d₆-DMSO): 7,54 (m, 1H), 7,90 (s, 1H), 8,20 (m, 1H), 8,55 (m, 1H), 8,79 (s, 1H), 9,06 (m, 1H).

### Stufe 2: 3-[4-(4,4,5,5-Tetramethyl-[1,3,2]dioxaborolan-2-yl)-pyrazol-1-yl]-pyridin

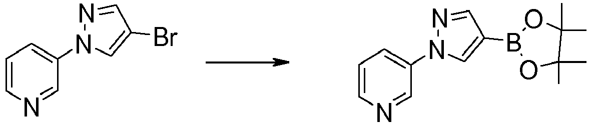

Unter Argon wurden 3-(4-Bromo-pyrazol-1-yl)-pyridin (1,00 g, 4,46 mmol), 1,1'-Bis(diphenylphosphino)ferrocen (dppf) (74 mg, 0,13 mmol), Palladiumdichlorid-dppf (109 mg, 0,13 mmol), Kaliumacetat (1,31 g, 13,3 mmol), Pinacolatodiboran (1,19 g, 4,68 mmol) mit Dioxan (11 mL) versetzt. Die Mischung wurde 24 h unter Rückfluß erhitzt und anschließend abgekühlt. Das Lösungsmittel wurde im Vakuum am Rotationsverdampfer entfernt und der Rückstand mit Dichlormethan (100 ml) und Wasser (100 ml) versetzt, der entstandene Feststoff wurde abgesaugt und verworfen. Das Filtrat wurde mit Dichlormethan extrahiert und die organische Phase mit Magnesiumsulfat getrocknet. Das Lösungsmittel wurde im Vakuum am Rotationsverdampfer entfernt und der Rückstand wurde chromatographiert (Essigester, Cyclohexan).
Ausbeute: 680 mg (55% d. Th.), logP (HCOOH) 2,19, [M⁺+1] 272,2
¹H-NMR (d₆-DMSO): 1,30 (s, 12H), 7,51 (m, 1H), 7,90 (s, 1H), 8,25 (m, 1H), 8,51 (m, 1H), 8,73 (s, 1H), 9,12 (m, 1H).

### Stufe 3: 2-(6-Bromo-pyridin-2-yl)-pyrimidin

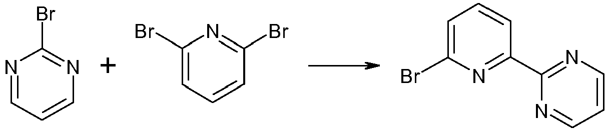

54,5 ml (137,1mmol) einer 2,5-molaren Lösung von n-Butyllithium (n-Buli) wurden unter Argon mit 70 ml THF verdünnt. Bei einer Temperatur unterhalb von -70°C wurden 32,5g (137,1 mmol) 2.6-Dibrompyridin in 150 ml THF gelöst zugetropft. Man rührte 15 min und versetzte dann, weiterhin bei einer Temperatur unterhalb von -70°C, mit 19,2 ml (107 mmol) einer 5.6-molaren Lösung von Zinkchlorid in Diethylether. Man ließ auftauen, versetzte mit einer Lösung von 17,4 g (109 mmol) Brompyrimidin in 50 ml THF und einer Suspension von 3,9 g (3,4 mmol) Tetrakis(triphenylphosphin)palladium in 50 ml THF. Die Mischung wurde 4 h unter Rückfluss gekocht, nach dem Abkühlen versetzte man mit Na-EDTA in Wasser und verdünnter Natronlauge bis pH=10. Man saugte ab, um das Ungelöste zu entfernen, extrahierte die wässrige Phase dreimal mit Essigester, trocknete die vereinigten organischen Phasen mit MgSO₄ und dampfte ein. Den Rückstand kristallisierte man aus 60 ml Benzotrifluorid um, dabei wurde kurz mit Aktivkohle gekocht und heiß filtriert.
Ausbeute: 14,82 g (52% d.Th), logP (HCOOH) 1,3 [M+1] 236,0
¹H-NMR (CD₃CN): 7,4 (t, 1H), 7,65 (d, 1H), 7,8 (dd, 1H), 8,4 (d, 1H), 8,9 (m, 2H)

### Stufe 4: 2-[6-(1-Pyridin-3-yl-1H-pyrazol-4-yl)-pyridin-2-yl]-pyrimidin

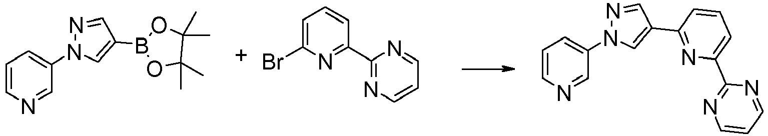

Unter Argon wurde eine Mischung von Acetonitril (50 ml) und Natriumcarbonat (IN in Wasser, 30 ml) hergestellt (Lösung A). 3-[4-(4,4,5,5-Tetramethyl-[1,3,2]dioxaborolan-2-yl)-pyrazol-1-yl]-pyridin (2,0 g, 7,37 mmol), 2-(6-Bromo-pyridin-2-yl)-pyrimidin (1,74 g, 7,37 mmol) und Tetrakis-(triphenylphosphin)-palladium (255 mg, 0,22 mmol) wurden mit der Lösung A unter Argon versetzt und über Nacht bei 75 °C gerührt. Die Mischung wurde abgekühlt und eingeengt. Der Rückstand wurde mit Dichlormethan/Wasser versetzt und der entstandene Feststoff abgetrennt und verworfen. Der Filtrat wurde extrahiert und die vereinigten organischen Phasen über Magnesiumsulfat getrocknet und eingeengt. Der Rückstand wurde zunächst einmal mit Chloroform und dann zweimal mit Diethylether gewaschen.
Ausbeute: 1,11 g (50% d.Th), logP (HCOOH) 1,36, [M⁺+1] 301,1
¹H-NMR (d₆-DMSO): 7,55 (m, 2H), 7,93 (d, 1H), 8,02 (m, 1H), 8,21 (d, 1H), 8,31 (m, 1H), 8,43 (s, 1H), 8,56 (m, 1H), 9,00 (m, 2H), 9,15 (m, 2H).

### Alternative Synthese zu Beispiel 1:

### Stufe 1: 6-((E)-2-Dimethylamino-vinyl)-pyridin-2-carbonitril

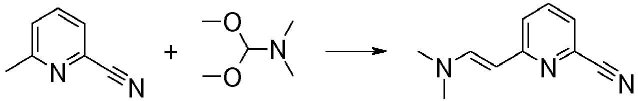

20g (169mol) 6-Methyl-pyridin-2-carbonitril wurden in einer Mischung von jeweils 100 ml DMF und DMF-Dimethylacetal über Nacht im Autoklaven bei 175 °C umgesetzt. Nach Abkühlen wurde eingedampft, in Dichlormethan gelöst, filtriert, eingeengt und der Rückstand im Kugelrohr vakuumdestilliert.
Ausbeute (anteilig berechnet aus 2 Ansätzen) 26.8g (73%d.Th), [M+] aus GCMS 173
¹H-NMR (CD₃CN): 2.9 (s, 6H), 5.15 (d, 1H), 7,1 (m, 2H), 7,5 (m, 2H)

### Stufe 2: 2-(6-Cyano-pyridin-2-yl)-3-dimethylamino-allyliden]-dimethyl-ammoniumhexafluoro phosphat

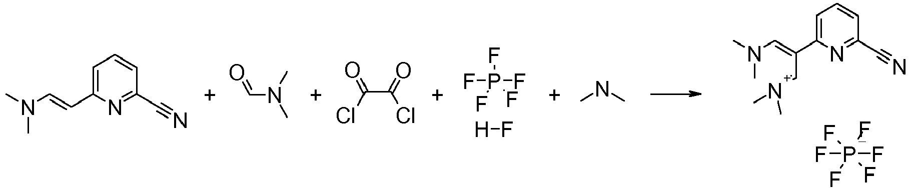

5 ml (65 mmol) DMF wurden in ca. 100 ml Dichlormethan unter Eisbadkühlung mit 7 ml (80 mmol) Oxalylchlorid versetzt, die erhaltene Mischung wurde eingeengt, wieder in Dichlormethan suspendiert und unter Eis/Ethanol-Badkühlung zu einer Lösung von 7,5 g (43 mmol) 6-((E)-2-Dimethylamino-vinyl)-pyridin-2-carbonitril in 100 ml Dichlormethan gegeben. Man rührte 15 min und dampfte dann ein. Der Rückstand wurde unter Eis/Ethanol-Badkühlung mit ca. 50 ml Ethanol und 40 ml Dimethylamin als Lösung in Ethanol (5.6M, entsprechend 224 mmol) versetzt. Man rühert 10 min, versetzte mit 6,5 ml Hexafluorphosphorsäure (60%ige wäßrige Lösung, entsprechend 44 mol), rührte noch 15 min im Kühlbad, saugte den gebildeten kristallinen Niederschlag ab, wusch diesen mit kaltem Ethanol und trocknete ihn am Rotationsverdampfer.
Ausbeute 13,1g (81% d. Th), logP (HCOOH) -0,07, [M+] 229,2 (Kation ohne PF6-)
¹H-NMR (CD₃CN) 2,45 (br, 6H), 3,3 (s, 6H), 7,5 (s, 2H), 7,6 (m, 1H), 7,85 (m, 1H), 8,0 (m, 1H)

### Stufe 3: 6-(1-Pyridin-3-yl-1H-pyrazol-4-yl)-pyridin-2-carbonitril

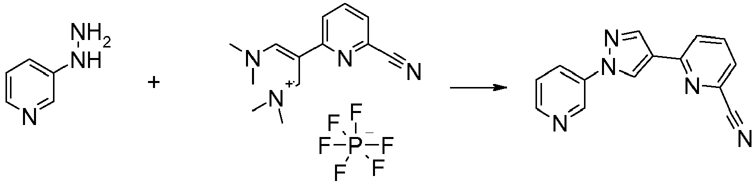

2,2 g (5,9 mmol) 2-(6-Cyano-pyridin-2-yl)-3-dimethylamino-allyliden]-dimethylammoniumhexafluorophosphat und 0,65 g (5,9 mmol) 3-Hydrazinopyridin wurden in ca. 50 ml Dioxan/Acetonitril gelöst. Man ließ über Nacht bei (Raumtemperatur) RT stehen und engte dann ein. Der Rückstand wurde mit aq. Natriumchlorid, aq. Na-Citrat, verdünnter Natrolauge bis pH=9 und Ethylacetat versetzt. Die wäßrige Phase wurde danach noch zweimal mit Dichlormethan/2-Propanol extrahiert. Die vereinigten organischen Phasen wurden mit MgSO₄ getrocknet und eingedampft, der Rückstand wurde aus Ethanol umkristallisiert, dabei wurde heiß filtriert, das Filtrat etwas eingeengt, der gebildete Niederschlag abgesaugt, mit MTBE gewaschen und am Rotationsverdampfer getrocknet.
Ausbeute: 0.76 g (52% d. Th), logP (neutral)1,79; [M+1] 248,1
¹H-NMR (d₆-DMSO): 7,6 (m, 1H), 7,9 (m, 1H), 8,1 (m, 2H), 8,3 (m, 1H), 8,5 (m, 1H), 8,6 (m, 1H), 9,2 (m, 1H), 9,3 (2, 1H)

### Stufe 4: 6-(1-Pyridin-3-yl-1H-pyrazol-4-yl)-pyridin-2-carboxamidin

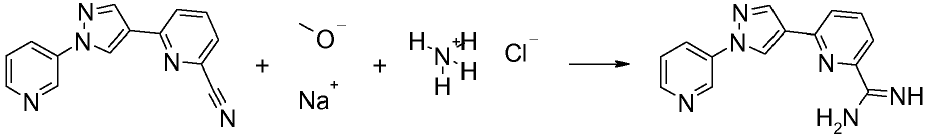

0,7 g (2,8 mmol) 6-(1-Pyridin-3-yl-1*H* pyrazol-4-yl)-pyridin-2-carbonitril wurden in 150 ml abs. Methanol unter Erwärmen gelöst und mit 1 ml Natriummethanolat-Lösung in Methanol (30%, entspricht 5,4mol) versetzt. Man rührte bei 65°C bis das Dünnschichtchromatogramm (DC) komplette Umsetzung anzeigt, versetzte mit 1,5 g Ammoniumchlorid und engte ein. Der Rückstand wurde mit zweimal mit jeweils 30 ml Ethanol gekocht, heiß filtriert und die vereinigten Filtrate etwas eingeengt. Man ließ 20 min bei RT stehen, saugte den gebildeten Niederschlag ab, wusch ihn mit MTBE und trocknete am Rotationsverdampfer.
Ausbeute: 0,74 g (95% d. Th), log P (neutral) 1,99; [M+1] 265,2
¹H-NMR (d₆-DMSO) 7,5 (br, 3H), 7,65 (m, 1H), 8,2 (m, 2H), 8,35 (, 2H), 8,6 (d, 1H), 8,7 (s, 1H), 9,2 (m, 1H), 9,7 (s, 1H)

### Stufe 5: 2-[6-(1-Pyridin-3-yl-1H-pyrazol-4-yl)-pyridin-2-yl]-pyrimidin

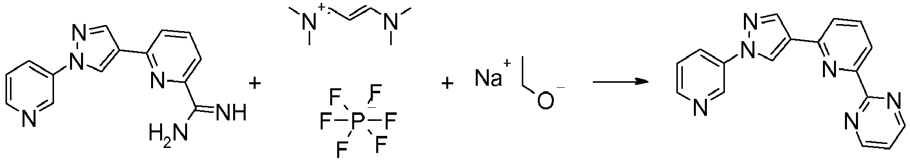

0,73 g (2,7 mmol) 6-(1-Pyridin-3-yl-1*H*-pyrazol-4-yl)-pyridin-2-carboxamidin und 0,8 g (2,9 mmol) (3-Dimethylamino-allylidene)-dimethyl-ammoniumhexafluorophosphat wurden in 30 ml Ethanol mit 3 ml Natriumethanolat als Lösung in Ethanol (21%, enstprechend 8,1 mmol) 6 h unter Argon unter Rückfluß erhitzt. Man engte ein, versetzt mit Na-Citrat-Puffer, verd. Natronlauge bis pH=9, aq. NaCl, Ethylacetat/THF, extrahierte die wäßrige Phase noch drei mal mit Dichlormethan/2-Propanol, trocknete die vereinigten organischen Phasen mit MgSO₄ und dampfte ein. Der Rückstand wurde aus Ethanol umkristalisiert, dabei wurde kurz mit Aktivkohle gekocht und heiß filtriert, das Filtrat wurde etwas eingeengt und der gebildete Niederschlag mit MTBE gewaschen und am Rotationsverdampfer getrocknet. Der Rückstand wurde durch eine Filtration durch Kieselgel mit Aceton weiter gereingt.
Ausbeute: 0,37 g (43% d. Th), logP (neutral) 1,52, [M+1] 301,1
¹H-NMR (CD₃CN): 7,45 (t, 1H), 7,5 (dd, 1H), 7,8 (d, 1H), 7,95 (t, 1H), 8,2 (m, 1H), 8,3 (d, 1H), 8,35 (s, 1H), 8,55 (d, 1H), 8,8 (s, 1H), 8,95 (m, 2H), 9,1 (d, 1H)

### Beispiel 2:

### Stufe 1: 3-Dimethylamino-1-pyrimidin-2-yl-propenon

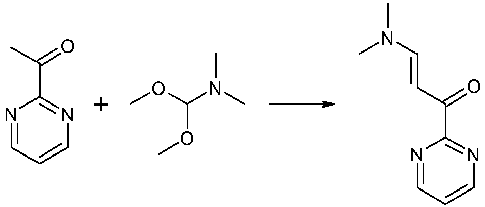

11 g (90 mmol) 2-Acetylpyrimidin wurden mit 23 g (193 mmol) DMF-DMA (Dimethylacetamid) an einer sehr kurzen Destillationsbrücke 1 h bei 100°C gerührt, dabei ging etwas Destillat über. Man dampfte ein, der Rückstand wurde aus Benzotrifluorid umkristallisiert.
Ausbeute:11,4 g (70% d. Th)
¹H-NMR (d₆-DMSO): 3,1 (s, 6H), 6 (d, 1H), 7,5 (t, 1H), 7,7 (d, 1H), 8,9 (d, 2H)

### Stufe 2: 2-(1H-Pyrazol-3-yl)-pyrimidin

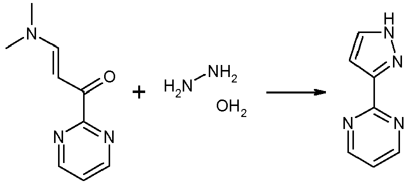

11,2 g (63,2 mmol) 3-Dimethylamino-1-pyrimidin-2-yl-propenon wurden mit 4,5 ml (92,5 mmol) Hydrazin-Hydrat in 200 ml EtOH 2 h unter Rückfluß erhitzt. Man dampfte ein und kristallisierte den Rückstand aus Benzotrifluorid um.
Ausbeute: 8,72 g (94% d. Th) logP(HCOOH) 0,1
¹H-NMR (d₆-DMSO) 9,65 (s, 1H), 7,4 (s, 1H), 7,6-7,8 (br, 1H), 8,85 (m, 2H), 13-13,5 (br, 1H)

### Stufe 3: 1'-(Pyridin-3-yl)-3-(pyrimidin-2-yl)-1'H-1,4'-bipyrazole

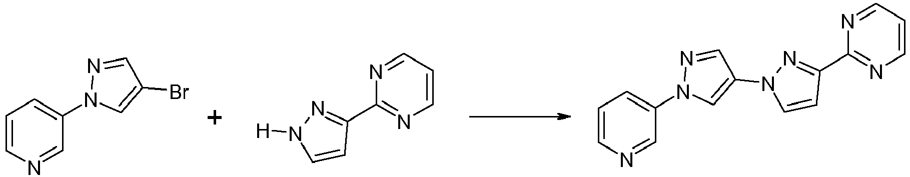

3-(4-Bromo-pyrazol-1-yl)-pyridin (800 mg, 3,57 mmol), 2-(1H-Pyrazol-3-yl)-pyrimidin (783 mg, 5,35 mmol), Caesiumcarbonat (1,86 g, 5,71 mmol), Cu₂O (29 mg, 0,17 mmol) und Salicylaldoxim (97,8 mg, 0,71 mmol) wurden unter Argon in DMF (4 ml) bei 130 °C gerührt. Nach 36 h wurde die Mischung abgekühlt und filtriert. Das Filtrat wurde eingeengt und der Rückstand wurde zuerst mit Essigester/Cyclohexan und dann mit Essigester/Isopropanol an Kieselgel chromatographiert.
Ausbeute: 274 mg (27% d.Th), logP (HCOOH) 1,18, [M⁺+1] 290,1
¹H-NMR (d₆-DMSO): 7,19 (m, 1H), 7,48 (m, 1H), 7,60 (m, 1H), 8,33 (m, 1H), 8,40 (s, 1H), 8,43 (m, 1H), 8,58 (m, 1H), 8,90 (m, 2H), 9,19 (m, 1H), 9,24 (s, 1H)

### Beispiel 3: 1'-Pyridin-3-yl-3-trifluoromethyl-1'H [1,4']bipyrazolyl

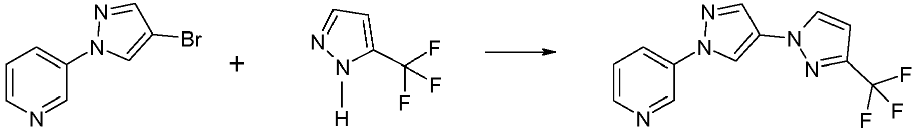

3-(4-Bromo-pyrazol-1-yl)-pyridin (1,00 g, 4,46 mmol), 3-Trifluoromethylpyrazol (911 mg, 6,69 mmol), Caesiumcarbonat (2,33 g, 7,14 mmol), Cu₂O (36 mg, 0,22 mmol) und Salicylaldoxim (122 mg, 0,89 mmol) wurden unter Argon in DMF (5 ml) versetzt und bei 130 °C gerührt. Nach 24 h wurde die Mischung auf Raumtemperatur abgekühlt und über Kieselgel (Aceton) filtriert. Das Lösungsmittel wurde im Vakuum entfernt. Der Rückstand wurde mit einer gesättigten NaCl-Lösung versetzt und die organischen Komponenten wurde mit Essigester extrahiert. Die organische Phase wurde mit Magnesiumsulfat getrocknet und das Lösungsmittel wurde im Vakuum entfernt. Der Rückstand wurde chromatographiert (Essigester, Cyclohexan).
Ausbeute: 200 mg (15% d.Th), logP (HCOOH) 2,27, [M⁺+1] 280,1
¹H-NMR (d₆-DMSO): 6,97 (m, 1H), 7,57 (m, 1H), 8,28 (m, 1H), 8,31 (s, 1H), 8,44 (m, 1H), 8,58 (m, 1H), 9,13 (m, 2H).

### Beispiel 4: 2-[1-(Pyridin-3-yl)-1H-pyrazol-4-yl]-6-(trifluormethyl)pyridin

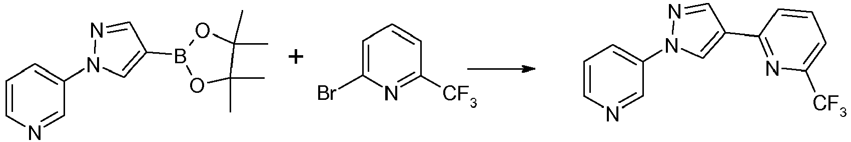

Unter Argon wurde eine Mischung aus Acetonitril (4,8 ml) und Natriumcarbonat (IN im Wasser, 5,9 ml) hergestellt (Lösung A). 3-[4-(4,4,5,5-Tetramethyl-[1,3,2]dioxaborolan-2-yl)-pyrazol-1-yl]-pyridin (400 mg, 1,47 mmol), 2-Brom-6-(trifluoromethyl)pyridin (333 mg, 1,47 mmol) und Tetrakis-(triphenylphosphin)-palladium (51 mg, 0,04 mmol) wurden mit der Lösung A unter Argon versetzt und über Nacht bei 75 °C gerührt. Die Mischung wurde abgekühlt und eingeengt. Der Rückstand wurde mit Essigester/Wasser versetzt und extrahiert. Die organische Phase wurde über Magnesiumsulfat getrocknet und eingeengt. Der Rückstand wurde zunächst an Kieselgel (Essigester, Cyclohexan) und anschließend mit der präparativen HPLC (RP, Acetonitril, Wasser) gereinigt.
Ausbeute: 210 mg (49% d.Th), logP (HCOOH) 2,51, [M⁺+1] 291,1
¹H-NMR (d₆-DMSO): 7,56 (m, 1H), 7,72 (m, 1H), 8,09 (m, 2H), 8,31 (m, 1H), 8,40 (s, 1H), 8,56 (m, 1H), 9,18 (m, 2H)

### Beispiel 5 :

### Stufe 1: 6-(1-Pyridin-3-yl-1H-pyrazol-4-yl)-pyridin-2-carbothionsäurcamid

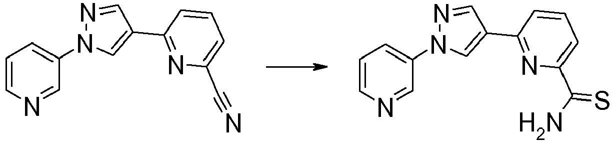

1 g (4 mmol) 6-(1-Pyridin-3-yl-1*H*-pyrazol-4-yl)-pyridin-2-carbonitril wurden in 30 ml Pyridin unter Erwärmen gelöst und mit 1,5 ml Ammoniumsulfid (40%ige wäßrige Lösung, entsprechend 8,8 mmol) versetzt. Nach 1 h wurde eingedampft. Der Rückstand wurde aus Ethanol umkristallisiert, dabei wurde heiß filtriert, das Filtrat etwas eingeengt, der gebildete Niederschlag abgesaugt, mit MTBE gewaschen und am Rotationsverdampfer getrocknet.
Ausbeute: 1,2 g (98%d. Th), logP (neutral)1,77, [M+1] 282,1
¹H-NMR (d₆-DMSO): 7,6 (m, 1H), 7,9 (m, 2H), 8,3 (m, 1H), 8,4 (d, 1H), 8,55 (m, 2H), 9,25 (d, 1H), 9,4 (s, 1H)

### Stufe 2: N-Hydroxy-6-(1-pyridin-3-yl-1H-pyrazol-4-yl)-pyridin-2-carboxamidin

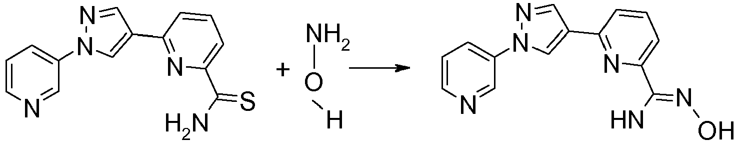

0,4 g (1,4 mmol) 6-(1-Pyridin-3-yl-1*H*-pyrazol-4-yl)-pyridin-2-carbothionsäureamid wurden in 40 ml Dioxan-THF 1:3 unter Erwärmen gelöst, mit 0,4 ml Hydroxylamin (50%ige wäßrige Lösung, entsprechend 6,8 mmol) versetzt und 1 h bei 60°C gerührt, dann wurde eingedampft. Das so erhaltene Rohprodukt wurde nicht gereinigt.
Ausbeute : 0,34 g, logP (neutral) 1,23, [M+H] 281,1

### Stufe 3: 2-[1,2,4]Oxadiazol-3-yl-6-(1-pyridin-3-yl-1H-pyrazol-4-yl)-pyridin

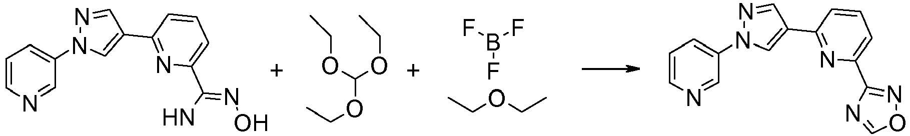

0,24 g *N*-Hydroxy-6-(1-pyridin-3-yl-1*H*-pyrazol-4-yl)-pyridin-2-carboxamidin (Rohprodukt aus der vorhergehenden Stufe) wurden in THF/Triethylorthoformat gelöst und mit 0,2 g BF₃-ET₂O versetzt. Nach 2 h wurde mit Ethylacetat, aq. Zitratpuffer pH=6, aq. NaCl versetzt und die wäßrige Phase 3 mal mit Ethylacetat extrahiert. Die vereinigten organischen Phasen wurden mit MgSO₄ getrocknet und eingedampft, der Rückstand durch Chromatographie an Kieselgel (Petrolether/Aceton) gereinigt.
Ausbeute: 0,1 g logP(neutral) 1,54, [M+1) 291,1
¹H-NMR (d₆-DMSO): 7,55 (dd, 1H), 8,0 (m, 2H), 8,05 (m, 1H), 8,3 (m, 1H), 8,4 (s, 1H), 8,55 (m, 1H), 9,2 (m, 2H), 9,7 (s, 1H)

### Beispiel 6:

### 2-(1-Methyl-1H-[1,2,-4]triazol-3-yl)-6-(1-pyridin-3-yl-1H-pyrazol-4-yl)-pyridin

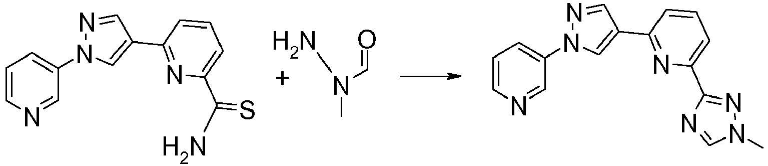

0,57 g (2 mmol) 6-(1-Pyridin-3-yl-1*H*-pyrazol-4-yl)-pyridin-2-carbothionsäureamid und 2,5 ml (27 mmol) 1-Methyl-1-Formylhydrazin wurden 6 h bei 145°C gerührt. Nach dem Abkühlen wurde die Mischung durch Chromatographie an Kieselgel (Cyclohexan/Aceton) gereinigt.
Ausbeute: 0,31 g (50% d. Th), logP (neutral) 1,29; [M+1] 304,2
¹H-NMR (d₆-DMSO): 4 (s, 3H), 7,55 (m, 1H), 7,8 (m, 1H), 7,95 (m, 2H), 8,3 (m, 1H), 8,4 (s, 1H), 8,55 (s, 1H), 8,6 (m, 1H), 9,1 (s, 1H), 9,2 (m, 1H)

### Beispiel 7: 3-[6-(1-Pyridin-3-yl-1H-pyrazol-4-yl)-pyridin-2-yl]-[1,2,4]triazin

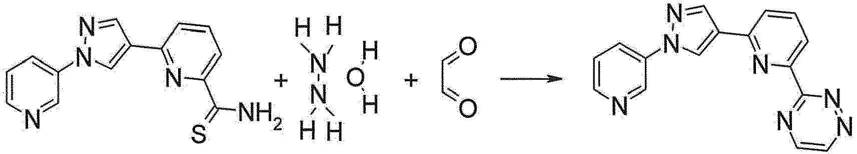

0,4 g (1,4 mmol) 6-(1-Pyridin-3-yl-1*H*-pyrazol-4-yl)-pyridin-2-carbothionsäureamid wurden in 30 ml Ethanol mit 0,2 ml (4,1mmol) Hydrazin-Hydrat 20 min unter Rückfluß erhitzt, dabei bildete sich eine klare Mischung. Man versetzte mit 0,6 ml Glyoxal (40%ige wäßrige Lösung, entsprechend 4 mmol) und erhitzte weitere 20 min unter Rückfluß. Die Mischung wurde filtriert und eingedampft, der Rückstand durch Chromatographie an Kieselgel (Petrolether/Aceton) gereinigt.
Ausbeute: 0,13 g (29% d. Th), logP (neutral) 1,32, [M+1] 302,1
¹H-NMR (d₆-DMSO): 7,55 (dd, 1H), 8,0 (m, 1H), 8,1 (m, 1H), 8,3 (m, 2H), 8,45 (s, 1H), 8,55 (m, 1 H), 9,1 (d, 1H), 9,2 (m, 2H), 9,5 (m, 1H)

### Beispiel 8:

### Stufe 1: N-Methoxy-N-methyl-1-(pyridin-3-yl)-1H-pyrazol-4-carboxamid

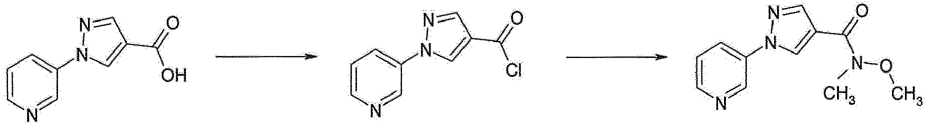

1-(Pyridin-3-yl)-1H-pyrazol-4-carbonsäure (7,54 g, 39,8 mmol) wurde unter Argon in Dichlormethan (190 ml) vorgelegt und mit Dimethylformamid (2 ml) versetzt. Dann wurde Oxalsäuredichlorid (14,67 g, 115 mmol) bei Raumtemperatur zugegeben und die Mischung 1 h bei Raumtemperatur gerührt. Das Lösungsmittel wurde am Rotationsverdampfer unter Vakuum entfernt, der Rückstand mit Toluol (10 ml) versetzt und die Mischung eingeengt. Das Säurechlorid wurde direkt weiter umgesetzt.

Das Säurechlorid wurde in Dioxan (125 ml) gelöst und Diisopropylethylamin (Hünig Base, 22,9 g, 177 mmol) zugetropft. Die Mischung wurde anschließend 30 min bei Raumtemperatur gerührt. (Methoxyamino)methanhydrochlorid (4,32 g, 44,3 mmol) wurde in Dioxan (245 ml) suspendiert und portionsweise zugegeben (leicht exotherm). Die Mischung wurde über Nacht bei Ramtemperatur gerührt. Vom Ungelösten wurde abfiltriert. Der Filtrat wurde eingeengt und mit Chloroform versetzt. Der Feststoff wurde abgesaugt und per Säulenchromatographie gereinigt (Ethylacetat).
Ausbeute: 6,510 g (63% d.Th), logP (HCOOH) 0,91, [M⁺+1] 233,1
¹H-NMR (d₆-DMSO): 3,27 (s, 3H), 3,77 (s, 3H), 7,53-7,57 (m, 1H), 8,14 (s, 1H), 8,27-8,30 (m, 1H), 8,58 (m, 1H), 8,89 (s, 1H), 9,14 (m, 1H)

### Stufe 2: 1-[1-(Pyridin-3-yl)-1H-pyrazol-4-yl]ethanon

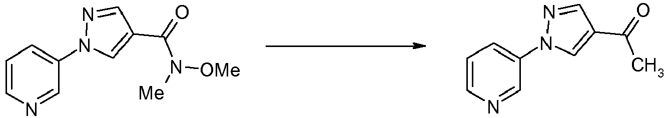

N-Methoxy-N-methyl-1-(pyridin-3-yl)-1H-pyrazol-4-carboxamid (5,00 g, 21,5 mmol) wurde unter Argon in trockenem THF (200 ml) vorgelegt und auf 0 °C abgekühlt. Methylmagnesiumchlorid (17,9 ml, 3M in THF) wurde zugetropft und die Mischung über Nacht bei Raumtemperatur gerührt. Die Reaktionsmischung wurde mit gesättigter Ammoniumchlorid-Lösung (160 ml) versetzt und die Phasen wurden getrennt. Die wässrige Phase wurde nochmal mit Ethylacetat extrahiert und die vereinigten organischen Phasen mit Magnesiumsulfat getrocknet. Das Lösungsmittel wurde am Rotationsverdampfer im Vakuum entfernt.
Ausbeute: 4,110 g (99% d.Th), logP (HCOOH) 0,76, [M⁺+1] 188,1
¹H-NMR (d₆-DMSO): 2,47 (s, 3H), 7,57 (m, 1H), 8,21 (s, 1H), 8,28 (m, 1H), 8,59 (m, 1H), 9,14 (m, 1H), 9,19 (s, 1H)

### Stufe 3: (2E)-3-(Dimethylamino)-1-[1-(pyridin-3-yl)-1H-pyrazol-4-yl]prop-2-en-1-on

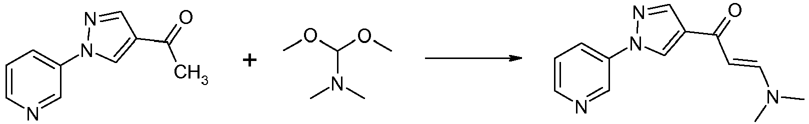

1-[1-(Pyridin-3-yl)-1H-pyrazol-4-yl]ethanon (860 mg, 4,59 mmol), Dimethylformamiddimethylacetal (6 ml) und Dimethylformamid (6 ml) wurden in einem Rundkolben zusammengegeben und die Mischung 6 h bei 100 °C mit einer Destillationsbrücke erwärmt. Die Mischung wurde dann abgekühlt und eingeengt.
Ausbeute: 1,13 g (100% d.Th), logP (HCOOH) 0,93, [M⁺+1] 243,2
¹H-NMR (d₆-DMSO): 3,01 (bs, 6H), 5,65 (d, 1H), 7,55 (m, 1H), 7,63 (d, 1H), 8,13 (s, 1H), 8,26 (m, 1H), 8,54 (m, 1H), 8,99 (s, 1H), 9,13 (m, 1H)

### Stufe 4: 1'-(Pyridin-3-yl)-1H,1'H-3,4'-bipyrazol

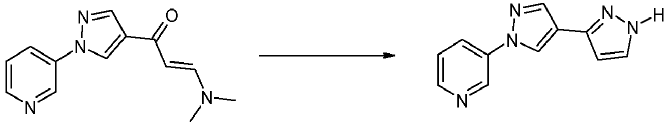

(2E)-3-(Dimethylamino)-1-[1-(pyridin-3-yl)-1H-pyrazol-4-yl]prop-2-en-1-on (800 mg, 3,30 mmol) wurde in Ethanol (7 ml) vorgelegt und Hydrazin (6,60 ml, 6,60 mmol) wurde zugetropft. Die Mischung wurde 5 h unter Rückfluß erhitzt, anschließend abgekühlt und eingengt.
Ausbeute: 700 mg (100% d.Th), logP (HCOOH) 0,79, [M⁺+1] 212,1
¹H-NMR (d₆-DMSO): 6,51 (m, 1H), 7,54 (m, 1H), 7,73 (m, 1H), 8,09 (m, 1H), 8,22 (m, 1H), 8,52 (m, 1H), 8,81 (m, 1H), 9,11 (m, 1H), 12,65 (m, 1H)

### Stufe 5: 1'-(Pyridin-3-yl)-1-[4-(trifluormethyl)pyrimidin-2-yl]-1H,1'H-3,4'-bipyrazol

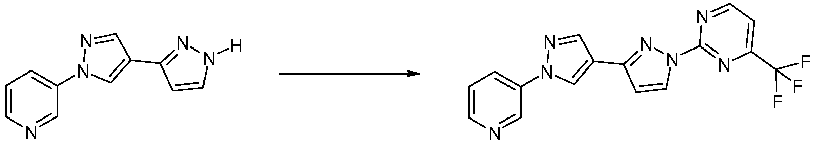

1'-(Pyridin-3-yl)-1H,1'H-3,4'-bipyrazol (200 mg, 0,94 mmol) wurde unter Argon in Dimethylamid vorgelegt. Natriumhydrid (56,8 mg, 1,42 mmol, 60% in Paraffinöl) wurde zugegeben und die Mischung 30 min bei Raumtemperatur gerührt (leicht exotherm). 2-Chlor-4-trifluormethylpyrimidin (173 mg, 0,94 mmol) wurde zugegeben und die Mischung über Nacht bei 40 °C gerührt. Anschlie-ßend wurde die Mischung eingeengt und der Rückstand mit Dichlormethan verrührt.
Ausbeute: 240 mg (71% d.Th), logP (HCOOH) 2,15, [M⁺+1] 358,1
¹H-NMR (d₆-DMSO): 7,00 (m, 1H), 7,56 (m, 1H), 7,90 (d, 1H), 8,31 (m, 2H), 8,56 (m, 1H), 8,73 (m, 1H), 9,10 (s, 1H), 9,17-9,22 (m, 2H)

### Beispiel 9: 2-(Pentafluorethyl)-4-[1-(pyridin-3-yl)-1H-pyrazol-4-yl]pyrimidin

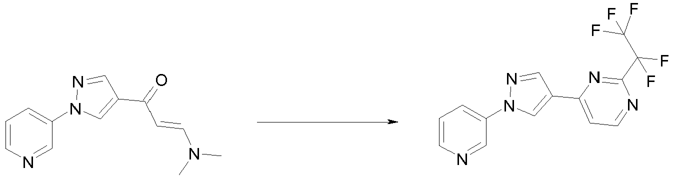

(2E)-3-(Dimethylamino)-1-[1-(pyridin-3-yl)-1H-pyrazol-4-yl]prop-2-en-1-on (200 mg, 0,82 mmol), Natriummethylat (56 mg, 1,03 mmol), 2,2,3,3,3-Pentafluorpropionamidin (166 mg, 1,02 mmol) wurden zusammen in Methanol (2 ml) vorgelegt. Die Mischung wurde über Nacht bei 55 °C gerührt. Es wurde abgekühlt und eingedampft. Der Rückstand wurde mittels Säulenchromatographie gereinigt (Aceton, Isopropanol, 1:1).
Ausbeute: 125 mg (44% d.Th), logP (HCOOH) 2,7, [M⁺+1] 342,1
¹H-NMR (d₆-DMSO): 7,58 (m, 1H), 8,13 (d, 1H), 8,33 (m, 1H), 8,52 (s, 1H), 8,61 (m, 1H), 9,03 (d, 1H), 9,18 (m, 1H), 9,37 (s, 1H)

### Beispiel 10

### Stufe 1: 1-[6-(1-Pyridin-3-yl-1H-pyrazol-4-yl)-pyridin-2-yl]-ethanon

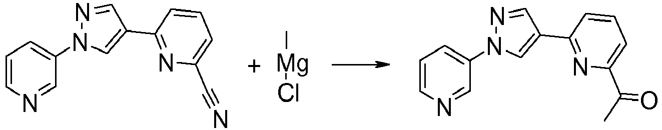

0,52 g (2,1 mmol) 6-(1H-Pyridin-3-yl-1*H*-pyrazol-4-yl)-pyridin-2-carbonitril wurden unter Argon in 100 ml THF mit 1,5 ml Methylmagnesiumchlorid (3 M, entsprechend 24 mmol) und mehreren Spatelspitzen CuBr-Dimethylsulfid-Komplex 2 h bei Raumtemperatur gerührt. Man versetzte mit verdünnter Salzsäure, Citrat-Puffer und verd. Natronlauge bis pH=9 sowie aq. NaCl und Ethylacetat. Die wäßrige Phase wurde 3 mal mit Ethylacetat extrahiert, die vereinigten organischen Phasen mit MgSO₄ getrocknet und eingedampft. Der Rückstand wurde durch Chromatographie an Kieselgel (Petrolether /Aceton) gereinigt.
Ausbeute: 0,12 g (20% d. Th). logP (neutral) 1,94 [M+1]265,2
¹H-NMR (d₆-DMSO): 2,7 (s, 3H), 7,55 (m, 1H), 7,8 (m, 1H), 8,0 (m, 2H), 8,3 (m, 1H), 8,45 (s, 1H), 5,8 (m, 1H), 9,2 (m, 2H)

### Stufe 2: 2-(2-Methyl-1,3-dioxolan-2-yl)-6-(1-pyridin-3-yl-1H-pyrazol-4-yl)-pyridin

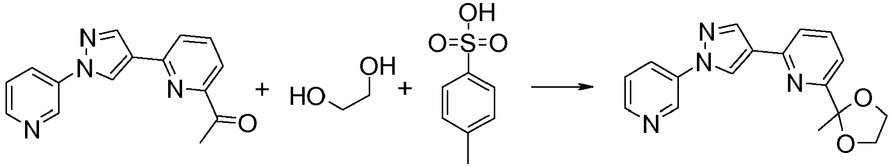

0,13 g (0,49 mmol) 1-[6-(1-Pyridin-3-yl-1*H*-pyrazol-4-yl)-pyridin-2-yl]-ethanon wurden in 100 ml 1.1.1-Benzotrifluorid mit 0,1 g (0,58 mmol) 4-Toluolsulfonsäure und 1,8 ml (32 mmol) 1.2-Ethandiol in einer Rückflußapparatur mit Soxleth-Extraktor, der mit Molekularsieb 4A beladen war, 2 h unter Rückfluß erhitzt. Das Reaktionsgemisch wurde eingedampft, der Rückstand mit Etyhlacetat, verd. Natronlauge, Citratpuffer bis pH=9 sowie aq. NaCl versetzt, die wäßrige Phase zweimal mit Ethylacetat extrahiert, die vereinigten organischen Phasen mit MgSO₄ getrocknet und eingedampft. Der Rückstand wurde durch Chromatgraphie an Kieselgel (Petrolether/Aceton) gereinigt.
Ausbeute: 0,12 g (75% d. Th), log P(neutral) 1,89, [M+1] 309,1
1H-NMR (d₆-DMSO): 1,7 (s, 3H), 3,9 (m, 2H), 4,1 (m, 2H), 7,4 (d, 1H), 7,55 (m, 1H), 7,7 (m, 1H), 7,8 (m, 1H), 8,3 (d, 1H), 8,35 (s, 1H), 8,55 (m, 1H), 9,1 (s, 1H), 9,2 (s, 1H)

### Besipiel 11

### Stufe 1: 1-(6-Bromo-pyridin-2-yl)-2-methoxy-ethanon

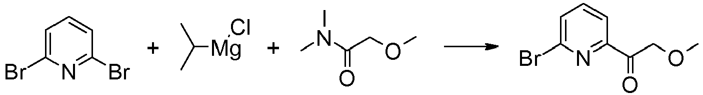

14,5 g (61,2 mmol) 2.6-Dibrompyridin wurden in ca. 50 ml THF gelöst und unter Argon mit 35 ml Isopropylmagnesiumchlorid 2M (entspr. 70mmol) versetzt. Die Mischung erwärmte sich. Man rührte noch 1,5 h bei Raumtemperatur und 2 h bei 30°C, kühlte mit einem CO₂/EtOH-Bad und versetzte mit einer Lösung von 8 g (68,2 mmol) 2-Methoxy-*N,N*-dimethyl-acetamide in THF, verdünnte das eingedickte Reaktionsgemenge mit weiterem THF, ließ auftauen, versetzte mit Ethylacetat, aq. Zitronensäure, verd. Natronlauge bis pH=6, aq. NaCl, extrahierte die wäßrige Phase dreimal mit Ethylacetat, trocknete die vereinigten organischen Phasen mit MgSO₄ und dampfte ein. Der Rückstand wurde im Kugelrohr vakuumdestilliert und das Destillat aus wenig Benzotrifluorid umkristallisiert.
Ausbeute (anteilig berechnet aus zwei Ansätzen): 5,36 g (34% d. Th) logP (neutral) 1,73 [M+1] 232,0 (schwereres Isotop)
¹H-NMR (CD₃CN): 3,4 (s, 3H), 4,85 (s, 2H), 7,75 (, 1H), 7,8 (m, 1H), 7,95 (m, 1H)

### Stufe 2: 2-Methoxy-1-[6-(1-pyridin-3-yl-1H-pyrazol-4-yl)-pyridin-2-yl]-ethanon

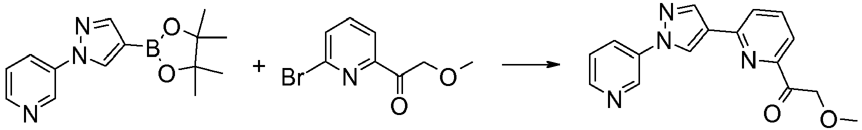

0,042 g (0,23 mmol) Palladium(II)chlorid, 0,27 g (1 mmol) Triphenylphosphan und 1 g Tetrabutylammoniumchlorid wurden unter Argon in 20 ml DMF 10 min bei 100°C gerührt und nach Abkühlen mit 0,9 g (3,9 mmol) 1-(6-Bromo-pyridin-2-yl)-2-methoxy-ethanon, 0,98 g (3,6 mmol) 3-[4-(4,4,5,5-Tetramethyl-[1,3,2]dioxaborolan-2-yl)-pyrazol-1-yl]-pyridin und 1 g (7,2 mmol) Kaliumcarbonat versetzt. Man rührte noch 2 h bei 105°C. Nach dem Abkühlen wurde eingedampft, der Rückstand mit aq. NaCl, Ethylacetat, verd. Natronlauge bis pH=9 versetzt und die wäßrige Phase vier mal mit Ethylacetat extrahiert. Die vereinigten organischen Phasen wurden mit MgSO₄ getrocknet und eingedampft. Der Rückstand wurde durch Chromatographie an Kieselgel (Petrolether/Aceton)gereinigt.
Ausbeute: 0,62 g (58%d. Th) logP(HCOOH) 1,66 [M+1] 295,1
¹H-NMR (d₆-DMSO): 3,4 (s, 3H) 5,2 (s, 2H), 7,54 (m, 1H), 7,8 (m, 1H), 8,05(m, 2H), 8,3 (m, 1H), 8945 (s, 1H), 8,55 (m, 1H), 9,2 (m, 2H)

### Stufe 3: 2-(1,1-Difluor-2-methoxy-ethyl)-6-(1-pyridin-3 -yl-1H-pyrazol-4-yl)-pyridin

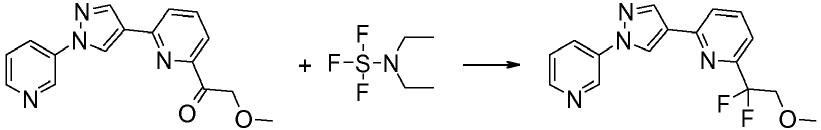

0,2 g (0,68 mmol) 2-Methoxy-1-[6-(1-pyridin-3-yl-1*H*-pyrazol-4-yl)-pyridin-2-yl]-ethanon wurden mit 1,5 ml (11,3 mmol) Diethylaminoschwefeltrifluorid in Tetrachlorkohlenstoff mit zwei Tropfen Trifluoressigsäure 1 h bei 77°C in einer Rückflußapparatur mit PFA-Kolben gerührt. Nach Abkühlen wurde mit Eis, dann Zitrat-Puffer, verd. Natronlauge bis pH=9, aq. NaCl und Ethylacetat versetzt, die wäßrige Phase 3 mal mit Ethylacetat extrahiert, die vereinigten organischen Phasen mit MgSO₄ getrocknet und eingedampft. Der Rückstand wurde durch Chromatographie an Kieselgel (Petrolether/Aceton) gereinigt.
Ausbeute: 0,12 g (54% d. Th), logP (neutral) 2,3 [M+1] 317,2
¹H-NMR (CD₃CN): (CD₃CN): 3,4 (s, 3H), 4,15 (t, 2H), 7,45 (m, 1H), 7,55 (m, 1H), 7,75 (d, 1H), 7,9 (dd,1H), 8,15 (m, 1H), 8,3 (s, 1H), 8,55 (m, 1H), 8,75 (s, 1H), 9,1 (m, 1H)

Analog wurden weitere erfindungsgemäße Verbindungen hergestellt, die in der folgenden Tabelle aufgeführt sind, in ihr geben die Beispiel Nr. 1 bis 11 die Endprodukte der oben ausführlich beschriebenen Herstellungsbeispiele wieder.

| Beispiel Nr. | | logP (neutral) | logP (HCOOH) | [M⁺+1] | ¹H-NMR Daten/(Lösungsmittel) |
|---|---|---|---|---|---|
| 1 | 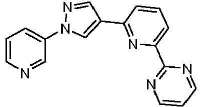 | 1,52 | 1,36 | 301,1 | (CD₃CN): 7,45 (t, 1H), 7,5 (dd, 1H), 7,8 (d, 1H), 7,95 (t, 1H), 8,2 (m, 1H), 8,3 (d, 1H), 8,35 (s, 1H), 8,55 (d, 1H), 8,8 (s, 1H), 8,95 (m, 2H), 9,1 (d, 1H) |
| 2 | 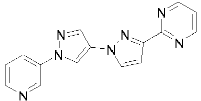 | | 1,18 | 290,1 | (D₆-DMSO): 7,19 (m, 1H), 7,48 (m, 1H), 7,60 (m, 1H), 8,33 (m, 1H), 8,40 (s, 1H), 8,43 (m, 1H), 8,58 (m, 1H), 8,90 (m, 2H), 9,19 (m, 1H), 9,24 (s, 1H) |
| 3 | 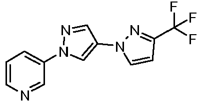 | | 2,27 | 280,1 | (D₆-DMSO): 6,97 (m, 1H), 7,57 (m, 1H), 8,28 (m, 1H), 8,31 (s, 1H), 8,44 (m, 1H), 8,58 (m, 1H), 9,13 (m, 2H) |
| 4 | 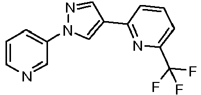 | | 2,51 | 291,1 | (D₆-DMSO): 7,56 (m, 1H), 7,72 (m, 1H), 8,09 (m, 2H), 8,31 (m, 1H), 8,40 (s, 1H), 8,56 (m, 1H), 9,18 (m, 2H) |
| 5 | 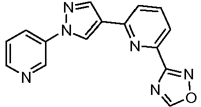 | 1,54 | | 291,1 | (D₆-DMSO): 7,55 (dd, 1H), 8,0 (m, 2H), 8,05 (m, 1H), 8,3 (m, 1H), 8,4 (s, 1H), 8,55 (m, 1H), 9,2 (m, 2H), 9,7 (s, 1H) |
| 6 | 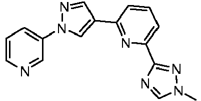 | 1,29 | | 304,2 | (D₆-DMSO): 4 (s, 3H), 7,55 (m, 1H), 7,8 (m, 1H), 7,95 (m, 2H), 8,3 (m, 1H), 8,4 (s, 1H), 8,55 (s, 1H), 8,6 (m, 1H), 9,1 (s, 1H), 9,2 (m, 1H) |
| 7 | 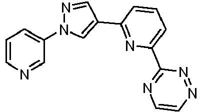 | 1,32 | | 302,1 | (D₆-DMSO): 7,55 (dd, 1H), 8,0 (m, 1H), 8,1 (m, 1H), 8,3 (m, 2H), 8,45 (s, 1H), 8,55 (m, 1H), 9,1 (d, 1H), 9,2 (m, 2H), 9,5 (m, 1H) |
| 8 | 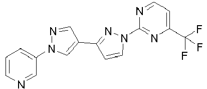 | | 2,15 | 358,1 | (D₆-DMSO): 7,00 (m, 1H), 7,56 (m, 1H), 7,90 (d, 1H), 8,31 (m, 2H), 8,56 (m, 1H), 8,73 (m, 1H), 9,10 (s, 1H), 9,17-9,22 (m, 2H) |
| 9 | 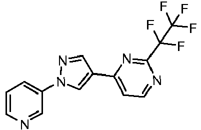 | | 2,7 | 342,1 | (D₆-DMSO): 7,58 (m, 1H), 8,13 (d, 1H), 8,33 (m, 1H), 8,52 (s, 1H), 8,61 (m, 1H), 9,03 (d, 1H), 9,18 (m, 1H), 9,37 (s, 1H) |
| 10 | 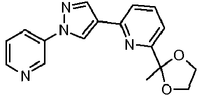 | 1,89 | | 309,1 | (D₆-DMSO): 1,7 (s, 3H), 3,9 (m, 2H), 4,1 (m, 2H), 7,4 (d, 1H), 7,55 (m, 1H), 7,7 (d, 1H), 7,8 (m, 1H), 8,3 (m, 1H), 8,35 (s, 1H), 8,55 (m, 1H), 9,1 (s, 1H), 9,2 (m, 1H) |
| 11 | 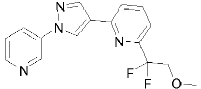 | 2,3 | | 317,2 | (CD₃CN): 3,4 (s, 3H), 4,15 (t, 2H), 7,45 (m, 1H), 7,55 (m, 1H), 7,75 (d, 1H), 7,9 (dd, 1H), 8,15 (m, 1H), 8,3 (s, 1H), 8,55 (m, 1H), 8,75 (s, 1H), 9,1 (m, 1H) |
| 12 | 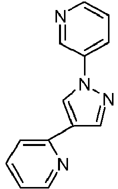 | | 0,81 | 223,1 | (D₆-DMSO), 7,25 (m, 1H), 7,55 (m, 1H), 7,8 (m, 2H), 8,3 (m, 1H), 8,35 (s, 1H), 8,55 (m, 2H), 9,1 (s, 1H), 9,15 (m, 1H) |
| 13 | 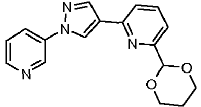 | | 1,59 | 309,2 | (D₆-DMSO): 1,50 (m, 1H), 2,08 (m, 1H), 4,02 (m, 2H), 4,18 (m, 2H), 5,53 (s, 1H), 7,40 (d, 1H), 7,56 (m, 1H), 7,77 (d, 1H), 7,86 (m, 1H), 8,30 (m, 1H), 8,33 (s, 1H), 8,54 (m, 1H), 9,07 (s, 1H), 9,16 (m, 1H) |
| 14 | 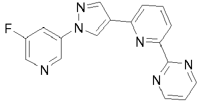 | | 1,81 | 319,1 | (D₆-DMSO): 7,55 (m, 1H), 7,92 (m, 1H), 8,05 (m, 1H), 8,22 (m, 1H), 8,31 (m, 1H), 8,45 (m, 1H), 8,55 (m, 1H), 9,0 (m,2H), 9,10 (m, 1H), 9,22 (m, 1H) |
| 15 | 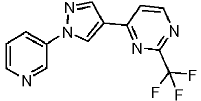 | | 2,13 | 292 | (D₆-DMSO): 7,60 (m, 1H), 8,11 (d, 1H), 8,32 (m, 1H), 8,55 (s, 1H), 8,60 (m, 1H), 9,00 (d, 1H), 9,19 (m, 1H), 9,39 (s, 1H) |
| 16 | 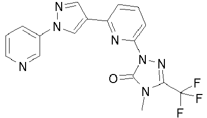 | | 2,1 | 388,1 | (D₆-DMSO): 3,40 (s, 3H), 7,56 (m, 1H), 7,77 (m, 2H), 8,04 (t, 1H), 8,28 (m, 1H), 8,35 (s, 1H), 8,57 (m, 1H), 9,10 (s, 1H), 9,16 (m, 1H) |
| 17 | 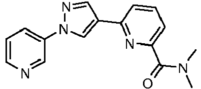 | | 1,24 | 294,1 | (D₆-DMSO): 3,04 (s, 6H), 7,39 (d, 1H), 7,55 (m, 1H), 7,84 (d, 1H), 7,93 (t, 1H), 8,29 (m, 1H), 8,37 (s, 1H), 8,55 (m, 1H), 9,12 (s, 1H), 9,17 (m, 1H) |
| 18 | 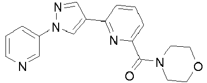 | | 1,23 | 336,2 | (D₆-DMSO): 3,67 (m, 8H), 7,46 (d, 1H), 7,56 (m, 1H), 7,86 (d, 1H), 7,95 (t, 1H), 8,36 (s, 1H), 8,55 (m, 1H), 9,10 (s, 1H), 9,17 (m, 1H) |
| 19 | 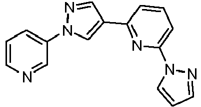 | | 2,21 | 289,2 | (D₆-DMSO): 6,59 (m, 1H), 7,57 (m, 1H), 7,72 (d, 1H), 7,78 (d, 1H), 7,81 (m, 1H), 8,00 (t, 1H), 8,33 (m, 1H), 8,49 (s, 1H), 8,57 (m, 1H), 8,91 (m, 1H), 9,20 (m, 1H), 9,27 (s, 1H) |
| 20 | 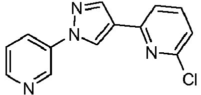 | | 2,05 | 257 | (D₆-DMSO):7,34 (d, 1H), 7,56 (m, 1H), 7,78 (d, 1H), 7,88 (t, 1H), 8,30 (m, 1H), 8,36 (s, 1H), 8,56 (m, 1H), 9,13 (s, 1H), 9,17 (m, 1H) |
| 21 | 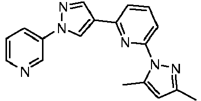 | | 2,64 | 317,2 | (D₆-DMSO): 2,22 (s, 3H), 2,72 (s, 3H), 6,12 (s, 1H), 7,56 (m, 1H), 7,66 (m, 2H), 7,95 (t, 1H), 8,28 (m, 1H), 8,36 (s, 1H), 8,57 (m, 1H), 9,09 (s, 1H), 9,16 (m, 1H) |
| 22 | 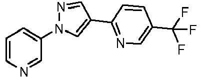 | | 2,38 | 291,1 | (D₆-DMSO): 7,57 (m, 1H), 8,01 (m, 1H), 8,22 (m, 1H), 8,29 (m, 1H), 8,46 (s, 1H), 8,57 (m, 1H), 8,91 (m, 1H), 9,17 (m, 1H), 9,27 (s, 1H) |
| 23 | 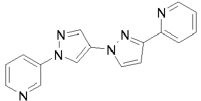 | | 1,19 | 290,1 | (D₆-DMSO): 7,09 (s, 1H), 7,37 (m, 1H), 7,61 (m, 1H), 7,91 (m, 1H), 8,04 (m, 1H), 8,32 (m, 1H), 8,37 (m, 2H), 8,59 (m, 1H), 8,63 (m, 1H), 9,18 (m, 2H) |
| 24 | 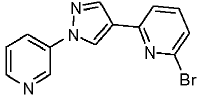 | | 2,18 | 302 | (D₆-DMSO): 5,69 (s, 1H), 7,48 (d, 1H), 7,54 (m, 1H), 7,81 (m, 1H), 8,29 (m, 1H), 8,34 (s, 1H), 8,55 (m, 1H), 9,12 (s, 1H), 9,17 (m, 1H) |
| 25 | 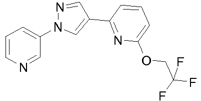 | | 2,96 | 321,1 | (D₆-DMSO): 5,11 (q, 2H), 6,82 (d, 1H), 7,49 (d, 1H), 7,58 (m, 1H), 7,82 (t, 1H), 8,28 (m, 1H), 8,39 (s, 1H), 8,56 (m, 1H), 9,15 (m, 2H) |
| 26 | 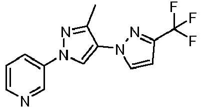 | | 2,51 | 294,1 | (D₆-DMSO): 2,39 (s, 3H), 6,96 (m, 1H), 7,56 (m, 1H), 8,22 (m, 1H), 8,29 (m, 1H), 8,54 (m, 1H), 9,01 (s, 1H), 9,09 (m, 1H) |
| 27 | 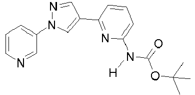 | | 2,87 | 282,1 | (D₆-DMSO): 1,50 (s, 9H), 7,41 (d, 1H), 7,56 (m, 1H), 7,62 (d, 1H), 7,76 (t, 1H), 8,23 (m, 1H), 8,28 (s, 1H), 8,55 (m, 1H), 9,00 (s, 1H), 9,13 (m, 1H), 9,19 (m, 1H) |
| 28 | 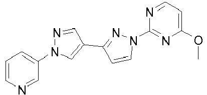 | | 1,71 | 320,1 | (D₆-DMSO): 4,07 (s, 3H), 6,85 (d, 1H), 6,90 (m, 1H), 7,56 (m, 1H), 8,24 (s, 1H), 8,29 (m, 1H), 8,54 (m, 2H), 8,69 (m, 1H), 9,03 (s, 1H), 9,17 (m, 1H) |
| 29 | 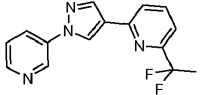 | | 2,46 | 287,1 | (D₆-DMSO):2,09 (t, 3H), 7,55 (m, 2H), 7,91 (d, 1H), 8,00 (m, 1H), 8,31 (m, 1H), 8,40 (s, 1H), 8,57 (m, 1H), 9,16 (m, 2H) |
| 30 | 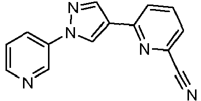 | 1,73 | | 248,1 | (D₆-DMSO): 7,55 (m, 1H), 7,85 (m, 1H), 8,1 (m, 2H), 8,3 (m, 1H), 8,4 (s, 1H), 8,55 (d, 1H), 9,15 (m, 1H), 9,25 (s, 1H) |
| 31 | 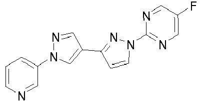 | | 1,52 | 308,1 | (D₆-DMSO): 6,92 (m, 1H), 7,57 (m, 1H), 8,23-8,34 (m, 2H), 8,56 (m, 1H), 8,64 (m, 1H), 8,93 (m, 2H), 9,05 (s, 1H), 9,19 (m, 1H) |
| 32 | 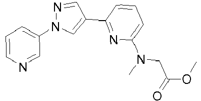 | | 2,18 | 324,1 | (D₆-DMSO): 2,08 (s, 3H), 3,63 (s, 3H), 4,39 (s, 2H), 6,55 (d, 1H), 7,03 (d, 1H), 7,53-7,59 (m, 2H), 8,21 (s, 1H), 8,24 (m, 1H), 8,54 (m, 1H), 8,92 (s, 1H), 9,12 (m, 1H) |
| 33 | 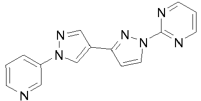 | | 1,33 | 290,1 | (D₆-DMSO): 6,92 (m, 1H), 7,45 (t, 1H), 7,56 (m, 1H), 8,26 (s, 1H), 8,29 (m, 1H), 8,55 (m, 1H), 8,69 (s, 1H), 8,87 (d, 1H), 8,90 (m, 1H), 9,06 (s, 1H), 9,17 (m, 1H) |
| 34 | 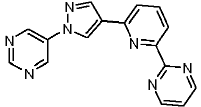 | | 1,17 | 301,1 | (D₆-DMSO): 7,54 (m, 1H), 7,75 (m, 1H), 7,95 (m, 1H), 8,13 (m,2H), 8,39 (m,3H), 8,72 (m, 1H), 9,0 (m,2H) |
| 35 | 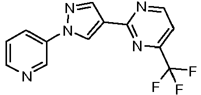 | | 2,23 | 292,1 | (D₆-DMSO): 7,57 (m, 1H), 7,79 (d, 1H), 8,36 (m, 1H), 8,44 (s, 1H), 8,59 (m, 1H), 9,17 (d, 1H), 9,22 (m, 1H), 9,26 (s, 1H) |
| 36 | 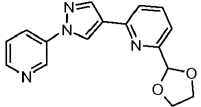 | | 1,5 | 295,1 | (D₆-DMSO): 4,01 (m, 2H), 4,15 (m, 2H), 5,78 (s, 1H), 7,39 (d, 1H), 7,57 (m, 1H), 7,79 (d, 1H), 7,88 (t, 1H), 8,29 (m, 1H), 8,35 (s, 1H), 8,56 (m, 1H), 9,09 (s, 1H), 9,17 (m, 1H) |
| 37 | 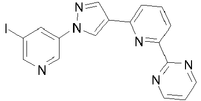 | | 2,37 | 426,9 | (D₆-DMSO): 7,54 (m, 1H), 7,90 (m, 1H), 8,05 (m, 1H), 8,23 (m, 1H), 8,42 (m, 1H), 8,75 (m,2H), 9,00 (m,2H), 9,20 (m,2H). |
| 38 | 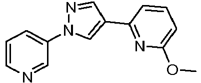 | | 2,12 | 253,1 | (D₆-DMSO): 3,96 (s, 3H), 6,68 (d, 1H), 7,40 (d, 1H), 7,60 (m, 1H), 7,75 (t, 1H), 8,32 (m, 1H), 8,38 (s, 1H), 8,57 (m, 1H), 9,16 (s, 1H), 9,19 (m, 1H) |
| 39 | 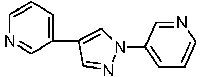 | | 0,39 | 223,1 | (D₆-DMSO): 7,45 (m, 1H), 7,55 (m, 1H), 8,1 (m, 1H), 8,25 (m, 1H), 8,35 (s, 1H), 8,45 (m, 1H), 8,55 (d, 1H), 8,95 (m, 1H), 9,1 (s, 1H), 9,15 (m, 1H) |
| 40 | 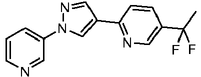 | | 2,04 | 287,1 | (D₆-DMSO): 2,04 (t, 3H), 7,56 (m, 1H), 7,91 (d, 1H), 8,01 (m, 1H), 8,29 (m, 1H), 8,41 (s, 1H), 8,56 (m, 1H), 8,77 (m, 1H), 9,17 (m, 1H), 9,19 (s, 1H) |
| 41 | 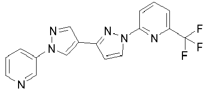 | | 3,18 | 357,1 | (D₆-DMSO): 6,95 (s, 1H), 7,56 (m, 1H), 7,81 (m, 1H), 8,24-8,31 (m, 4H), 8,55 (m, 1H), 8,63 (s, 1H), 9,05 (s, 1H), 9,16 (s, 1H) |
| 42 | 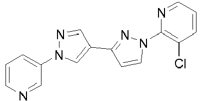 | | 1,81 | 323,1 | (D₆-DMSO): 6,84 (m, 1H), 7,55 (m, 2H), 8,18 (m, 2H), 8,29 (m, 2H), 8,54 (m, 2H), 8,96 (s, 1H), 9,15 (m, 1H) |
| 43 | 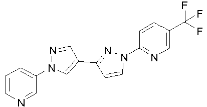 | | 3,22 | 357,1 | (D₆-DMSO): 6,96 (s, 1H), 7,56 (m, 1H), 8,17 (d, 1H), 8,28 (m, 2H), 8,38 (m, 1H), 8,55 (m, 1H); 8,72 (s, 1H), 8,85 (s, 1H), 9,05 (s, 1H), 9,17 (m, 1H) |
| 44 | 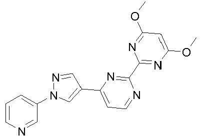 | | 1,56 | 362,1 | (D₆-DMSO): 4,00 (s, 6H), 6,35 (s, 1H), 7,57 (m, 1H), 7,93 (d, 1H), 8,32 (m, 1HG), 8,53 (s, 1H), 8,58 (m, 1H), 8,93 (d, 1H), 9,18 (m, 1H), 9,33 (s, 1H) |
| 45 | 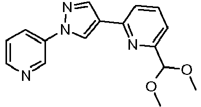 | | 1,75 | 297,1 | (D₆-DMSO): 3,40 (s, 6H), 5,31 (s, 1H), 7,35 (d, 1H), 7,57 (m, 1H), 7,62 (d, 1H), 7,77 (m, 1H), 8,30 (m, 1H), 8,34 (s, 1H), 8,56 (m, 1H), 9,07 (s, 1H), 9,16 (m, 1H) |
| 46 | 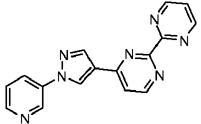 | | 0,86 | 302,1 | (D₆-DMSO): 7,60 (m, 1H), 7,68 (m, 1H), 8,00 (m, 1H), 8,38 (m, 1H), 8,59 (m, 2H), 9,04 (m, 3H), 9,22 (m, 1H), 9,45 (s, 1H) |
| 47 | 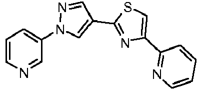 | | 1,55 | 307 | (D₆-DMSO): 7,36 (m, 1H), 7,58 (m, 1H), 7,92 (t, 1H), 8,16 (d, 1H), 8,22 (s, 1H), 8,32-8,36 (m, 2H), 8,58 (m, 1H), 8,63 (m, 1H), 9,19 (m, 1H), 9,22 (s, 1H) |
| 48 | 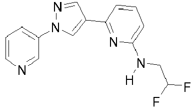 | | 1,52 | 302,1 | (D₆-DMSO): 3,79 (m, 2H), 6,17 (m, 1H), 6,47 (d, 1H), 6,73 (m, 1H), 6,99 (d, 1H), 7,45 (t, 1H), 7,54 (m, 1H), 8,24 (s, 1H), 8,26 (m, 1H), 8,54 (m, 1H), 8,64 (s, 1H), 9,15 (m, 1H) |
| 49 | 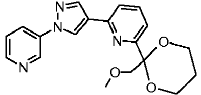 | 1,69 | | 353,2 | |
| 50 | 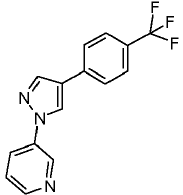 | | 2,98 | 290,1 | (D₆-DMSO): 7,55 (m, 1H), 7,75 (m, 2H), 7,95 (m, 2H), 8,3 (d, 1H), 8,35 (s, 1H), 8,55 (m, 1H), 9,15 (m, 2H) |
| 51 | 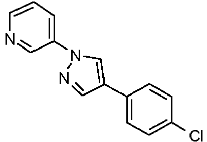 | | 2,71 | 256,1 | (D₆-DMSO): 7,45 (m, 2H), 7,55 (m, 1H), 7,75 (m, 2H), 8,25 (m, 2H), 8,55 (br, 1H), 9 (s, 1H), 9,15 (br, 1H) |
| 52 | 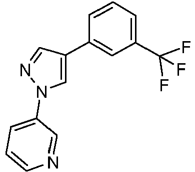 | | 2,99 | 290,1 | (D₆-DMSO): 7,55-7,7 (m, 3H), 8,05 (m, 2H), 8,3 (d, 1H), 8,4 (s, 1H), 8,55 (m, 1H), 9,15 (m, 2H) |
| 53 | 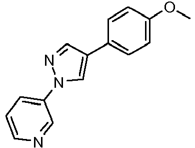 | | 2,12 | 252,1 | (D₆-DMSO): 3,8 (s, 3H), 7 (m, 2H), 7,55 (m, 1H), 7,6 (m 2H), 8,15 (s, 1H), 8,25 (d, 1H), 8,5 (m, 1H), 8,9 (s, 1H), 9,1 (m, 1H) |
| **54** | 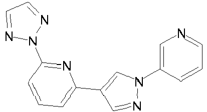 | | 1,63 | 290,1 | (D₆-DMSO): 7,59-7,62 (m, 1H), 7,88-7,90 (m, 1H), 7,91-7,93 (m, 1H), 8,13(t, 1H), 8,23 (s, 2H), 8,36-8,38 (m,1H), 8,46 (s, 1H), 8,58-8,59 (m, 1H), 9,21-9,22 (m, 1H), 9,28 (s, 1H) |
| **55** | 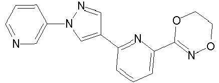 | | 1,4 | 308,2 | (D6-DMSO): 4,21(t,2H), 4,56(t,2H), 7,54-7,60 (m, 1H), 7,64-7,69 (m, 1H), 7,88-7,95(m,2H), 8,33-8,35 (m, 1H), 8,38 (s, 1H), 8,56-8,57 (m, 1H), 9,18-9,20(m,2H) |
| **56** | 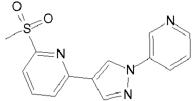 | | 1,31 | 301,1 | (D6-DMSO): 3,42(s,3H), 7,59-7,63 (m, 1H), 7,87-7,89 (m, 1H), 8,11-8,13 (m, 1H), 8,20 (t, 1H), 8,33-8,36 (m, 1H), 8,54 (s, 1H), 8,58-8,60 (m, 1H), 9,21-9,22 (m, 1H), 9,37 (s, 1H) |
| **57** | 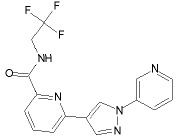 | | 2,12 | 348,2 | (D6-DMSO): 4,12-4,21 (m, 2H), 7,57-7,63(m,2H), 7,91-7,94 (m, 1H), 7,98-8,06(m,2H), 8,25-8,29 (m, 1H), 8,54-8,59 (m, 1H), 8,63 (s, 1H), 9,15-9,16 (m, 1H), 9,22 (broad, NH), 9,31 (s, 1H) |
| **58** | 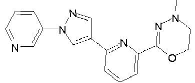 | 1,68 | 1,54 | 321,1 | (D6-DMSO): 2,78(s,3H), 3,02-3,05(m,2H), 4,50-4,52(m,2H), 7,56-7,59 (m, 1H), 7,63 (d, 1H), 7,75 (d, 1H), 7,77-7,84 (m, 1H), 8,31-8,35(m,2H), 8,55-8,56 (m, 1H), 9,12 (s, 1H), 9,18-9,19 (m, 1H) |
| **59** | 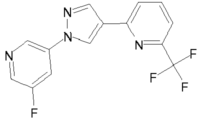 | 3,14 | 3,1 | 309,1 | (D6-DMSO): 7,77-7,79 (m, 1H), 8,10-8,19(m,2H), 8,36-8,39 (m, 1H), 8,49-8,50 (m, 1H), 8,60-8,61 (m, 1H), 9,12-9,13 (m, 1H), 9,32-9,33 (m, 1H) |
| **60** | 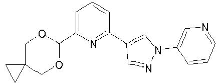 | 2,12 | 1,97 | 335,2 | (D6-DMSO): 0,38-0,42(m,2H), 0,66-0,70(m,2H), 3,37-3,40(m,2H), 4,30-4,33(m,2H), 5,62 (s, 1H), 7,44-7,46 (m, 1H), 7,56-7,60 (m, 1H), 7,80-7,82 (m, 1H), 7,88-7,92 (m1H), 8,31-8,35 (m, 1H), 8,38 (s, 1H), 8,55-8,57 (m, 1H), 9,17 (s, 1H), 9,19-9,20 (m, 1H) |
| **61** | 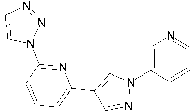 | | 1,68 | 290,2 | (D6-DMSO): 7,61-7,64 (m, 1H), 7,92-7,94 (m, 1H), 7,98-8,00 (m, 1H), 8,08-8,09 (m, 1H), 8,16 (t, 1H), 8,3-8,35 (m, 1H), 8,59-8,62(m,2H), 9,21-9,23(m,2H), 9,44-9,45 (m, 1H) |
| **62** | 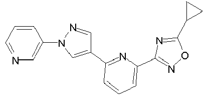 | | 2,16 | 331,1 | (D6-DMSO): 1,22-1,25 (m, 2H), 1,29-1,33 (m, 2H), 2,46-2,48 (m, 1H), 7,57-7,61 (m, 1H), 7,87-7,90 (m, 1H), 7,98-8,00 (m, 1H), 8,03-8,08 (m, 1H), 8,33-8,36 (m, 1H), 8,43 (s, 1H), 8,57-8,58 (m, 1H), 9,20-9,21 (m, 1H), 9,23 (s, 1H) |
| **63** | 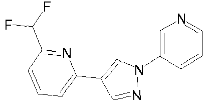 | | 2,02 | 273,0 | (D6-DMSO): 6,97 (t, 1H), 7,56-7,61(m,2H), 7,98 (d, 1H), 8,06 (t, 1H), 8,32-8,35 (m, 1H), 8,43 (s, 1H), 8,57-8,58 (m, 1H), 9,18-9,19 (m, 1H), 9,24 (s, 1H) |
| **64** | 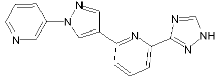 | | 1,14 | 290,1 | (D6-DMSO): 7,61-7,64 (m, 1H), 7,85-7,98(m,2H), 8,03 (t, 1H), 8,14 (s, 1H), 8,27-8,30 (m, 1H), 8,59-8,60 (m, 1H), 8,67 (s, 1H), 9,16-9,17 (m, 1H), 9,42 (s, 1H) |
| **65** | 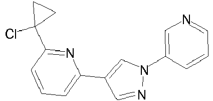 | | 3,22 | 297,1 | (D6-DMSO): 1,51-1,54 (m, 2H), 1,81-1,85 (m, 2H), 7,57-7,60 (m, 1H), 7,63-7,68(m,2H), 7,86-7,90 (m, 1H), 8,30-8,33 (m, 1H), 8,36 (s, 1H), 8,55-8,57 (m, 1H), 9,16 (s, 1H), 9,18-9,19 (m, 1H) |
| **66** | 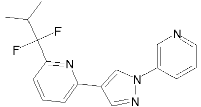 | 3,22 | | 315,1 | (D6-DMSO): 1 (d, 6H), 2,9 (sep, 1H), 7,5 (d, 1H), 7,55 (dd, 1H), 8,4 (m, 1H), 8,0 (m, 1H), 8,3 (m, 1H), 8,4 (s, 1H), 8,55 (d, 1H), 9,15 (s, 1H), 9,2 (m, 1H) |
| **67** | 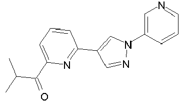 | 2,84 | | 293,2 | (CD3CN): 1,2 (d, 6H), 4,25 (sep, 1H), 7,5 (m, 1H), 7,85 (m, 2H), 7,95 (m, 1H), 8,2 (m, 1H), 8,35 (s, 1H), 8,55 (m, 1H), 8,8 (s, 1H), 9,2 (m, 1H) |
| **68** | 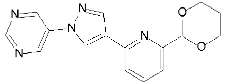 | 1,56 | 1,51 | 310,1 | (D6-DMSO): 1,47-1,50 (m, 1H), 2,02-2,09 (m, 1H), 3,96-4,02(m,2H), 4,16-4,20(m,2H), 5,54 (s, 1H), 7,41-7,43 (m, 1H), 7,79-7,81 (m, 1H), 7,89-7,93 (m, 1H), 8,46 (s, 1H), 9,17 (s, 1H), 9,24 (s, 1H), 9,40(s,2H) |
| **69** | 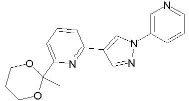 | | 1,78 | 323,2 | (D6-DMSO): 1,36-1,42 (m, 1H), 1,51 (s, 3H), 1,86-1,92 (m, 1H), 3,74-3,80(m,2H), 3,88-3,92(m,2H), 7,37 (d, 1H), 7,57-7,60 (m, 1H), 7,73-7,75 (m, 1H), 7,88-7,92 (m, 1H), 8,32-8,35 (m, 1H), 8,37 (s, 1H), 8,56-8,58 (m, 1H), 9,14 (s, 1H), 9,19-9,20 (m, 1H) |
| **70** | 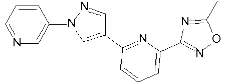 | | 1,66 | 305,1 | (D6-DMSO): 2,71(s,3H), 7,61-7,64 (m, 1H), 7,91-7,94 (m, 1H), 8,00-8,02 (m, 1H), 8,06-8,09 (m, 1H), 8,37-8,41 (m, 1H), 8,45 (s, 1H), 8,58-8,60 (m, 1H), 9,22-9,23 (m, 1H), 9,26 (s, 1H) |
| **71** | 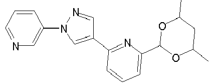 | | 2,37 | 337,2 | (D6-DMSO): 1,20 (d, 6H), 1,22 (m, 1H), 1,68 (m, 1H), 4,00 (m, 2H), 5,55 (s, 1H), 7,41 (d, 1H), 7,55 (m, 1H), 7,75 (d, 1H), 7,86 (m, 1H), 8,28 (m, m1H), 8,33 (s, 1H), 8,54 (m, 1H), 9,07 (s, 1H), 9,16 (m, 1H) |
| **72** | 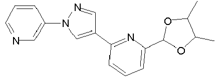 | | | 323,2 | *Bemerkung: Isomerengemisch 58% (logP_{HCOOH} 2,13) und 42% (logP_{HCOOH} 2, 09)* |
| **73** | 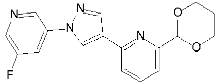 | 2,13 | 2,12 | 327,2 | (D6-DMSO): 1,40-1,51 (m, 1H), 2,02-2,11 (m, 1H), 3,96-4,04(m,2H), 4,16-4,20(m,2H), 5,54 (s, 1H), 7,40-7,42 (m, 1H), 7,78-7,81 (m, 1H), 7,89-7,92 (m, 1H), 8,32-8,36 (m, 1H), 8,42 (s, 1H), 8,57-8,58 (m, 1H), 9,11-9,12 (m, 1H), 9,23 (s, 1H) |
| **74** | 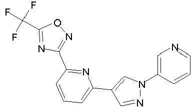 | | 2,77 | 359,1 | (D6-DMSO): 7,58-7,62 (m, 1H), 8,01-8,04 (m, 1H), 8,08-8,16(m,2H), 8,34-8,37 (m, 1H), 8,47 (s, 1H), 8,58-8,59 (m, 1H), 9,21-9,22 (m, 1H), 9,28 (s, 1H) |
| **75** | 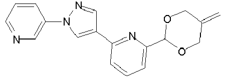 | | 1,9 | 321,1 | (D6-DMSO): 4,49(m,2H), 4,55(m,2H), 5,06(s,2H), 5,69 (s, 1H), 7,39 (d, 1H), 7,54 (m, 1H), 7,78 (d, 1H), 7,86 (t, 1H), 8,30 (m, 1H), 8,33 (s1H), 8,54 (m, 1H), 9,08 (s, 1H), 9,16 (m, 1H) |
| **76** | 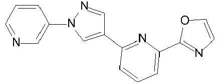 | 1,69 | 1,575 | 290,1 | (D6-DMSO): 7,48-7,49 (m, 1H), 7,58-7,61(m,2H), 7,93-8,06(m,3H), 8,34-8,37(m,2H), 8,57-8,59 (m, 1H), 9,20-9,21 (m, 1H), 9,25 (s, 1H) |
| **77** | 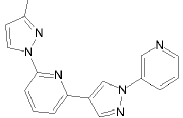 | | 2,51 | 303,2 | (D6-DMSO): 2,31(s,3H), 6,38-6,39 (m, 1H), 7,55-7,59 (m, 1H), 7,64-7,70(m,2H), 7,93-7,98 (m, 1H), 8,29-8,33 (m, 1H), 8,47-8,48 (m, 1H), 8,56-8,57 (m, 1H), 8,77-8,78 (m, 1H), 9,19-9,20 (m, 1H), 9,24-9,25 (m, 1H) |
| **78** | 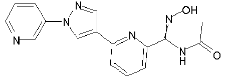 | | 1,49 | 305,1 | (D6-DMSO): 2,21(s,3H), 7,09 (broad, 2H), 7,60-7,63 (m, 1H), 7,81-7,83 (m, 1H), 7,92-7,95(m,2H), 8,29-8,33 (m, 1H), 8,57-8,59 (m, 1H), 8,63 (s, 1H), 9,19-9,20 (m, 1H), 9,47 (s, 1H) |
| **79** | 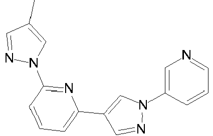 | | 2,59 | 303,1 | (D6-DMSO): 2,16-2,17(m,3H), 7,56-7,72(m,4H), 7,94-7,98 (m, 1H), 8,30-8,33 (m, 1H), 8,47-8,48 (m, 1H), 8,56-8,58 (m, 1H), 8,66-8,67 (m, 1H), 9,19-9,20 (m, 1H), 9,24-9,25 (m, 1H) |
| **80** | 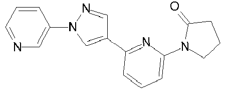 | | 1,86 | 306,1 | (D6-DMSO): 2,04-2,13(m,2H), 2,57-2,62(m,2H), 4,16(t,2H), 7,49-7,52 (m, 1H), 7,54-7,58 (m, 1H), 7,79-7,83 (m, 1H), 8,13-8,16 (m, 1H), 8,26-8,30 (m, 1H), 8,34 (s, 1H), 8,52-8,56 (m, 1H), 9,07 (s, 1H), 9,16-9,17 (m, 1H) |
| **81** | 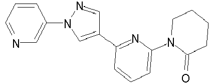 | | 1,59 | 320,2 | (D6-DMSO): 1,83-1,95 (m, 4H), 2,50-2,53(m,2H), 4,00(t,2H), 7,54-7,57(m,2H), 7,60-7,63 (m, 1H), 7,77-7,81 (m, 1H), 8,27-8,30 (m, 1H), 8,34 (s, 1H), 8,54-8,56 (m, 1H), 9,08 (s, 1H), 9,16-9,17 (m, 1H) |
| **82** | 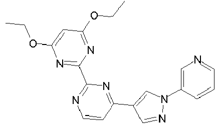 | | 2,23 | 390,1 | (D6-DMSO): 1,36 (t, 6H), 4,44(q,4H), 6,28 (s, 1H), 7,56-7,60 (m, 1H), 7,92 (d, 1H), 8,29-8,33 (m, 1H), 8,52 (s, 1H) 8,58-8,60 (m, 1H), 8,93 (d, 1H), 9,17-9,18 (m, 1H), 9,32 (s, 1H) |
| **83** | 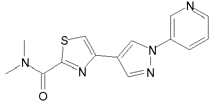 | | 1,51 | 300,1 | (D6-DMSO): 2,53(s,6H), 7,54-7,57 (m, 1H), 8,04 (s, 1H), 8,25 (s, 1H), 8,26-8,28 (m, 1H), 8,54-8,55 (m, 1H), 8,97-8,98 (m, 1H), 9,14-9,15 (m, 1H) |
| **84** | 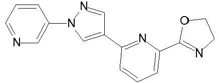 | 1,45 | 0,9 | 292,1 | (D6-DMSO): 4,03(t,2H), 4,47(t,2H), 7,55-7,61 (m, 1H), 7,85-7,89 (m, 1H), 7,92-7,98(m,2H), 8,32-8,37 (m, 1H), 8,39 (s, 1H), 8,56-8,59 (m, 1H), 9,18-9,23(m,2H) |
| **85** | 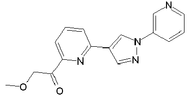 | | 1,66 | 295,1 | (D6-DMSO): 3,45 (s, 3H), 5,2 (s, 2H), 7,55 (dd, 1H), 7,8 (m, 1H), 8,05 (m 2H), 8,3 (d, 1H), 8,45 (s, 1H), 8,6 (m, 1H), 9,2 (m, 2H) |
| **86** | 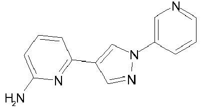 | 1,3 | 0,29 | 238,1 | (D6-DMSO): 5,88 (broad, 1H), 6,35 (d, 1H), 6,93 (d, 1H), 7,42 (t, 1H), 7,54-7,58 (m, 1H), 8,22 (m, 1H), 8,26-8,30 (m, 1H), 5,53-8,55 (m1H), 8,97 (s, 1H), 9,15 (d, 1H) |
| **87** | 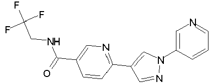 | 1,69 | 1,53 | 348,1 | (D6-DMSO): 4,10-4,19(m,2H), 7,57-7,61 (m, 1H), 7,94-7,96 (m, 1H), 8,29-8,36(m,2H), 8,48 (s, 1H), 8,56-8,58 (m, 1H), 9,05-9,06 (m, 1H), 9,20 (d, 1H), 9,26 (t, 1H), 9,33 (s, 1H) |
| **88** | 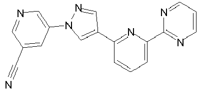 | | 1,91 | 326,0 | (CDC13): 7,30-7,69 (m,3H), 7,88-7,95 (m, 1H), 8,22-9,05 (m,6H), 9,30 (m, 1H) |
| **89** | 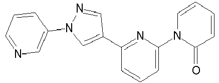 | | 1,37 | 316,1 | (D6-DMSO): 6,36-6,40 (m, 1H), 6,50-6,53 (m, 1H), 7,50-7,58(m,2H), 7,65-7,68 (m, 1H), 7,83-7,86 (m, 1H), 7,98-8,03(m,2H), 8,28-8,32 (m, 1H), 8,40 (s, 1H), 8,55-8,56 (m, 1H), 9,16-9,18(m,2H) |
| **90** | 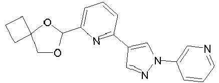 | 2,53 | 2,37 | 335,1 | (D6-DMSO): 1,56-1,69 (m, 1H), 1,70-1,78 (m, 1H), 2,18-2,26(m,2H), 2,30-2,40(m,2H), 4,00-4,07 (m, 1H), 4,19 (d, 1H), 5,81 (s, 1H), 7,35 (d, 1H), 7,56-7,59 (m, 1H), 7,81 (d,1H), 7,91 (t, 1H), 8,31-8,33 (d, 1H), 8,39 (s, 1H), 8,55-8,57 (m, 1H), 9,18-9,20(m,2H) |
| **91** | 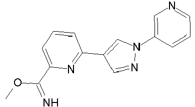 | | 0,84 | 280,2 | (D6-DMSO): 3,94(s,3H), 7,56-7,59 (m, 1H), 7,66-7,68 (m, 1H), 7,90-7,93 (m, 1H), 7,96-8,00 (m, 1H), 8,29-8,33 (m, 1H), 8,50 (s, 1H), 8,55-8,57 (m, 1H), 9,19-9,20 (m, 1H), 9,32 (s, 1H), 9,49 (broad, 1H) |
| **92** | 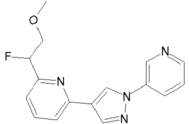 | 1,98 | | 299,2 | (CD3CN): 3,4 (s, 3H), 3,9 (m, 2H), 5,7 (m, 1H), 7,3 (m, 1H), 7,5 (m, 1H) 7,6 (d, 1H), 7,8 (m, 1H), 8,15 (d, 1H), 8,25 (s, 1H), 8,55 (m, 1H), 8,7 (s, 1H), 9,1 (m, 1H) |
| **93** | 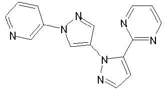 | | 1,16 | 290,1 | |
| **94** | 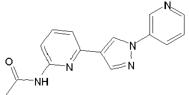 | | 1,31 | 280,1 | (D6-DMSO): 2,13(s,3H), 7,46-7,48 (m, 1H), 7,54-7,58(m,2H), 7,78 (t, 1H), 7,89 (d, 1H), 8,23-8,27 (m, 1H), 8,29 (s, 1H), 8,54-8,56 (m, 1H), 9,02 (s, 1H), 9,12-9,13 (m, 1H), 10,15 (s, 1H) |
| **95** | 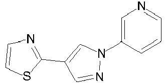 | | 1,32 | 229,0 | (D6-DMSO): 7,54-7,56 (m, 1H), 7,66 (d,1H), 7,84 (d, 1H), 8,26 (s, 1H), 8,28-8,32 (m, 1H), 8,55-8,57 (m, 1H), 9,13 (d, 1H), 9,16-9,17 (m, 1H) |
| **96** | 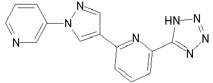 | 0,32 | 1,36 | 291,1 | (D6-DMSO): 5,7 (br, 1H), 7,65 (dd, 1H), 8-8,15 (m, 3H), 8,3 (d, 1H), 8,6 (m, 1H), 8,65 (s, 1H), 9,2 (m, 1H), 9,4 (s, 1H) |
| **97** | 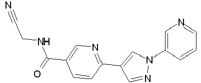 | 1,2 | 0,95 | 305,1 | (D6-DMSO): 4,4 (s, 2H), 7,6 (dd, 1H), 7,95 (d, 1H), 8,3 (d, 1H), 8,35 (d, 1H), 8,5 (s, 1H), 8,6 (d, 1H), 9,05 (m, 1H), 9,2 (m, 1H), 9,3-9,4 (m, 2H), |
| **98** | 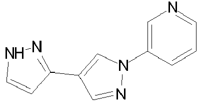 | | 0,78 | 212,1 | (D6-DMSO): 6,51 (broad, 1H), 7,48-7,57 (m, 1H), 7,71 (broad, 1H), 8,08 (broad, 1H), 8,19-8,27 (m, 1H), 8,52 (broad, 1H), 8,81 (s, 1H), 9,12 (broad, 1H) |
| **99** | 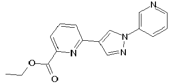 | 1,96 | 1,83 | 295,1 | (D6-DMSO): 1,37 (t, 3H), 4,39(q,2H), 7,57-7,61 (m, 1H), 7,88-7,93 (m, 1H), 8,01-8,07(m,2H), 8,32-8,35 (m, 1H), 8,42 (s, 1H), 8,56-8,58 (m, 1H), 9,18-9,22 (m, 1H), 9,33 (s, 1H) |
| **100** | 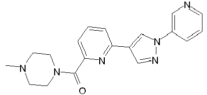 | | 0,46 | 349,1 | (D6-DMSO): 2,30-2,40 (m, 1H), 3,50(m,2H), 3,67(m,2H), 7,39-7,42 (m, 1H), 7,54-7,58 (m, 1H), 7,83-7,85 (m, 1H), 7,94 (t, 1H), 8,27-8,31 (m, 1H), 8,34-8,35 (m, 1H), 8,55-8,57 (m, 1H), 9,10-9,11 (m, 1H), 9,16-9,17 (m, 1H) |
| **101** | 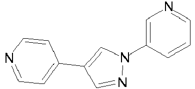 | 1,24 | 0,01 | 223,1 | (D6-DMSO): 7,55 (m, 1H), 7,7 (d, 2H), 8,25 (d, 1H), 8,4 (s, 1H), 8,55 (m, 3H), 9,15 (m, 1H), 9,2 (s, 1H) |
| **102** | 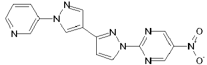 | | 1,64 | 335,1 | (D6-DMSO): 7,06 (d, 1H), 7,54-7,58 (m, 1H), 8,29-8,32(m,2H), 8,55-8,57 (m, 1H), 8,79-8,80 (m, 1H), 9,13-9,14 (m, 1H), 9,17-9,18 (m, 1H), 9,57 (s, 1H) |
| **103** | 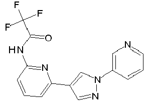 | 2,28 | 2,21 | 334,0 | (D6-DMSO): 7,60 (m, 1H), 7,75 (m, 1H), 7,80 (m, 1H), 7,99 (m, 1H), 8,32 (m, 1H), 8,38 (m, 1H), 8,60 (m, 1H), 9,20 (m,2H), 11,9 (m, 1H) |
| **104** | 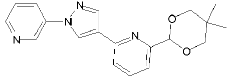 | 2,55 | 2,44 | 337,1 | (D6-DMSO): 0,78(s,3H), 1,25 (s,3H), 3,66-3,69(m,4H), 5,44 (s, 1H), 7,44 (d, 1H), 7,53-7,56 (m, 1H), 7,76 (m, 1H), 7,88 (m, 1H), 8,28 (m1H), 8,30 (s, 1H), 8,54 (m, 1H), 9,07 (s, 1H), 9,15 (m1H) |
| **105** | 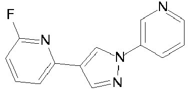 | | 1,77 | 241,1 | (D6-DMSO): 6,97-7,00 (m, 1H), 7,54-7,58 (m, 1H), 7,71-7,74 (m, 1H), 7,98-8,04 (m, 1H), 8,27-8,31 (m, 1H), 8,35 (s, 1H), 8,55-8,57 (m, 1H), 9,13-9,14 (m, 1H), 9,16-9,17 (m, 1H) |
| **106** | 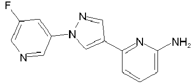 | 1,59 | 0,64 | 256,1 | (D6-DMSO): 5,90 (m,2H), 6,38 (m, 1H), 6,95 (m, 1H), 7,43 (m, 1H), 8,28 (m,2H), 8,57 (m, 1H), 9,05 (m,2H) |
| **107** | 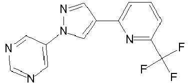 | 2,38 | 2,36 | 292,1 | (D6-DMSO): 7,77-7,79 (m, 1H), 8,11-8,19(m,2H), 8,54 (s, 1H), 9,19 (s, 1H), 9,34 (s, 1H), 9,41(s,2H) |
| **108** | 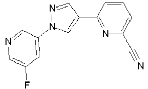 | | 2,2 | 266,1 | (D6-DMSO): 7,89-7,94 (m, 1H), 8,12-8,15(m,2H), 8,34-8,38 (m, 1H), 8,52 (s, 1H), 8,60-8,61 (m, 1H), 9,12-9,13(m,4H), 9,37 (s, 1H) |
| **109** | 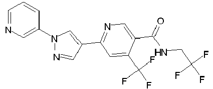 | 2,18 | 2,11 | 416,0 | (D6-DMSO): 4,12 (m,2H), 7,60 (m, 1H), 8,32 (m,2H), 8,60 (m,2H), 8,87 (m, 1H), 9,20 (m, 1H), 9,45 (m,2H) |
| **110** | 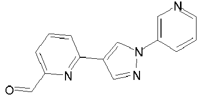 | | 1,56 | 251,1 | (D6-DMSO): 7,54-7,59 (m, 1H), 7,77-7,82 (m, 1H), 8,07-8,09(m,2H), 8,29-8,34 (m, 1H), 8,45 (s, 1H), 8,56-8,58 (m, 1H), 9,17-9,18 (m, 1H), 9,22 (s, 1H), 10,05 (s, 1H) |
| **111** | 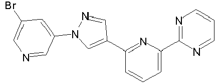 | | 2,17 | 379,0 | (D6-DMSO): 7,55 (m, 1H), 7,91 (m, 1H), 8,03 (m, 1H), 8,21 (m, 1H), 8,45 (m, 1H), 8,65 (m,2H), 8,99 (m,2H), 9,20 (m,2H) |
| **112** | 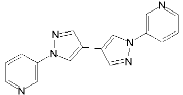 | | 1,37 | 289,1 | (D6-DMSO): 7,54-7,57(m,2H), 8,11 (s,2H), 8,18-8,23(m,2H), 8,51-8,55(m,2H), 8,79-8,82(m,2H), 8,84(s,2H), 9,09-9,10(m,2H) |
| **113** | 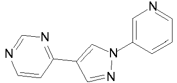 | | 0,89 | 224,1 | (D6-DMSO): 7,55 (m, 1H), 7,85 (d, 1H), 8,3 (d, 1H), 8,5 (s, 1H), 8,6 (m, 1H), 8,8 (d, 1H), 9,1 (s, 1H), 9,2 (m, 1H), 9,3 (s, 1H) |
| **114** | 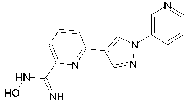 | | 0,86 | 281,0 | (D6-DMSO): 5,96(m,2H), 7,56 (m, 1H), 7,70-7,83(m,3H), 8,28 (m1H), 8,49 (s, 1H), 8,55(m,2H), 9,18 (m, 1H), 9,30 (s, 1H), 9,72 (s, 1H) |

Gemäß den oben beschriebenen Herstellverfahren wurden die folgenden Zwischenprodukte der Formel (VI) erhalten.

| | | | | | |
|---|---|---|---|---|---|
| **VI-1** | 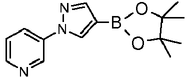 | | 2,19 | 272,2 | (d₆-DMSO): 1,30(s,12H), 7,51(m,1H), 7,90(s,1H), 8,25(m,1H), 8,51(m,1H), 8,73(s,1H), 9,12(m,1H) |
| **VI-2** | 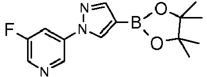 | | 2,88 | 290,1 | (d₆-DMSO): 1,30 (s,12H), 7,96(s,1H), 8,28-8,32(m,1H), 8,55-8,56(m,1H), 8,91(s,1H), 9,08(s,1H) |
| **VI-3** | 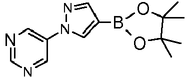 | | 2,10 | 273,2 | (d₆-DMSO): 1,30(s,12H), 8,00(s,1H), 8,94(s,1H), 9,15(s,1H), 9,36(s,2H) |

### Biologische Beispiele

### Beispiel Nr. 1

### Myzus-Test (Spritzbehandlung)

| | |
|---|---|
| Lösungsmittel: | 78 GewichtsteileAceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 80 % bei einer Aufwandmenge von 500g/ha : 35,105,110,112

Bei diesem Test zeigen z. B. die folgenden Verbindungen der Herstellungsbeispiele Wirkung von 80 % bei einer Aufwandmenge von 100g/ha : 25

Bei diesem Test zeigen z. B. die folgenden Verbindungen der Herstellungsbeispiele Wirkung von 90 % bei einer Aufwandmenge von 500g/ha :
8,13,14,16,29,40,43,50,51,61,65,87,92,95,96,97,103,106,107,108,109

Bei diesem Test zeigen z. B. die folgenden Verbindungen der Herstellungsbeispiele Wirkung von 100 % bei einer Aufwandmenge von 500g/ha :
1,2,3,4,5,6,7,9,10,11,12,15,17,18,19,21,22,23,24,26,27,28,30,31,32,33,34,36,37,38,39,42, 44,45,46,47,48,49,54,56,57,58,59,60,62,63,64,66,67,68,69,70,71,72,73,74,75,76,77,78,79, 80,81, 82,83,84,85,86,88,89,90,91,93,94,98,99,100,101,102

Bei diesem Test zeigen z. B. die folgenden Verbindungen der Herstellungsbeispiele Wirkung von 100 % bei einer Aufwandmenge von 100g/ha : 55

### Beispiel Nr. 2

### Phaedon-Test (Spritzbehandlung)

| | |
|---|---|
| Lösungsmittel: | 78,0 GewichtsteileAceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pektinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 500g/ha : 16,22

### Beispiel Nr. 3

### Spodoptera frugiperda-Test (Spritzbehandlung)

| | |
|---|---|
| Lösungsmittel: | 78,0 GewichtsteileAceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*zein mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 500g/ha: 16, 32

### Beispiel Nr. 4

### Tetranychus-Test; OP-resistent (Spritzbehandlung)

| | |
|---|---|
| Lösungsmittel: | 78,0 GewichtsteileAceton |
| | 1,5 Gewichtsteile Dimethylformamid |
| Emulgator : | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben (*Phaseolus vulgaris*), die von allen Stadien der Gemeinen Spinnmilbe (*tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90 % bei einer Aufwandmenge von 500g/ha : 34

## Patentansprüche

1. Nicht-therapeutische Verwendung von Verbindungen der Formel (I), 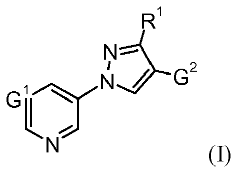 worin
G¹ für N, CH oder C-Halogen steht,
R¹ für Wasserstoff oder Alkyl steht und
G² für gegebenenfalls substituiertes Heterocyclyl, für gegebenenfalls substituiertes Heteroaryl oder für gegebenenfalls substituiertes Aryl steht,
sowie Salze, Metallkomplexe und N-Oxide der Verbindungen der Formel (I), zur Bekämpfung von tierischen Schädlingen.

2. Verbindungen der Formel (I) 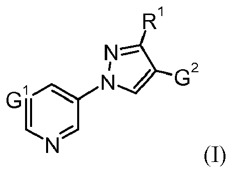 worin
G¹ für N, CH, C-Halogen C-Nitro, C-Cyano, C-Alkyl, C-Haloalkyl, C-Cycloalkyl, C-Alkoxy, C-Haloalkoxy steht,
R¹ für Wasserstoff, Alkyl Haloalkyl, Cycloalkyl, Halogen, Cyano, Alkoxy, Haloalkoxy, Amino, Alkylamino, Dialkylamino oder Alkylthio steht,
G² für A- R²ₐ, D- R³_{b} oder E- R⁴_{c} steht, worin
A für Heterocyclyl aus der Reihe 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, Oxazolin-2-yl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 5,6-Dihydro-[1,3,4]-oxadiazin-2-yl, 5,6-Dihydro-[1,3,4]-thiadiazin-2-yl, 5,6-Dihydro-[1,4,2]-dioxazin-3-yl, Hydroxypyridyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl und 1,2,4-Hexahydrotriazin-3-yl steht,
R² für einen Rest aus der Reihe Halogen, Cyano, Nitro, Alkyl, Halogenalkyl, gegebenenfalls substituiertes Cycloalkyl, Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, gegebenenfalls substituiertes Cycloalkylalkyl, Alkylcarbonyl, Alkoxycarbonyl, Aminocarbonyl, Pyridyl und Pyrimidyl, wobei Pyridyl und Pyrimidyl selbst wiederum substituiert sein können durch Halogen, Alkyl, Halogenalkyl, Alkoxy und Halogenalkoxy, steht,
a für eine Zahl aus der Reihe 0, 1, 2 und 3 steht,
D für einen Heteroarylrest aus der Reihe Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazolyl, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl steht,
R³ für einen Rest aus der Reihe gegebenenfalls substituiertes Cycloalkyl, gegebenenfalls substituiertes Cycloalkenyl, gegebenenfalls substituiertes Cycloalkylalkyl, wobei im Cycloalkylteil des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, Bis(alkoxy)alkyl, Bis(halogenalkoxy)alkyl, Alkoxy(alkylsulfanyl)alkyl, Alkoxy(alkylsulfinyl)alkyl, Alkoxy(alkylsulfonyl)alkyl, Bis(alkylsulfanyl)alkyl, Bis(halogenalkylsulfanyl)alkyl, Bis(hydroxyalkylsulfanyl)alkyl, Alkoxycarbonylalkyl, alpha-Hydroxyimino-alkoxycarbonylalkyl, alpha-Alkoxyimino-alkoxycarbonylalkyl, C(X)NR⁵R⁶, worin X für Schwefel, oder NOH steht, R⁵ für Wasserstoff oder Alkyl steht und R⁶ für einen Rest aus der Reihe Wasserstoff, Alkyl, Halogenalkyl, Cyanoalkyl, Alkinyl, Cycloalkyl, Cycloalkylalkyl, Alkylcarbonyl, Alkoxyalkyl, Alkoxycarbonyl, Alkoxycarbonylalkyl, Alkylthioalkyl, Aryl, Arylalkyl und Hetarylalkyl stehen oder X für Schwefel, NOH, NR¹⁵ oder Sauerstoff steht und R⁵ und R⁶ gemeinsam mit dem Stickstoffatom, an das sie gebunden sind, einen Ring bilden, der gegebenenfalls ein oder mehrere weitere Heteroatome aus der Reihe NH, NCH₃, NC₂H₅, Sauerstoff und Schwefel enthält, wobei zwei Sauerstoffatome nicht unmittelbar benachbart sein dürfen oder R⁵ und R¹⁵ gemeinsam mit den Stickstoffatomen, an die sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen, NR⁷R⁸, worin R⁷ für Wasserstoff oder Alkyl steht und R⁸ für Alkinyl, Cycloalkylalkyl, Alkoxy, Halogenalkoxy, Alkoxycarbonylalkyl, Alkylthioalkyl, Arylalkyl oder Hetarylalkyl steht oder R⁷ und R⁸ gemeinsam mit dem Stickstoffatom, an das sie gebunden sind, einen Ring bilden, der gegebenenfalls ein oder mehrere weitere Heteroatome aus der Reihe NH, NCH₃, NC₂H₅, Sauerstoff und Schwefel enthält, wobei zwei Sauerstoffatome nicht unmittelbar benachbart sein dürfen, die Heterocyclylreste Cycloalkyl und Cycloalkenyl, in welchen eine oder zwei CH₂-Gruppen durch Sauerstoff oder Schwefel ersetzt sind, wobei zwei Sauerstoffatome nicht unmittelbar benachbart sein dürfen, insbesondere Dioxanyl, Dioxolanyl, Dioxepanyl, Dioxocanyl, Oxathianyl, Oxathiolanyl, Oxathiepanyl, Oxathiocanyl, Dithianyl, Dithiolanyl, Dithiepanyl, Dithiocanyl, Oxathianyloxid, Oxathiolanyloxid, Oxathiepanyloxid, Oxathiocanyloxid, Oxathianyldioxid, Oxathiolanyldioxid, Oxathiepanyldioxid, Oxathiocanyldioxid, wobei in diesen Heterocyclylresten zwei am selben C-Atom gebundene H-Atome durch =CH₂, - CH₂-CH₂-, -CH₂-CH₂-CH₂- oder -CH₂-CH₂-CH₂-CH₂- ersetzt sein können, Morpholinyl, Triazolinonyl, Oxazolinyl, Dihydrooxadiazinyl, Dihydrodioxazinyl, Dihydrooxazolyl, Dihydrooxazinyl und Pyrazolinonyl, wobei alle Heterocyclylreste selbst wiederum substituiert sein können durch Alkyl, Halogenalkyl, Alkoxy und Alkoxyalkyl, Phenyl, welches selbst wiederum substituiert sein kann durch Halogen, Cyano, Nitro, Alkyl und Halogenalkyl, die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl, welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl und die Heteroarylalkylreste Triazolylalkyl, Pyridylalkyl, Pyrimidylalkyl und Oxadiazolylalkyl,(welche selbst wiederum substituiert sein können durch Alkyl,
b für eine Zahl aus der Reihe 1, 2 und 3 steht,
E für Aryl steht,
R⁴ für einen Rest aus der Reihe Halogen, Nitro, Amino, Formyl, Cyano, Alkylamino, Halogenalkylamino, Dialkylamino, Alkyl, Halogenalkyl, gegebenenfalls substituiertes Cycloalkyl, gegebenenfalls substituiertes Cycloalkenyl, gegebenenfalls substituiertes Cycloalkylalkyl, wobei im Cycloalkylteil des Cycloalkylalkylrestes eine oder zwei CH₂-Gruppen durch Sauerstoff oder Schwefel ersetzt sein können, aber zwei Sauerstoffatome nicht unmittelbar benachbart sein dürfen, Alkoxy, Halogenalkoxy, Alkoxyalkyl, halogeniertes Alkoxyalkyl, Bis(alkoxy)alkyl, Bis(halogenalkoxy)alkyl, Alkoxy(alkylsulfanyl)alkyl, Alkoxy(alkylsulfinyl)alkyl, Alkoxy(alkylsulfonyl)alkyl, Bis(alkylsulfanyl)alkyl, Bis(halogenalkylsulfanyl)alkyl, Bis(hydroxyalkylsulfanyl)alkyl, Alkoxycarbonyl, Alkoxycarbonylalkyl, alpha-Hydroxyimino-alkoxycarbonylalkyl, alpha-Alkoxyimino-alkokycarbonylalkyl, C(X)NR⁵R⁶, worin X für Sauerstoff, Schwefel, NR¹⁵ oder NOH steht, R⁵ für Wasserstoff oder Alkyl steht und R⁶ und R¹⁵ unabhängig voneinander für einen Rest aus der Reihe Wasserstoff, Alkyl, Halogenalkyl, Cyanoalkyl, Alkinyl, Cycloalkyl, Cycloalkylalkyl, Alkylcarbonyl, Alkoxyalkyl, Alkoxycarbonyl, Alkoxycarbonylalkyl, Alkylthioalkyl, Aryl, Arylalkyl und Hetarylalkyl stehen und R⁶auch für OH stehen kann oder R⁵ und R⁶ gemeinsam mit dem Stickstoffatom, an das sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen oder R⁵ und R¹⁵ gemeinsam mit den Stickstoffatomen, an das sie gebunden sind, einen Ring bilden, der ein oder mehrere weitere Heteroatome aus der Reihe NH, NCH₃, NC₂H₅, Sauerstoff und Schwefel enthalten kann, wobei zwei Sauerstoffatome nicht unmittelbar benachbart sein dürfen, NR⁷R⁸, worin R⁷ für Wasserstoff oder Alkyl steht und R⁸ für einen Rest aus der Reihe Wasserstoff, Alkyl, Halogenalkyl, Cyanoalkyl, Alkinyl, Cycloalkyl, Cycloalkylalkyl, Alkoxy, Halogenalkoxy, Alkoxyalkyl, Alkoxycarbonyl, Alkoxycarbonylalkyl, Alkylthioalkyl, Aryl, Arylalkyl oder Hetarylalkyl steht oder R⁷ und R⁸ gemeinsam mit dem Stickstoffatom, an das sie gebunden sind, einen Ring bilden, der ein oder mehrere weitere Heteroatome aus der Reihe NH, NCH₃, NC₂H₅, Sauerstoff und Schwefel enthalten kann, Alkylthio, Alkylsulfinyl, Alkylsulfonyl, die Heterocyclylreste Cycloalkyl und Cycloalkenyl, in welchen eine oder zwei CH₂-Gruppen durch Sauerstoff oder Schwefel ersetzt sind, wobei zwei Sauerstoffatome nicht unmittelbar benachbart sein dürfen, insbesondere Dioxanyl, Dioxolanyl, Dioxepanyl, Dioxocanyl, Oxathianyl, Oxathiolanyl, Oxathiepanyl, Oxathiocanyl, Dithianyl, Dithiolanyl, Dithiepanyl, Dithiocanyl, Oxathianyloxid, Oxathiolanyloxid, Oxathiepanyloxid, Oxathiocanyloxid, Oxathianyldioxid, Oxathiolanyldioxid, Oxathiepanyldioxid, Oxathiocanyldioxid, wobei in diesen Heterocyclylresten zwei am selben C-Atom gebundene H-Atome durch =CH₂, -CH₂-CH₂-, -CH₂-CH₂-CH₂- oder -CH₂-CH₂-CH₂-CH₂- ersetzt sein können, Morpholinyl, Triazolinonyl, Oxazolinyl, Dihydrooxadiazinyl, Dihydrodioxazinyl, Dihydrooxazolyl, Dihydrooxazinyl und Pyrazolinonyl, wobei alle Heterocyclylreste selbst wiederum substituiert sein können durch Alkyl, Halogenalkyl, Alkoxy und Alkoxyalkyl, Phenyl, welches selbst wiederum substituiert sein kann durch Halogen, Cyano, Nitro, Alkyl und Halogenalkyl, die Heteroarylreste Pyridyl, Pyridyl-N-oxid, Pyrimidyl, Imidazolyl, Pyrazolyl, Oxazolyl, Thiazolyl, Furanyl, Thienyl, Triazolyl, Tetrazolyl, Oxadiazolyl, Thiadiazolyl, Pyrazinyl, Triazinyl, Tetrazinyl und Isochinolinyl, welche selbst wiederum substituiert sein können durch Halogen, Nitro, Alkyl, Halogenalkyl, Alkoxy, Halogenalkoxy, Alkoxyalkyl, Alkylthio, Alkylthioalkyl und Cycloalkyl und die Heteroarylalkylreste Triazolylalkyl, Pyridylalkyl, Pyrimidylalkyl und Oxadiazolylalkyl, welche selbst wiederum substituiert sein können durch Halogen und Alkyl,
und
c für eine Zahl aus der Reihe 0, 1, 2 und 3 steht,
sowie deren Salze, Metallkomplexe und N-Oxide.

3. Mittel, **gekennzeichnet durch** einen Gehalt von mindestens einer Verbindung der Formel (I) gemäß Anspruch 2.

4. Nicht-therapeutisches Verfahren zum Bekämpfen von Schädlingen **dadurch gekennzeichnet, dass** man eine Verbindung der Formel (I) 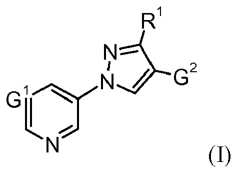 worin
G¹ für N, CH oder C-Halogen steht,
R¹ für Wasserstoff oder Alkyl steht und
G² für gegebenenfalls substituiertes Heterocyclyl, für gegebenenfalls substituiertes Heteroaryl oder für gegebenenfalls substituiertes Aryl steht,
sowie Salze, Metallkomplexe und N-Oxide der Verbindungen der Formel (I) oder eine Verbindung der Formel (I) gemäß Anspruch 2 oder ein Mittel gemäß Anspruch 3 auf die Schädlinge und/oder ihren Lebensraum einwirken lässt.

5. Verbindungen der Formel (VI) 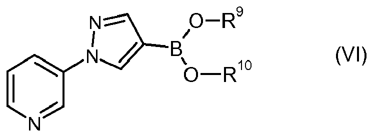 worin
R⁹ und R¹⁰ unabhängig voneinander für H, C₁-C₆-Alkyl stehen oder gegebenenfalls zusammen mit den Atomen, an die sie gebunden sind, einen Ring bilden, der bevorzugt keine weiteren Heteroatome enthält und der wiederum einfach oder mehrfach mit C₁-C₆-Alkyl substituiert sein kann.

6. Verbindungen der Formel (X) 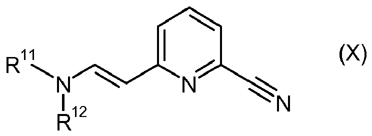 worin
R¹¹ und R¹² für C₁-C₆-Alkyl stehen oder zusammen mit dem Stickstoffatom, an das sie gebunden sind, einen Ring bilden.

7. Verbindungen der Formel (XI) 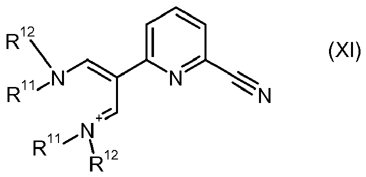 worin R¹¹ und R¹² für C₁-C₆-Alkyl stehen oder zusammen mit dem Stickstoffatom, an das sie gebunden sind, einen Ring bilden, in Form von Salzen.

8. Verbindung der Formel

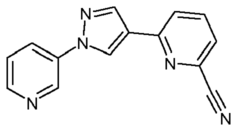

9. Verbindung der Formel 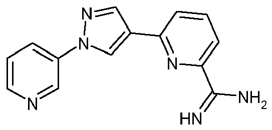 auch in Form ihrer Salze.

## Claims

1. Non-therapeutic use of compounds of the formula (I), 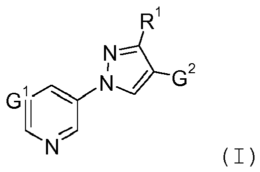 in which
G¹ represents N, CH or C-halogen,
R¹ represents hydrogen or alkyl and
G² represents optionally substituted heterocyclyl, represents optionally substituted heteroaryl or represents optionally substituted aryl,
and also salts, metal complexes and N-oxides of the compounds of the formula (I), for controlling animal pests.

2. Compounds of the formula (I) 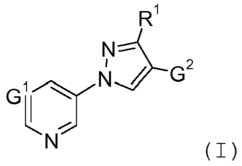 in which
G¹ represents N, CH, C-halogen C-nitro, C-cyano, C-alkyl, C-haloalkyl, C-cycloalkyl, C-alkoxy, C-haloalkoxy,
R¹ represents hydrogen, alkyl haloalkyl, cycloalkyl, halogen, cyano, alkoxy, haloalkoxy, amino, alkylamino, dialkylamino or alkylthio,
G² represents A-R²ₐ, D-R³_{b} or E-R⁴_{c} in which
A represents heterocyclyl from the group consisting of 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, oxazolin-2-yl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 5,6-dihydro-[1,3,4]-oxadiazin-2-yl, 5,6-dihydro-[1,3,4]-thiadiazin-2-yl, 5,6-dihydro-[1,4,2]-dioxazin-3-yl, hydroxypyridyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,3-dihydrothien-2-yl, 2,3-dihydrothien-3-yl, 2,4-dihydrothien-2-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl,
R² represents a radical from the group consisting of halogen, cyano, nitro, alkyl, haloalkyl, optionally substituted cycloalkyl, alkoxy, haloalkoxy, alkylthio, haloalkylthio, alkylsulphinyl, alkylsulphonyl, haloalkylsulphinyl, haloalkylsulphonyl, amino, alkylamino, dialkylamino, alkylcarbonylamino, alkoxycarbonylamino, alkoxyalkyl, haloalkoxyalkyl, alkenyl, alkynyl, optionally substituted cycloalkylalkyl, alkylcarbonyl, alkoxycarbonyl, aminocarbonyl, pyridyl and pyrimidyl (where pyridyl and pyrimidyl for their part may be substituted by halogen, alkyl, haloalkyl, alkoxy and haloalkoxy),
a represents a number from the group consisting of 0, 1, 2 and 3,
D represents a heteroaryl radical from the group consisting of pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazolyl, quinolinyl, isoquinolinyl, cinnolinyl, phthalazinyl, quinazolinyl, quinoxalinyl, naphthyridinyl, benzotriazinyl, purinyl, pteridinyl and indolizinyl,
R³ represents a radical from the group consisting of optionally substituted cycloalkyl, optionally substituted cycloalkenyl, optionally substituted cycloalkylalkyl, where in the cycloalkyl moiety of the cycloalkylalkyl radical one or two CH₂ groups may be replaced by oxygen or sulphur, but two oxygen atoms must not be directly adjacent to one another, bis(alkoxy)alkyl, bis(haloalkoxy)alkyl, alkoxy(alkylsulphanyl)alkyl, alkoxy(alkylsulphinyl)alkyl, alkoxy(alkylsulphonyl)alkyl, bis(alkylsulphanyl)alkyl, bis(haloalkylsulphanyl)alkyl, bis(hydroxyalkylsulphanyl)alkyl, alkoxycarbonylalkyl, alpha-hydroxyimino-alkoxycarbonylalkyl, alpha-alkoxyimino-alkoxycarbonylalkyl, C(X)NR⁵R⁶, (in which X represents sulphur, or NOH, R⁵ represents hydrogen or alkyl and R⁶ represents a radical from the group consisting of hydrogen, alkyl, haloalkyl, cyanoalkyl, alkynyl, cycloalkyl, cycloalkylalkyl, alkylcarbonyl, alkoxyalkyl, alkoxycarbonyl, alkoxycarbonylalkyl, alkylthioalkyl, aryl (in particular phenyl), arylalkyl (in particular benzyl) and hetarylalkyl (in particular 2-pyrimidylmethyl) or X represents sulphur, NOH, NR¹⁵ or oxygen and R⁵ and R⁶ together with the nitrogen atom to which they are attached form a ring which optionally contains one or more further heteroatoms from the group consisting of NH, NCH₃, NC₂H₅, oxygen and sulphur, where two oxygen atoms must not be directly adjacent to one another or R⁵ and R¹⁵ together with the nitrogen atoms to which they are attached form a ring which may contain one or more further heteroatoms from the group consisting of NH, NCH₃, NC₂H₅, oxygen and sulphur, where two oxygen atoms must not be directly adjacent to one another, NR⁷R⁷, in which R⁷ represents hydrogen or alkyl and R⁸ represents alkynyl, cycloalkylalkyl, alkoxy, haloalkoxy, alkoxycarbonylalkyl, alkylthioalkyl, arylalkyl or hetarylalkyl or R⁷ and R⁸ together with the nitrogen atom to which they are attached form a ring which optionally contains one or more further heteroatoms from the group consisting of NH, NCH₃, NC₂H₅, oxygen and sulphur, where two oxygen atoms must not be directly adjacent to one another, the heterocyclyl radicals cycloalkyl and cycloalkenyl in which one or two CH₂ groups are replaced by oxygen or sulphur, where two oxygen atoms must not be directly adjacent to one another, in particular dioxanyl, dioxolanyl, dioxepanyl, dioxocanyl, oxathianyl, oxathiolanyl, oxathiepanyl, oxathiocanyl, dithianyl, dithiolanyl, dithiepanyl, dithiocanyl, oxathianyl oxide, oxathiolanyl oxide, oxathiepanyl oxide, oxathiocanyl oxide, oxathianyl dioxide, oxathiolanyl dioxide, oxathiepanyl dioxide, oxathiocanyl dioxide, where in these heterocyclyl radicals two hydrogen atoms attached to the same carbon atom may be replaced by =CH₂, -CH₂-CH₂-, -CH₂-CH₂-CH₂- or -CH₂-CH₂-CH₂-CH₂-, morpholinyl, triazolinonyl, oxazolinyl, dihydrooxadiazinyl, dihydrodioxazinyl, dihydrooxazolyl, dihydrooxazinyl and pyrazolinonyl, where all heterocyclyl radicals for their part may be substituted by alkyl, haloalkyl, alkoxy and alkoxyalkyl, phenyl, which for its part may be substituted by halogen, cyano, nitro, alkyl and haloalkyl, the heteroaryl radicals pyridyl, pyridyl N-oxide, pyrimidyl, imidazolyl, pyrazolyl, oxazolyl, thiazolyl, furanyl, thienyl, triazolyl, tetrazolyl, oxadiazolyl, thiadiazolyl, pyrazinyl, triazinyl, tetrazinyl and isoquinolinyl, which for their part may be substituted by halogen, nitro, alkyl, haloalkyl, alkoxy, haloalkoxy, alkoxyalkyl, alkylthio, alkylthioalkyl and cycloalkyl and the heteroarylalkyl radicals triazolylalkyl, pyridylalkyl, pyrimidylalkyl and oxadiazolylalkyl, which for their part may be substituted by alkyl,
b represents a number from the group consisting of 1, 2 and 3,
E represents aryl,
R⁴ represents a radical from the group consisting of halogen, nitro, amino, formyl, cyano, alkylamino, haloalkylamino, dialkylamino, alkyl, haloalkyl, optionally substituted cycloalkyl, optionally substituted cycloalkenyl, optionally substituted cycloalkylalkyl, where in the cycloalkyl moiety of the cycloalkylalkyl radical one or two CH₂ groups may be replaced by oxygen or sulphur, but two oxygen atoms must not be directly adjacent to one another, alkoxy, haloalkoxy, alkoxyalkyl, halogenated alkoxyalkyl, bis(alkoxy)alkyl, bis(haloalkoxy)alkyl, alkoxy(alkylsulphanyl)alkyl, alkoxy(alkylsulphinyl)alkyl, alkoxy(alkylsulphonyl)alkyl, bis(alkylsulphanyl)alkyl, bis(haloalkylsulphanyl)alkyl, bis(hydroxyalkylsulphanyl)alkyl, alkoxycarbonyl, alkoxycarbonylalkyl, alpha-hydroxyimino-alkoxycarbonylalkyl, alpha-alkoxyimino-alkokycarbonylalkyl, C(X)NR⁵R⁶, in which X represents oxygen, sulphur, NR¹⁵ or NOH, R⁵ represents hydrogen or alkyl and R⁶ and R¹⁵ independently of one another represent a radical from the group consisting of hydrogen, alkyl, haloalkyl, cyanoalkyl, alkynyl, cycloalkyl, cycloalkylalkyl, alkylcarbonyl, alkoxyalkyl, alkoxycarbonyl, alkoxycarbonylalkyl, alkylthioalkyl, aryl, arylalkyl and hetarylalkyl and R⁶ may also represent OH or R⁵ and R⁶ together with the nitrogen atom to which they are attached form a ring which may contain one or more further heteroatoms from the group consisting of NH, NCH₃, NC₂H₅, oxygen and sulphur, where two oxygen atoms must not be directly adjacent to one another or R⁵ and R¹⁵ together with the nitrogen atoms to which they are attached form a ring which may contain one or more further heteroatoms from the group consisting of NH, NCH₃, NC₂H₅, oxygen and sulphur, where two oxygen atoms must not be directly adjacent to one another, NR⁷R⁸, in which R⁷ represents hydrogen or alkyl and R⁸ represents a radical from the group consisting of hydrogen, alkyl, haloalkyl, cyanoalkyl, alkynyl, cycloalkyl, cycloalkylalkyl, alkoxy, haloalkoxy, alkoxyalkyl, alkoxycarbonyl, alkoxycarbonylalkyl, alkylthioalkyl, aryl, arylalkyl and hetarylalkyl or R⁷ and R⁸ together with the nitrogen atom to which they are attached form a ring which may contain one or more further heteroatoms from the group consisting of NH, NCH₃, NC₂H₅, oxygen and sulphur, alkylthio, alkylsulphinyl, alkylsulphonyl, the heterocyclyl radicals cycloalkyl and cycloalkenyl in which one or two CH₂ groups are replaced by oxygen or sulphur, where two oxygen atoms must not be directly adjacent to one another, in particular dioxanyl, dioxolanyl, dioxepanyl, dioxocanyl, oxathianyl, oxathiolanyl, oxathiepanyl, oxathiocanyl, dithianyl, dithiolanyl, dithiepanyl, dithiocanyl, oxathianyl oxide, oxathiolanyl oxide, oxathiepanyl oxide, oxathiocanyl oxide, oxathianyl dioxide, oxathiolanyl dioxide, oxathiepanyl dioxide, oxathiocanyl dioxide, where in these heterocyclyl radicals two hydrogen atoms attached to the same carbon atom may be replaced by =CH₂, -CH₂-CH₂-, -CH₂-CH₂-CH₂- or -CH₂-CH₂-CH₂-CH₂-, morpholinyl, triazolinonyl, oxazolinyl, dihydrooxadiazinyl, dihydrodioxazinyl, dihydrooxazolyl, dihydrooxazinyl and pyrazolinonyl, where all heterocyclyl radicals for their part may be substituted by alkyl, haloalkyl, alkoxy and alkoxyalkyl, phenyl, which for its part may be substituted by halogen, cyano, nitro, alkyl and haloalkyl, the heteroaryl radicals pyridyl, pyridyl N-oxide, pyrimidyl, imidazolyl, pyrazolyl, oxazolyl, thiazolyl, furanyl, thienyl, triazolyl, tetrazolyl, oxadiazolyl, thiadiazolyl, pyrazinyl, triazinyl, tetrazinyl and isoquinolinyl, which for their part may be substituted by halogen, nitro, alkyl, haloalkyl, alkoxy, haloalkoxy, alkoxyalkyl, alkylthio, alkylthioalkyl and cycloalkyl and the heteroarylalkyl radicals triazolylalkyl, pyridylalkyl, pyrimidylalkyl and oxadiazolylalkyl, which for their part may be substituted by halogen and alkyl,
and
c represents a number from the group consisting of 0, 1, 2 and 3,
and also salts, metal complexes and N-oxides thereof.

3. Compositions, **characterized in that** they comprise at least one compound of the formula (I) according to Claim 2.

4. Non-therapeutic method for controlling pests, **characterized in that** a compound of the formula (I) 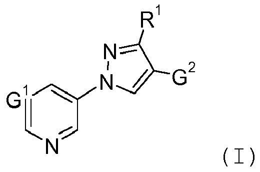 in which
G¹ represents N, CH or C-halogen,
R¹ represents hydrogen or alkyl and
G² represents optionally substituted heterocyclyl, represents optionally substituted heteroaryl or represents optionally substituted aryl,
and also salts, metal complexes and N-oxides of the compounds of the formula (I) or a compound of the formula (I) according to Claim 2 or a composition according to Claim 3 is allowed to act on the pests and/or their habitat.

5. Compounds of the formula (VI) 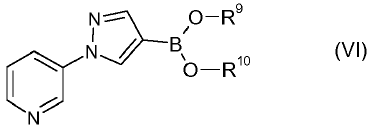 in which
R⁹ and R¹⁰ independently of one another represent H, C₁-C₆-alkyl or, if appropriate, together with the atoms to which they are attached, a ring which preferably does not contain any further heteroatoms and which for its part may be mono- or polysubstituted by C₁-C₆-alkyl.

6. Compounds of the formula (X) 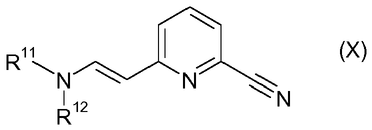 in which
R¹¹ and R¹² represent C₁-C₆-alkyl or, together with the nitrogen atom to which they are attached, form a ring.

7. Compounds of the formula (XI) 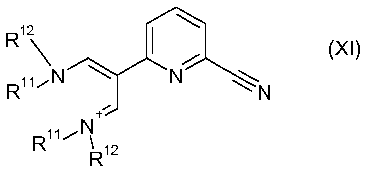 in which
R¹¹ and R¹² represent C₁-C₆-alkyl or, together with the nitrogen atom to which they are attached, form a ring, in the form of salts.

8. Compound of the formula

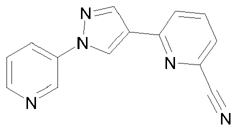

9. Compound of the formula 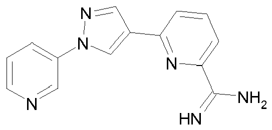 also in the form of its salts.

## Revendications

1. Utilisation non thérapeutique de composés de formule (I) 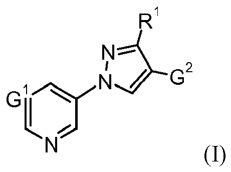 dans laquelle
G¹ représente N, CH ou C-halogène,
R¹ représente hydrogène ou alkyle et
G² représente hétérocyclyle le cas échéant substitué, hétéroaryle le cas échéant substitué ou aryle le cas échéant substitué,
ainsi que des sels, des complexes métalliques et des N-oxydes des composés de formule (I) pour lutter contre des organismes nuisibles animaux.

2. Composés de formule (I) 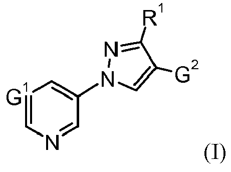 dans laquelle
G¹ représente N, CH, C-halogène C-nitro, C-cyano, C-alkyle, C-halogénoalkyle, C-cycloalkyle, C-alcoxy, C-halogénoalcoxy,
R¹ représente hydrogène, alkyle, halogénoalkyle, cycloalkyle, halogène, cyano, alcoxy, halogénoalcoxy, amino, alkylamino, dialkylamino ou alkylthio,
G² représente A-R²ₐ, D-R³_{b} ou E-R⁴_{c}, dans lesquelles
A représente hétérocyclyle de la série 2-tétrahydrofurannyle, 3-tétrahydrofurannyle, 2-tétrahydrothiényle, 3-tétrahydrothiényle, 2-pyrrolidinyle, 3-pyrrolidinyle, 3-isoxazolidinyle, 4-isoxazolidinyle, 5-isoxazolidinyle, 3-isothiazolidinyle, 4-isothiazolidinyle, 5-isothiazolidinyle, 3-pyrazolidinyle, 4-pyrazolidinyle, 5-pyrazolidinyle, oxazolin-2-yle, 2-oxazolidinyle, 4-oxazolidinyle, 5-oxazolidinyle, 2-thiazolidinyle, 4-thiazolidinyle, 5-thiazolidinyle, 2-imidazolidinyle, 4-imidazolidinyle, 5,6-dihydro-[1,3,4]-oxadiazin-2-yle, 5,6-dihydro-[1,3,4]-thiadiazin-2-yle, 5,6-dihydro-[1,4,2]-dioxazin-3-yle, hydroxypyridyle, 1,2,4-oxadiazolidin-3-yle, 1,2,4-oxadiazolidin-5-yle, 1,2,4-thiadiazolidin-3-yle, 1,2,4-thiadiazolidin-5-yle, 1,2,4-triazolidin-3-yle, 1,3,4-oxadiazolidin-2-yle, 1,3,4-thiadiazolidin-2-yle, 1,3,4-triazolidin-2-yle, 2,3-dihydrofur-2-yle, 2,3-dihydrofur-3-yle, 2,4-dihydrofur-2-yle, 2,3-dihydrothién-2-yle, 2,3-dihydrothién-3-yle, 2,4-dihydrothién-2-yle, 2-pyrrolin-2-yle, 2-pyrrolin-3-yle, 3-pyrrolin-2-yle, 3-pyrrolin-3-yle, 2-isoxazolin-3-yle, 3-isoxazolin-3-yle, 4-isoxazolin-3-yle, 2-isoxazolin-4-yle, 3-isoxazolin-4-yle, 4-isoxazolin-4-yle, 2-isoxazolin-5-yle, 3-isoxazolin-5-yle, 4-isoxazolin-5-yle, 2-isothiazolin-3-yle, 3-isothiazolin-3-yle, 4-isothiazolin-3-yle, 2-isothiazolin-4-yle, 3-isothiazolin-4-yle, 4-isothiazolin-4-yle, 2-isothiazolin-5-yle, 3-isothiazolin-5-yle, 4-isothiazolin-5-yle, 2,3-dihydropyrazol-1-yle, 2,3-dihydropyrazol-2-yle, 2,3-dihydropyrazol-3-yle, 2,3-dihydropyrazol-4-yle, 2,3-dihydropyrazol-5-yle, 3,4-dihydropyrazol-1-yle, 3,4-dihydropyrazol-3-yle, 3,4-dihydropyrazol-4-yle, 3,4-dihydropyrazol-5-yle, 4,5-dihydropyrazol-1-yle, 4,5-dihydropyrazol-3-yle, 4,5-dihydropyrazol-4-yle, 4,5-dihydropyrazol-5-yle, 2,3-dihydrooxazol-2-yle, 2,3-dihydrooxazol-3-yle, 2,3-dihydrooxazol-4-yle, 2,3-dihydrooxazol-5-yle, 3,4-dihydrooxazol-2-yle, 3,4-dihydrooxazol-3-yle, 3,4-dihydrooxazol-4-yle, 3,4-dihydrooxazol-5-yle, 2-pipéridinyle, 3-pipéridinyle, 4-pipéridinyle, 1,3-dioxan-5-yle, 2-tétrahydropyrannyle, 4-tétrahydropyrannyle, 2-tétrahydrothiényle, 3-hexahydropyridazinyle, 4-hexahydropyridazinyle, 2-hexahydropyrimidinyle, 4-hexahydropyrimidinyle, 5-hexahydropyrimidinyle, 2-pipérazinyle, 1,3,5-hexahydrotriazin-2-yle et 1,2,4-hexahydrotriazin-3-yle,
R² représente un radical de la série halogène, cyano, nitro, alkyle, halogénoalkyle, cycloalkyle le cas échéant substitué, alcoxy, halogénoalcoxy, alkylthio, halogénoalkylthio, alkylsulfinyle, alkylsulfonyle, halogénoalkylsulfinyle, halogénoalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle le cas échéant substitué, alkylcarbonyle, alcoxycarbonyle, aminocarbonyle, pyridyle et pyrimidyle, pyridyle et pyrimidyle pouvant eux-mêmes à leur tour être substitués par halogène, alkyle, halogénoalkyle, alcoxy et halogénoalcoxy,
a vaut un nombre de la série 0, 1, 2 ou 3,
D représente un radical hétéroaryle de la série pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzo-isofuryle, benzothiényle, benzo-isothiényle, indolyle, iso-indolyle, indazolyle, benzothiazolyle, benzo-isothiazolyle, benzoxazolyle, benzo-isoxazolyle, benzimidazolyle, 2,1,3-benzoxadiazolyle, quinoléinyle, isoquinoléinyle, cinnolinyle, phtalazinyle, quinazolinyle, quinoxalinyle, naphtyridinyle, benzotriazinyle, purinyle, ptéridinyle et indolizinyle,
R³ représente un radical de la série cycloalkyle le cas échéant substitué, cycloalcényle le cas échéant substitué, cycloalkylalkyle le cas échéant substitué, où, dans la partie cycloalkyle du radical cycloalkylalkyle, un ou deux groupes CH₂ peuvent être remplacés par oxygène ou soufre, mais deux atomes d'oxygène ne peuvent pas être directement adjacents ; bis(alcoxy)alkyle, bis(halogénoalcoxy)alkyle, alcoxy(alkylsulfanyl)alkyle, alcoxy(alkylsulfinyl)alkyle, alcoxy(alkylsulfonyl)alkyle, bis(alkylsulfanyl)alkyle, bis(halogénoalkylsulfanyl)alkyle, bis(hydroxyalkylsulfanyl)alkyle, alcoxycarbonylalkyle, alpha-hydroxyiminoalcoxycarbonylalkyle, alpha-alcoxyiminoalcoxycarbonylalkyle, C(X)NR⁵R⁶, où X représente soufre, ou NOH, R⁵ représente hydrogène ou alkyle et R⁶ représente un radical de la série hydrogène, alkyle, halogénoalkyle, cyanoalkyle, alcynyle, cycloalkyle, cycloalkylalkyle, alkylcarbonyle, alcoxyalkyle, alcoxycarbonyle, alcoxycarbonylalkyle, alkylthioalkyle, aryle, arylalkyle et hétarylalkyle ou X représente soufre, NOH, NR¹⁵ ou oxygène et R⁵ et R⁶ forment, ensemble avec l'atome d'azote auquel ils sont liés, un cycle qui contient le cas échéant un ou plusieurs autres hétéroatomes de la série NH, NCH₃, NC₂H₅, oxygène et soufre, deux atomes d'oxygène ne pouvant pas être directement adjacents ou R⁵ et R¹⁵ forment, ensemble avec les atomes d'azote auxquels ils sont liés, un cycle, qui peut contenir un ou plusieurs autres hétéroatomes de la série NH, NCH₃, NC₂H₅, oxygène et soufre, deux atomes d'oxygène ne pouvant pas être directement adjacents, NR⁷R⁸, où R⁷ représente hydrogène ou alkyle et R⁸ représente alcynyle, cycloalkylalkyle, alcoxy, halogénoalcoxy, alcoxycarbonylalkyle, alkylthioalkyle, arylalkyle ou hétéroarylalkyle ou R⁷ et R⁸ forment, ensemble avec l'atome d'azote auquel ils sont liés, un cycle, qui contient le cas échéant un ou plusieurs autres hétéroatomes de la série NH, NCH₃, NC₂H₅, oxygène et soufre, deux atomes d'oxygène ne pouvant pas être directement adjacents, les radicaux hétérocyclyle, cycloalkyle et cycloalcényle, dans lesquels un ou deux groupes CH₂ peuvent être remplacés par oxygène ou soufre, deux atomes d'oxygène ne pouvant pas être directement adjacents, représentant en particulier dioxanyle, dioxolanyle, dioxépanyle, dioxocanyle, oxathianyle, oxathiolanyle, oxathiépanyle, oxathiocanyle, dithianyle, dithiolanyle, dithiépanyle, dithiocanyle, oxyde d'oxathianyle, oxyde d'oxathiolanyle, oxyde d'oxathiépanyle, oxyde d'oxathiocanyle, dioxyde d'oxathianyle, dioxyde d'oxathiolanyle, dioxyde d'oxathiépanyle, dioxyde d'oxathiocanyle, où, dans ces radicaux hétérocyclyle, deux atomes H liés au même atome de C peuvent être remplacés par =CH₂, -CH₂-CH₂-, -CH₂-CH₂-CH₂- ou -CH₂-CH₂-CH₂-CH₂- ; morpholinyle, triazolinonyle, oxazolinyle, dihydrooxadiazinyle, dihydrodioxazinyle, dihydrooxazolyle, dihydrooxazinyle et pyrazolinonyle, où tous les radicaux hétérocyclyle peuvent eux-mêmes à leur tour être substitués par alkyle, halogénoalkyle, alcoxy et alcoxyalkyle, phényle, qui peut lui-même à son tour être substitué par halogène, cyano, nitro, alkyle et halogénoalkyle ; les radicaux hétéroaryle pyridyle, pyridyl-N-oxyde, pyrimidyle, imidazolyle, pyrazolyle, oxazolyle, thiazolyle, furannyle, thiényle, triazolyle, tétrazolyle, oxadiazolyle, thiadiazolyle, pyrazinyle, triazinyle, tétrazinyle et isoquinoléinyle, qui peuvent eux-mêmes à leur tour être substitués par halogène, nitro, alkyle, halogénoalkyle, alcoxy, halogénoalcoxy, alcoxyalkyle, alkylthio, alkylthioalkyle et cycloalkyle et les radicaux hétéroarylalkyle triazolylalkyle, pyridylalkyle, pyrimidylalkyle et oxadiazolylalkyle, (qui peuvent eux-mêmes à leur tour être substitués par alkyle),
b vaut un nombre de la série 1, 2 ou 3,
E représente aryle,
R⁴ représente un radical de la série halogène, nitro, amino, formyle, cyano, alkylamino, halogénoalkylamino, dialkylamino, alkyle, halogénoalkyle, cycloalkyle le cas échéant substitué, cycloalcényle le cas échéant substitué, cycloalkylalkyle le cas échéant substitué, où, dans la partie cycloalkyle du radical cycloalkylalkyle, un ou deux groupes CH₂ peuvent être remplacés par oxygène ou soufre, mais deux atomes d'oxygène ne peuvent pas être directement adjacents, alcoxy, halogénoalcoxy, alcoxyalkyle, alcoxyalkyle halogéné, bis(alcoxy)alkyle, bis(halogénoalcoxy)alkyle, alcoxy(alkylsulfanyl)alkyle, alcoxy(alkylsulfinyl)alkyle, alcoxy(alkylsulfonyl)alkyle, bis(alkylsulfanyl)alkyle, bis(halogénoalkylsulfanyl)alkyle, bis(hydroxyalkylsulfanyl)alkyle, alcoxycarbonyle, alcoxycarbonylalkyle, alpha-hydroxyiminoalcoxycarbonylalkyle, alpha-alcoxyiminoalcoxycarbonylalkyle, C(X)NR⁵R⁶, où X représente oxygène, soufre, NR¹⁵ ou NOH, R⁵ représente hydrogène ou alkyle et R⁶ et R¹⁵ représentent, indépendamment l'un de l'autre, un radical de la série hydrogène, alkyle, halogénoalkyle, cyanoalkyle, alcynyle, cycloalkyle, cycloalkylalkyle, alkylcarbonyle, alcoxyalkyle, alcoxycarbonyle, alcoxycarbonylalkyle, alkylthioalkyle, aryle, arylalkyle et hétéroarylalkyle et R⁶ peut également représenter OH ou R⁵ et R⁶ peuvent former, ensemble avec l'atome d'azote auquel ils sont liés, un cycle qui peut contenir un ou plusieurs autres hétéroatomes de la série NH, NCH₃, NC₂H₅, oxygène et soufre, deux atomes d'oxygène ne pouvant pas être directement adjacents ou R⁵ et R¹⁵ forment, ensemble avec les atomes d'azote auxquels ils sont liés, un cycle qui peut contenir un ou plusieurs autres hétéroatomes de la série NH, NCH₃, NC₂H₅, oxygène et soufre, deux atomes d'oxygène ne pouvant pas être directement adjacents, NR⁷R⁸, où R⁷ représente hydrogène ou alkyle et R⁸ représente un radical de la série hydrogène, alkyle, halogénoalkyle, cyanoalkyle, alcynyle, cycloalkyle, cycloalkylalkyle, alcoxy, halogénoalcoxy, alcoxyalkyle, alcoxycarbonyle, alcoxycarbonylalkyle, alkylthioalkyle, aryle, arylalkyle ou hétéroarylalkyle ou R⁷ et R⁸ forment, ensemble avec l'atome d'azote auquel ils sont liés, un cycle qui peut contenir un ou plusieurs hétéroatomes de la série NH, NCH₃, NC₂H₅, oxygène et soufre ; alkylthio, alkylsulfinyle, alkylsulfonyle, les radicaux hétérocyclyle, cycloalkyle et cycloalcényle, dans lesquels un ou deux groupes CH₂ sont remplacés par oxygène ou soufre, deux atomes d'oxygène ne pouvant pas être directement adjacents, en particulier dioxanyle, dioxolanyle, dioxépanyle, dioxocanyle, oxathianyle, oxathiolanyle, oxathiépanyle, oxathiocanyle, dithianyle, dithiolanyle, dithiépanyle, dithiocanyle, oxyde d'oxathianyle, oxyde d'oxathiolanyle, oxyde d'oxathiépanyle, oxyde d'oxathiocanyle, dioxyde d'oxathianyle, dioxyde d'oxathiolanyle, dioxyde d'oxathiépanyle, dioxyde d'oxathiocanyle, où, dans ces radicaux hétérocyclyle, deux atomes H liés au même atome de C peuvent être remplacés par =CH₂, -CH₂-CH₂-, -CH₂-CH₂-CH₂- ou -CH₂-CH₂-CH₂-CH₂- ; morpholinyle, triazolinonyle, oxazolinyle, dihydrooxadiazinyle, dihydrodioxazinyle, dihydrooxazolyle, dihydrooxazinyle et pyrazolinonyle, tous les radicaux hétérocyclyle pouvant eux-mêmes à leur tour être substitués par alkyle, halogénoalkyle, alcoxy et alcoxyalkyle, phényle, qui peut lui-même à son tour être substitué par halogène, cyano, nitro, alkyle et halogénoalkyle, les radicaux hétéroaryle pyridyle, pyridyl-N-oxyde, pyrimidyle, imidazolyle, pyrazolyle, oxazolyle, thiazolyle, furannyle, thiényle, triazolyle, tétrazolyle, oxadiazolyle, thiadiazolyle, pyrazinyle, triazinyle, tétrazinyle et isoquinoléinyle, qui peuvent eux-mêmes à leur tour être substitués par halogène, nitro, alkyle, halogénoalkyle, alcoxy, halogénoalcoxy, alcoxyalkyle, alkylthio, alkylthioalkyle et cycloalkyle et les radicaux hétéroarylalkyle triazolylalkyle, pyridylalkyle, pyrimidylalkyle et oxadiazolylalkyle, qui peuvent eux-mêmes à leur tour être substitués par halogène et alkyle, et
c vaut un nombre de la série 0, 1, 2 ou 3,
ainsi que leurs sels, complexes métalliques et N-oxydes.

3. Agent, **caractérisé par** une teneur en au moins un composé de formule (I) selon la revendication 2.

4. Procédé non thérapeutique pour lutter contre les organismes nuisibles, **caractérisé en ce qu'**on laisse agir un composé de formule (I) 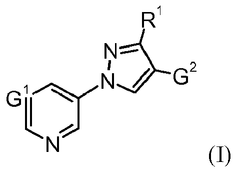 dans laquelle
G¹ représente N, CH ou C-halogène,
R¹ représente hydrogène ou alkyle et
G² représente hétérocyclyle le cas échéant substitué, hétéroaryle cas échéant substitué ou aryle le cas échéant substitué,
ainsi que des sels, des complexes métalliques et des N-oxydes des composés de formule (I) ou un composé de formule (I) selon la revendication 2 ou un agent selon la revendication 3 sur les organismes nuisibles et/ou leur espace de vie.

5. Composés de formule (VI) 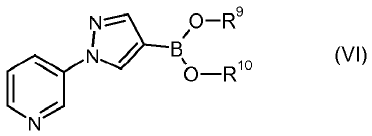 dans laquelle
R⁹ et R¹⁰ représentent, indépendamment l'un de l'autre, H, C₁-C₆-alkyle ou forment, le cas échéant ensemble avec les atomes auxquels ils sont liés, un cycle qui ne contient de préférence pas d'autres hétéroatomes et qui peut à son tour être monosubstitué ou polysubstitué par C₁-C₆-alkyle.

6. Composés de formule (X) 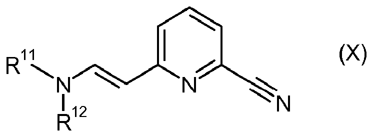 dans laquelle
R¹¹ et R¹² représentent C₁-C₆-alkyle ou forment, ensemble avec l'atome d'azote auquel ils sont liés, un cycle.

7. Composés de formule (XI) 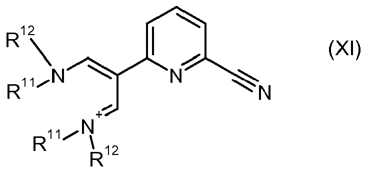 dans laquelle
R¹¹ et R¹² représentent C₁-C₆-alkyle ou forment, ensemble avec l'atome d'azote auquel ils sont liés, un cycle, sous forme de sels.

8. Composé de formule

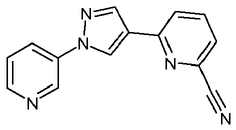

9. Composé de formule 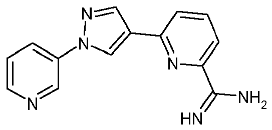 également sous forme de ses sels.
